# DEMANDE DE BREVET EUROPEEN

(11) **EP 1 785 487 A2**
(43) Date de publication de la demande: **16.05.2007**
(21) Numéro de dépôt: 07000325.6
(22) Date de dépôt: 29.10.1991
(51) Int. Cl.: C12N 15/52, C12Q 1/68, C12Q 1/10

(54) **Polypeptides impliqués dans l'expression de la résistance aux antibiotiques glycopeptides**

(30) Priorité: 31.10.1990 FR 9013579
(62) Demande divisionnaire de: 91920753.0
(71) Demandeur: INSTITUT PASTEUR, 75724 Paris Cédex 15 (FR)
(72) Inventeur: Arthur, Michel, 75010 Paris (FR); Dukta-Malen, Sylvie, 94260 Fresnes (FR); Molinas, Catherine, 75018 Paris (FR); Courvalin, Patrice, 75015 Paris (FR)
(74) Mandataire: Desaix, Anne

(57) **Abrégé**

L'invention concerne une composition de polypeptides, caractérisée en ce qu'elle comprend au moins une protéine ou partie de protéine choisie parmi les séquences d'acides aminés identifiées dans la liste des séquences par SED ID NO 1 (VanH), SEQ ID NO 2 (VanA), SEQ ID NO 3 (VanX) ou SEQ ID NO 19 (VanC), ou toute protéine ou partie de protéine reconnue par les anticorps dirigés contre VanH, VanA, VanX ou VanC, ou toute protéine ou partie de protéine codée par une séquence hybridant avec l'un des enchainements nucléotidiques identifiés dans la liste des séquences par SEQ ID NO 8, SEQ ID NO 9 ou SEQ ID NO 10 ou avec l'une des séquences V1 ou V2 suivantes dans des conditions stringentes ou peu stringentes :

V1 : GGX GAA GAT GGX TCX TTX CAA GGX
           G   C     AG  C     G
                         A

V2 : AAT ACX ATX CCX GGX TTT AC
       C       T           C
               C

L'invention concerne également les séquences nucléotidiques codant pour ces polypeptides ainsi que leur utilisation pour le diagnostic à une résistance aux glycopeptides.

## Description

L'invention concerne les polypeptides associés à l'expression de la résistance à des antibiotiques de la famille des glycopeptides, notamment chez des bactéries à Gram-positif, en particulier de la famille des cocci à Gram-positif. L'invention vise également une séquence nucléotidique codant pour ces polypeptides. Elle concerne aussi l'utilisation de ces polypeptides et de leur séquence nucléotidique en tant que moyens de détection in vitro d'une résistance à des glycopeptides. Parmi les cocci à Gram-positif, l'invention vise tout particulièrement les entérocoques, les streptocoques et les staphylocoques qui présentent un intérêt particulier pour la mise en oeuvre de l'invention.

Les glycopeptides, parmi lesquels la vancomycine et la teicoplanine, sont des antibiotiques inhibiteurs de la synthèse de la paroi bactérienne. Ces antibiotiques sont très utilisés pour le traitement des infections sévères dues à des cocci à Gram-positif (entérocoques, streptocoques et staphylocoques), en particulier dans les cas d'allergie et de résistance aux pénicillines. Malgré un long usage clinique de la vancomycine, cet antibiotique est resté actif sur la quasi totalité des souches jusqu'en 1986, date à laquelle ont été isolées les premières souches résistantes. Depuis, la résistance aux glycopeptides a été détectée par de nombreux microbiologistes en Europe et aux Etats Unis, notamment chez des souches isolées de malades immunodéprimés, rendant nécessaire une évaluation systématique de la sensibilité des germes en milieu hospitalier.

L'activité des glycopeptides dépend de la formation d'un complexe entre l'antibiotique et les précurseurs du peptidoglycane, plus que de l'interaction directe avec des enzymes du métabolisme de la paroi cellulaire. En particulier on a constaté que les glycopeptides se fixent aux résidus terminaux D-alanyl-D-alanine (D-ala-D-ala) des précurseurs du peptidoglycane.

L'émergence récente d'une résistance aux glycopeptides notamment chez des entérocoques, a conduit à l'obtention de certains résultats au niveau de la connaissance des facteurs conférant cette résistance.

On a par exemple constaté dans une souche d'entérocoque particulière, Enteroccus faecium BM4147, que le déterminant de la résistance aux glycopeptides était localisé sur un plasmide de 34 kb, le plasmide pIP816. Ce déterminant a été cloné dans E.coli (Brisson Noël et al, 1990, Antimicrob Agents Chemother 34, 924-927).

D'après les résultats obtenus jusqu'à présent, la résistance aux glycopeptides était associée avec la production d'une protéine de poids moléculaire d'environ 40 kDa, la synthèse de cette protéine étant induite par des concentrations sous-inhibitrices de certains glycopeptides comme la vancomycine.

En réalisant une étude plus approfondie de la résistance de certaines souches de cocci à Gram-positif vis à vis de glycopeptides notamment de la vancomycine ou de la teicoplanine, les inventeurs ont constaté que cette résistance serait liée à l'expression de plusieurs protéines ou polypeptides codés par des séquences généralement portées par des plasmides chez les souches résistantes. Les nouveaux résultats obtenus par les inventeurs permettent en outre de distinguer les gènes codant pour deux phénotypes de résistance, d'une part les souches hautement résistantes aux glycopeptides, d'autre part des souches résistantes à un bas niveau.

Par souche résistante à un haut niveau, on entend une souche de bactérie en particulier une souche de cocci à Gram-positif pour laquelle les concentrations minimales inhibitrices (CMI) de vancomycine et de teicoplanine sont respectivement supérieures à 32 et 8 µg/ml. Les CMI de la vancomycine vis à vis des souches résistantes à bas niveau sont comprises entre 16 et 32 *µ*g/ml. Ces souches sont apparemment sensibles à la teicoplanine.

Les inventeurs ont isolé et purifié, parmi les composants nécessaires à l'expression de la résistance aux glycopeptides, une protéine particulière dénommée VANA ou VanA présentant une certaine homologie avec des D-alanine-D-alanine ligases. VanA est néanmoins fonctionnellement distincte des ligases.

On désignera en principe par "van..." une séquence de gène et par "Van..." une séquence d'acides aminés.

L'invention concerne des polypeptides ou protéines impliqués dans l'expression d'une résistance à des antibiotiques de la famille des glycopeptides et notamment à la vancomycine et/ou à la teicoplanine, ainsi que les séquences nucléotidiques codant pour de tels complexes.

L'invention vise également des sondes nucléotidiques utilisables pour la détection d'une résistance aux glycopeptides, notamment par la réaction de polymérisation en chaîne (PCR), ou par des tests faisant intervenir des anticorps.

L'invention concerne une composition de polypeptides, caractérisée en ce qu'elle comprend au moins une protéine ou partie de protéine choisie parmi les séquences d'acides aminés identifiées dans la liste des séquences par SED ID NO 1 (VanH), SEQ ID NO 2 (VanA), SEQ ID NO 3 (VanX) ou SEQ ID NO 19 (VanC), ou toute protéine ou partie de protéine reconnue par les anticorps dirigés contre VanH, VanA, VanX ou VanC, ou toute protéine ou partie de protéine codée par une séquence hybridant avec l'un des enchaînements nucléotidiques identifiés dans la liste des séquences par SEQ ID NO 8, SEQ ID NO 9, SEQ ID NO 10 ou SEQ ID NO 21 ou avec l'une des séquences V1 ou V2 suivantes dans des conditions stringentes ou peu stringentes :

V1 : GGX GAA GAT GGX TCX TTX CAA GGX
           G   C     AG    C   G
                           A

V2 : AAT ACX ATX CCX GGX TTT AC
       C       T           C
               C

Une première composition particulière selon l'invention impliquée dans l'expression de la résistance aux glycopeptides est caractérisée en ce qu'elle comprend au moins 3 protéines ou toute partie de l'une ou plusieurs de ces protéines nécessaires pour conférer à des bactéries à Gram-positif, la résistance à des antibiotiques de la famille des glycopeptides, notamment à la vancomycine et/ou à la teicoplanine, ou de favoriser cette résistance, en particulier dans des souches de la famille des cocci à Gram-positif, ces protéines ou parties de protéines étant
a) reconnues par des anticorps dirigés contre l'une des séquences identifiées dans la liste des séquences par SEQ ID NO 1, SEQ ID NO 2, SEQ ID NO 3,
b) soit codées par des gènes comportant une séquence identifiée par SEQ ID NO 8, SEQ ID NO 9 ou SEQ ID NO 10 ou hybridant avec l'une de ces séquences ou sa séquence complémentaire, dans des conditions stringentes ou peu stringentes ou avec les séquences V1 ou V2.

Ces séquences sont encore désignées respectivement par ORF3, ORF1 contenant le gène vanH, vanA (ou ORF2) ; elles caractérisent les protéines de résistance telles qu'obtenues à partir de la souche Enterococcus faecium BM4147 décrite par Leclerq et al (N Engl J Med 319:157-161).

Une autre protéine, VanC, apparentée à des D-Ala-D-Ala ligases mais de spécificité différente a été caractérisée chez Enterococcus gallinarum BM4173 ; le gène vanC présente des domaines ayant une homologie suffisante avec le gène vanA, pour que des sondes correspondant à des régions déterminées de vanA permettent sa détection.

E. gallinarum est un isolat résistant de façon constitutive à de faibles niveaux de vancomycine (Dutka-Malen et al, Antimicrob. Agents Chemother 34 (1990b) 1875-1879).

Par l'expression "polypeptides" on entend tout enchaînement d'acides aminés pouvant contenir des protéines, ou d'une taille inférieure à celle d'une protéine.

Les conditions de stringence dont il est question ci-dessus sont déterminées selon les modalités habituelles s'agissant de l'hybridation de séquences nucléotidiques. A titre d'exemple, s'agissant des séquences hybridant avec la séquence du gène vanA (SEQ ID NO 8) on pourra appliquer les conditions suivantes :
- pour une hybridation dans de conditions de forte stringence :
   * température de la réaction 65°C pendant une nuit dans une solution contenant 0,1% de SDS, 0,7% de lait en poudre écrémé, 6xSSC (1xSSC=0,15M NaCl et 0,015M de citrate de sodium à pH=7,0)
   * lavages à 65°C dans 2xSSC-0,1% SDS ;
- pour une hybridation dans des conditions peu stringentes, la température d'hybridation est 60°C pendant une nuit et la température des lavages 45°C.

L'expression d'une résistance à des glycopeptides peut se traduire par la persistance d'une infection due à des germes habituellement sensibles aux glycopeptides.

Un polypeptide ou une protéine est nécessaire à l'expression d'une résistance aux glycopeptides, dès lors que son absence rend la souche qui contient ce polypeptide ou cette protéine, plus sensible aux glycopeptides et que ce polypeptide ou protéine n'est pas présent chez les souches sensibles.

Différents niveaux de résistance aux glycopeptides existent parmi les souches de cocci à Gram-positif notamment.

Selon un mode de réalisation préféré de l'invention, les polypeptides entrant dans la composition ci-dessus définie, correspond à l'association des protéines indentifiées dans la liste des séquences par SEQ ID NO 1 (VanH), SEQ ID NO 2 (VanA), SEQ ID NO 3 (VanX).

Les inventeurs ont donc constaté que l'expression d'une résistance aux glycopeptides chez des bactéries à Gram-positif, nécessite l'expression d'au moins trois protéines ou de polypeptides issus de ces protéines.

Selon un premier mode de réalisation particulier de l'invention, les polypeptides de la composition sont encore caractérisés en ce que les séquences d'acides aminés nécessaires à l'expression de la résistance à des antibiotiques de la famille des glycopeptides sont sous le contrôle d'éléments de régulation, notamment des protéines correspondant aux séquences désignées par SEQ ID NO 4 ou SEQ ID NO 5 dans la liste des séquences,et qui correspondent respectivement à une séquence R de régulation, et à une séquence S senseur.

VanS et VanR constituent un système de régulation à deux composants, VanR étant un activateur de transcription et VanS stimulant la transcription dépendante de VanR. VanS est susceptible de moduler le niveau de phosphorylation de VanR en réponse à la vancomycine présente dans le milieu externe et intervient ainsi dans le contrôle de la transcription des gènes à résistance à la vancomycine.

Ces séquences de régulation sont notamment capables d'augmenter le niveau de résistance, dans la mesure où elles favorisent l'expression des protéines de résistance comprises dans les polypeptides de l'invention.

Selon un autre mode de réalisation avantageux de l'invention, les polypeptides de la composition ci-dessus sont codés par la séquence SEQ ID NO 6 identifiée dans la liste des séquences, qui représente la séquence codante des 5 protéines décrites précédemment.

Une autre séquence selon l'invention est désignée par SEQ ID NO 11 qui contient la séquence SEQ ID NO 6 ainsi qu'un enchaînement en amont de SEQ ID NO 6 codant pour une transposase (codée par le brin (-) de la séquence, et un enchaînement en aval de SEQ ID NO 6 correspondant à des gènes vanY et vanZ et à chaque extrémité, des séquences inversées répétées de 38 pb. SEQ ID NO 11 constitue un transposon dont les gènes interviennent à des niveaux différents dans l'établissement de la résistance aux glycopeptides.

L'invention vise également les protéines purifiées appartenant à la composition et aux polypeptides décrits précédemment. En particulier l'invention vise la protéine purifiée VanA, caractérisée en ce qu'elle correspond à la séquence d'acides aminés SEQ ID NO 2 dans la liste des séquences ou une protéine VanC, codée par un gène capable d'hybrider avec le gène vanA.

La protéine VanA comporte 343 acides aminés et a une masse moléculaire calculée de 37400 Da. La protéine VanC comporte 343 acides aminés et a une masse moléculaire calculée de 37504 Da.

D'autres protéines intéressantes dans le cadre de l'invention correspondent aux séquences identifiées par SEQ ID NO 1 (VanH), SEQ ID NO 3 (VanX), SEQ ID NO 4 (VanR), SEQ ID NO 5 (VanS) dans la liste des séquences.

La séquence indentifiée par l'abréviation SEQ ID NO 1 contient la protéine VanH codée par le gène vanH, cette protéine comporte 322 acides aminés et commence par une méthionine. Cette protéine est une enzyme impliquée la synthèse du peptidoglycane et a une masse moléculaire de 35,754 kDa. VanH présente certaines similitudes avec des déhydrogénases qui catalysent l'oxydation dépendante de NAD⁺ des acides 2-hydroxy-carboxyliques pour former les acides 2-keto-carboxyliques correspondants. En fait la protéine VanH pourrait mettre en oeuvre le NADP⁺ plutôt que le NAD⁺. La protéine VanH contient également plusieurs résidus de sites réactifs qui participent probablement directement dans la liaison du substrat et à la catalyse. VanH serait impliquée dans la synthèse d'un substrat de la ligase VanA. Ce substrat de VanA serait un acide D-α-hydroxy-carboxylique, qui serait condensé par VanA avec la D-alanine à la place d'un acide aminé D, ce qui affecterait la liaison du précurseur du peptidoglycane, avec la vancomycine, par perte d'une liaison hydrogène parce que l'une des liaisons hydrogène formées entre la vancomycine et le N-acétyl-D-Ala-D-Ala est réalisée avec le groupement NH du résidu D-alanine-terminal. Rappelons que "Ala" est l'abréviation de "alanine".

Les inventeurs ont pu mettre en évidence certaines interactions entre les protéines VanA et VanH et ont pu notamment décrire ceci: la nature de la protéine VanA (ligase D-alanine:D-alanine de spécificité altérée pour son substrat) qui a permis la résistance à des glycopeptides implique la biosynthèse d'un nouveau composé différent de D-Ala-D-Ala par VanA, peptide qui peut être incorporé aux peptidoglycanes mais n'est pas reconnu par la vancomycine. En particulier l'observation des similitudes entre le produit du gène vanH avec les α-céto-acides réductases D spécifiques a permis de déterminer que ce composé pouvait ne pas être un acide aminé D mais un acide hydroxy D, qui lorsqu'il était lié avec la D-alanine par VanH pouvait générer un nouveau depsipeptide précurseur du peptidoglycane.

L'invention vise aussi toute combinaison de ces différentes protéines dans un complexe de résistance, ainsi que des protéines hybrides comprenant une ou plusieurs des protéines ci-dessus, ou partie de ces protéines, en association avec une séquence d'acides aminés déterminée.

Entrent également dans le cadre de l'invention, les séquences nucléotidiques codant pour l'une des séquences d'acides aminés décrites ci-dessus.

Une séquence particulière est la séquence nucléotidique de 7,3 kb environ, correspondant au fragment de restriction HindIII-EcoRI, tel qu'obtenu à partir du plasmide pIP816 décrit dans la publication de Leclercq et al - 1988, précitée.

Cette séquence de 7,3 kb comprend l'enchaînement nucléotidique codant pour les 3 protéines de résistance et les 2 protéines de régulation dont il est question ci-dessus. Cette séquence codante est inclue dans un fragment interne BglII-XbaI. Elle comprend aussi une partie des séquences codantes de la transposase et de la résolvase.

L'invention vise aussi tout fragment nucléotidique comprenant le susdit fragment de restriction ainsi que toute partie du fragment HindIII-EcoRI, en particulier le fragment de 3,4 kb environ EcoRI-XbaI codant pour les 3 protéines de résistance ou le fragment de 1,7 kb environ EcoRV-SacII codant pour VanA ou encore le fragment de 3,3 kb environ HindIII-EcoRI codant pour les 2 protéines de régulation VanR et VanS.

Une autre définition d'une séquence nucléotidique de l'invention correspond à un fragment nucléotidique comportant dans l'ordre les sites de restriction suivants, tel qu'obtenu à partir de pIP816 précité :
HindIII, BglII, BglII, EcoRI, BamHI, XbaI, EcoRI

Une autre séquence nucléotidique selon l'invention est caractérisée en ce qu'elle correspond à l'enchaînement choisi parmi les séquences identifiées par SEQ ID NO 7, SEQ ID NO 6, SEQ ID NO il ou SEQ ID NO 22, ou en ce qu'elle comprend cet enchaînement ou toute partie de cet enchaînement, ou encore tout enchaînement ou partie d'enchaînement d'ADN complémentaire ou tout enchaînement d'ARN correspondant à l'un de ces ADN, susceptible,
- soit de constituer une sonde d'hybridation pour la détection d'une résistance à des antibiotiques de la famille des glycopeptides, notamment à la vancomycine et/ou à la teicoplanine, en particulier dans des souches de la famille des cocci à Gram-positif,
- soit de coder pour une séquence nécessaire ou associée à l'expression de la résistance à des antibiotiques de la famille des glycopeptides, notamment la vancomycine et/ou la teicoplanine, en particulier dans des souches de la famille des cocci à Gram-positif.

La séquence SEQ ID NO 7 code pour les 3 protéines de résistance VanH, VanA et VanX.

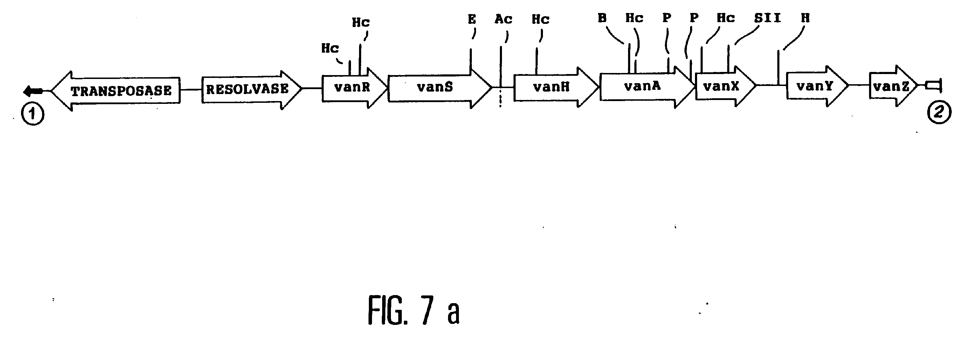

La séquence SEQ ID NO 22 et la séquence SEQ ID NO 11 comprennent un transposon représenté à la figure 7a; ce transposon contient les gènes nécessaires à l'expression de la résistance aux glycopeptides, ainsi que les gènes associés à cette résistance impliqués par exemple dans la régulation de l'expression des gènes nécessaires pour obtenir le phénotype de résistance ou impliqués dans la quantité de polypeptide de résistance obtenue.

Une séquence particulière répondant à la définition ci-dessus est l'une des séquences suivantes : 
V1 : GGX GAA GAT GGX TCX TTX CAA GGX
           G   C     AG  C    G
                         A
 ou

V2 : AAT ACX ATX CCX GGX TTT AC
       C       T           C
               C

V1 et V2 permettent la constitution de sondes le cas échéant en association avec d'autres nucléotides, selon le degré de spécificité recherché pour détecter vanA et vanC et peuvent aussi être utilisées comme amorces dans des réactions de polymérisation en chaîne.

D'autres enchaînements nucléotidiques préférés sont les enchaînements SEQ ID NO 8, SEQ ID NO 9, SEQ ID NO 10, SEQ ID NO 21, SEQ ID NO12(transposase), SEQ ID NO 13(résolvase), SEQ ID NO 14(vanY), SEQ ID NO 15(vanZ), SEQ ID NO 23(vanR), SEQ ID NO 24(vanS) ou d'une variante de l'un de ces enchaînements dès lors qu'elle code pour une protéine ayant des propriétés immunologiques et/ou fonctionnelles similaires à celles des protéines codées par les enchaînements SEQ ID NO 8 SEQ (vanA),ID NO 9 (vanH), SEQ ID NO 10 (vanX), ou SEQ ID NO 21(vanC), SEQ ID NO12(transposase), SEQ ID NO 13(résolvase), SEQ ID NO 14(vanY), SEQ ID NO 15(vanZ), SEQ ID NO 23(vanR), SEQ ID NO 24(vanS) ou en ce qu'elle permet la détection de souches résistantes à des antibiotiques de la famille des glycopeptides.

Des variantes englobent tous les fragments des séquences ayant les propriétés suivantes.

Ces séquences codent pour les protéines de résistance VanH, VanA et VanX.

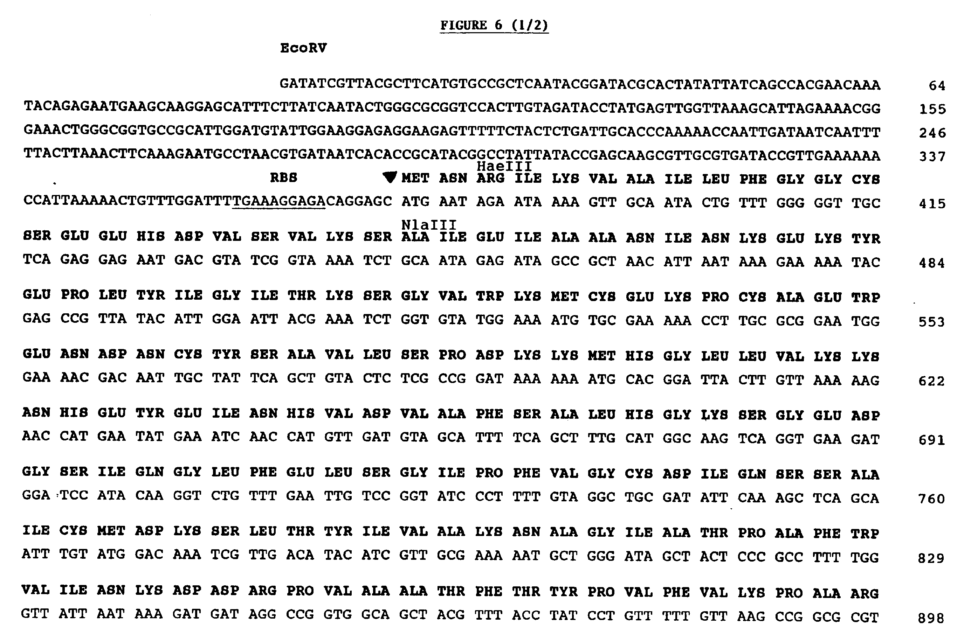

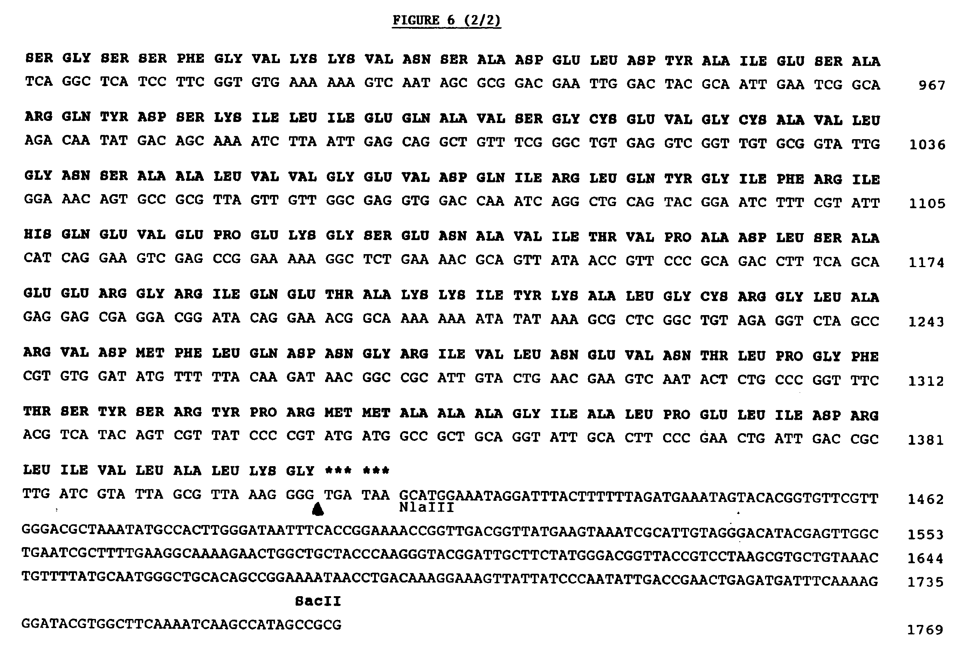

La séquence nucléotidique désignée par SEQ ID NO 8 correspond à un fragment d'ADN de 1029 pb situé entre le codon ATG en position 377 et le codon TGA en position 1406 sur le plasmide pAT214 (Fig. 6).

L'invention vise également une séquence nucléotidique correspondant à l'enchaînement SEQ ID NO 6 correspondant à la séquence codante des 5 protéines (2 protéines de régulation et 3 protéines de résistance), et comprenant également les séquences flanquantes associées à ces séquences codantes, ou comprenant cet enchaînement.

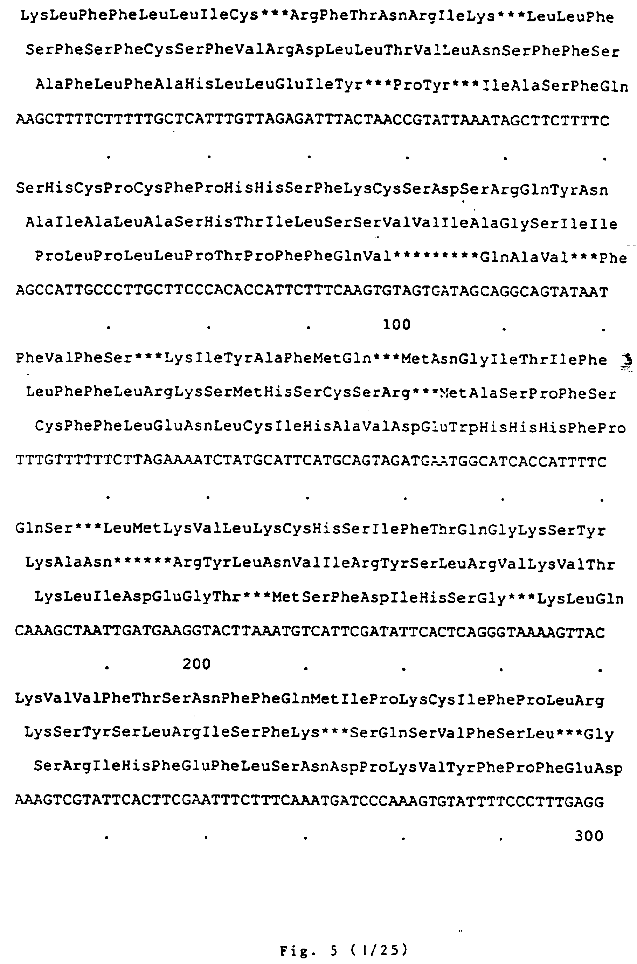

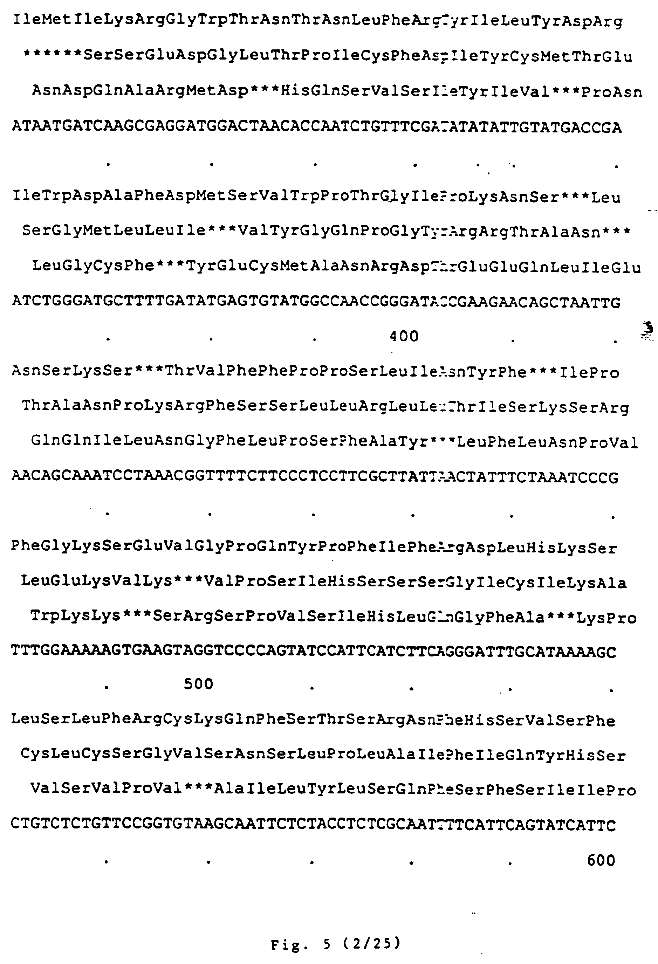

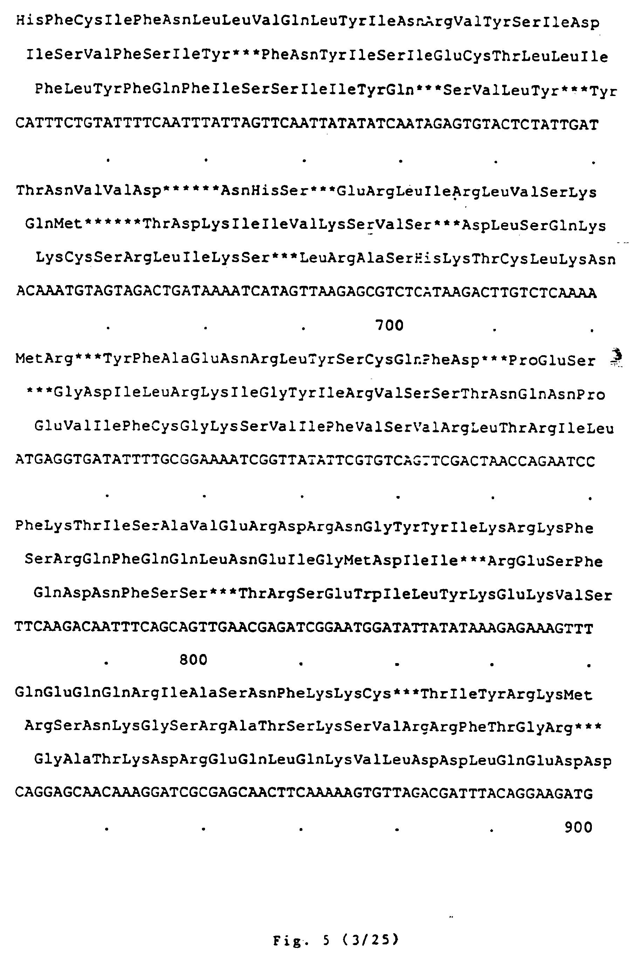

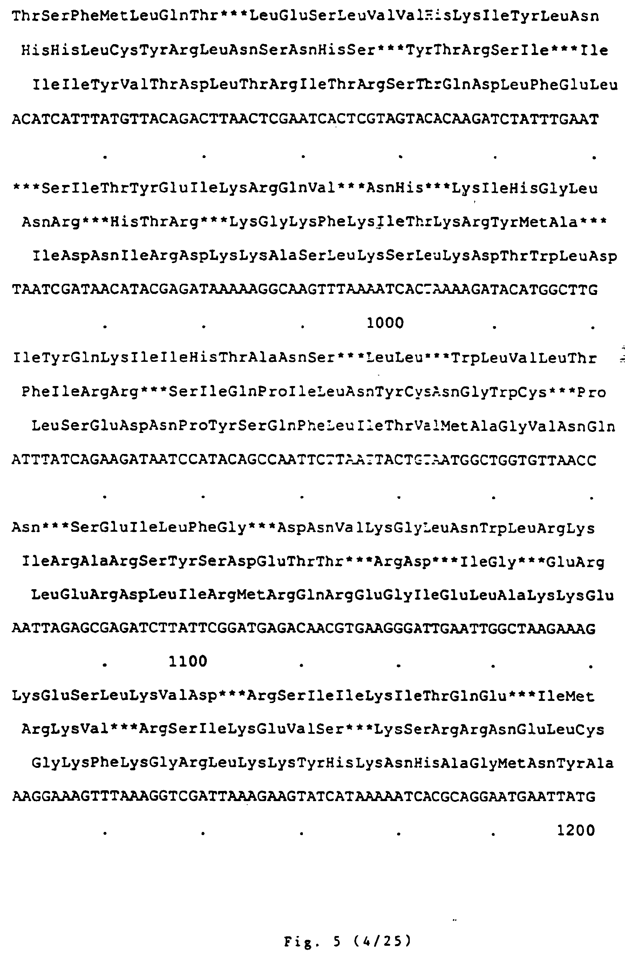

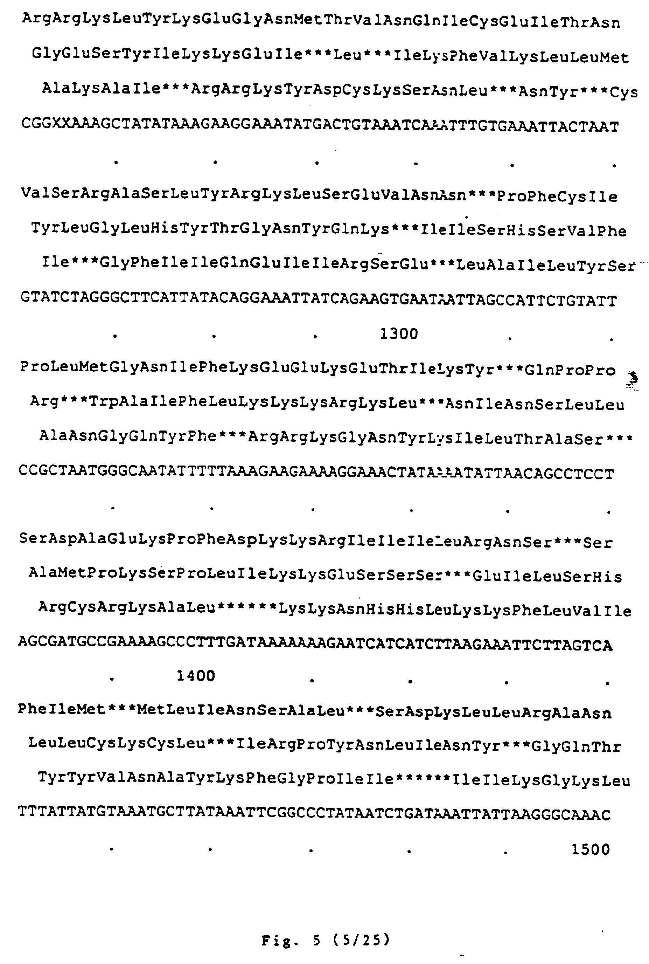

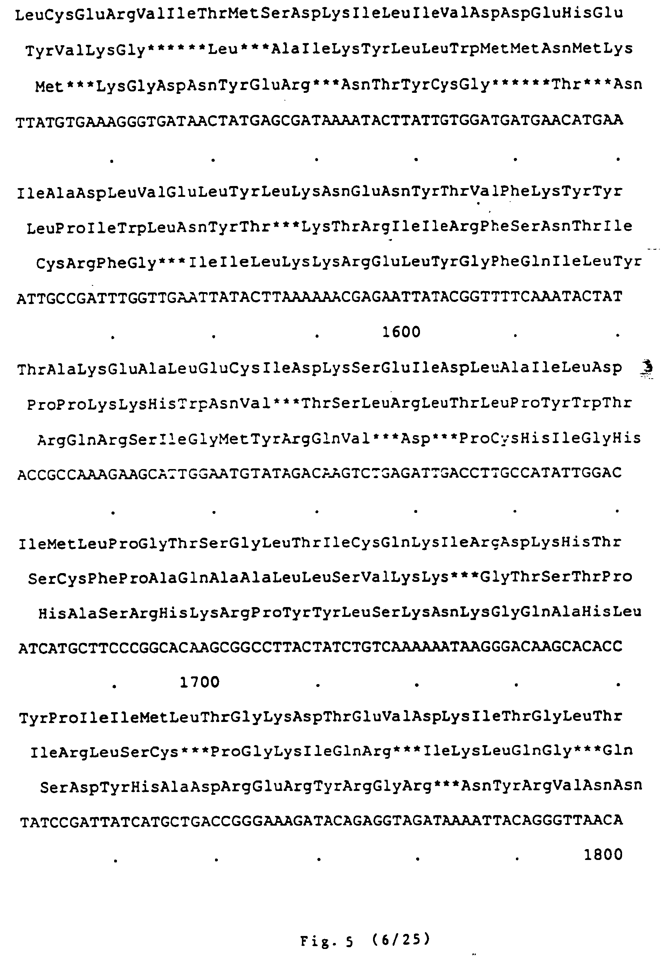

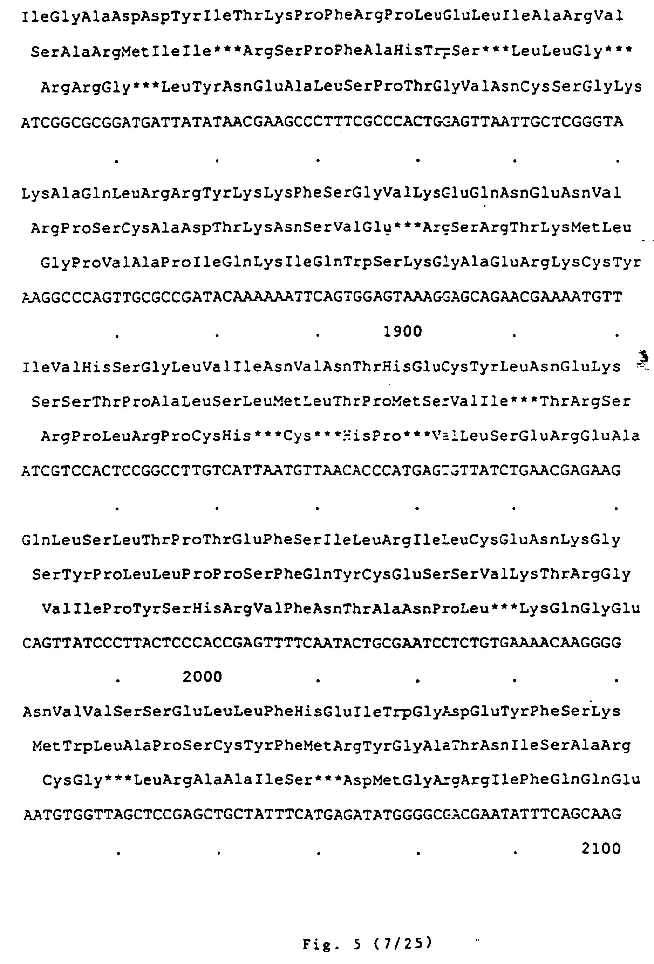

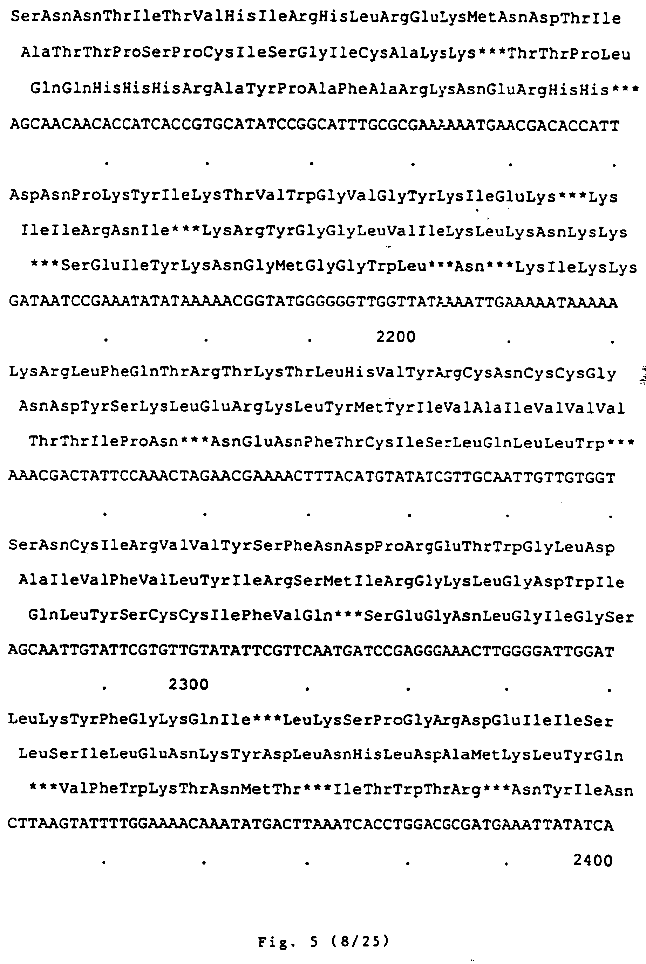

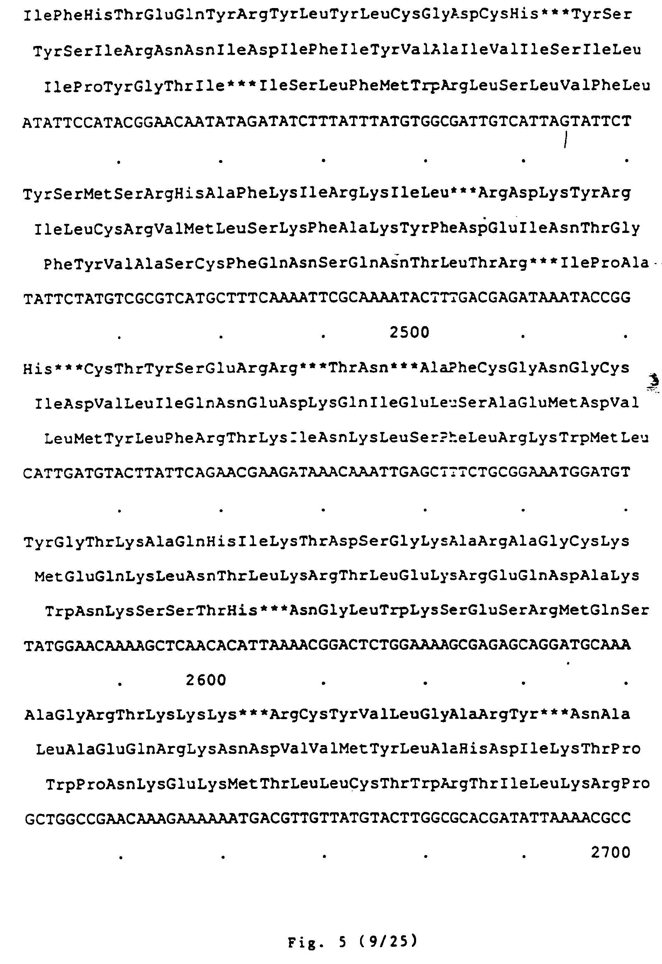

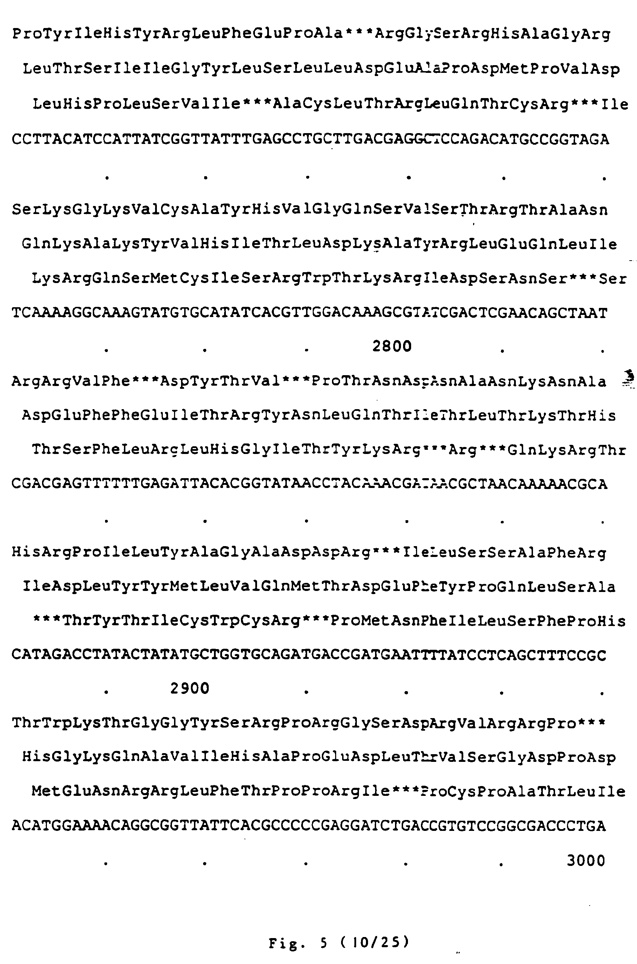

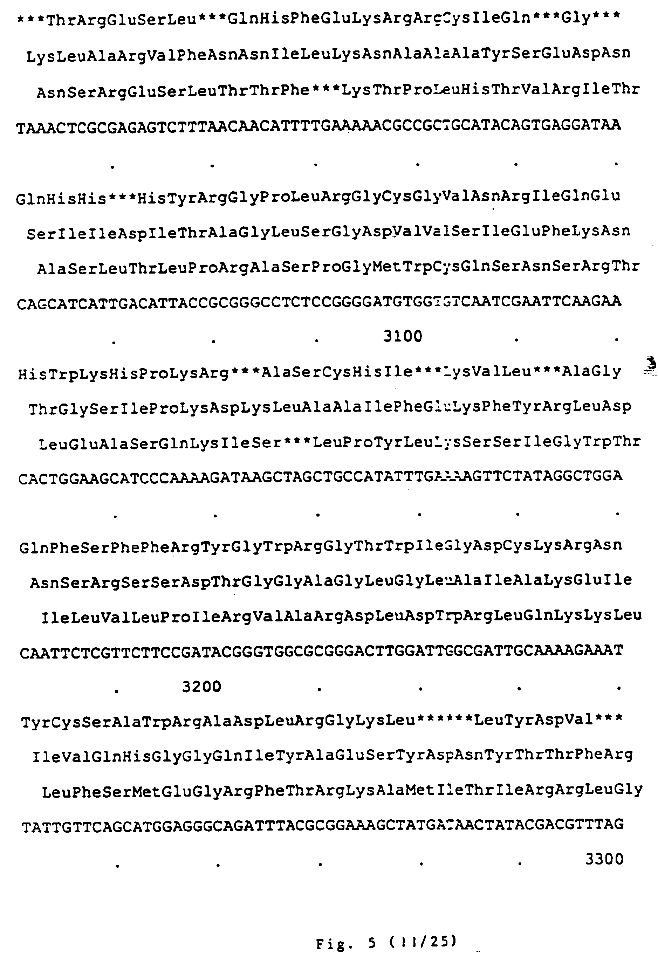

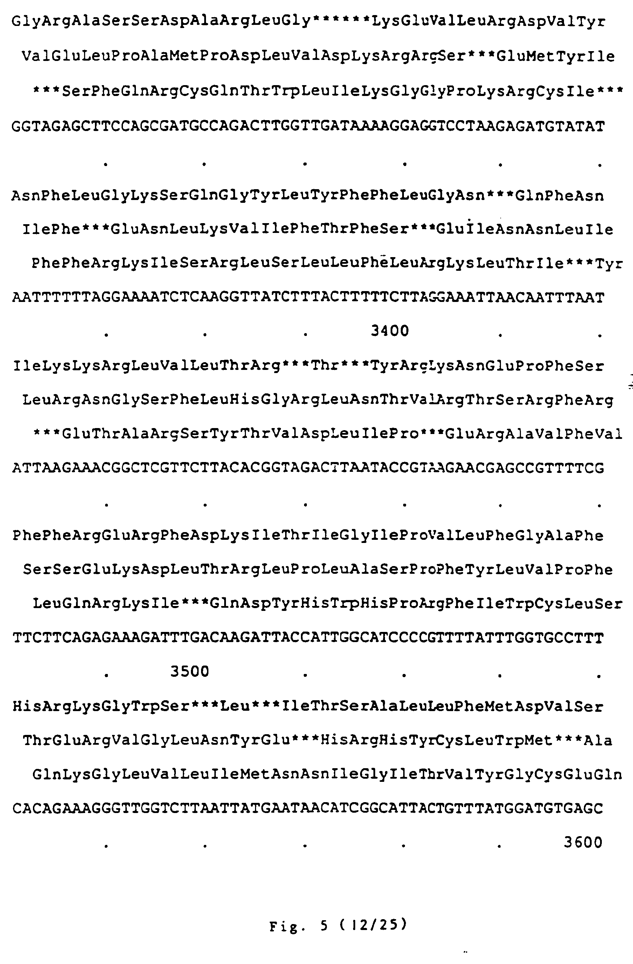

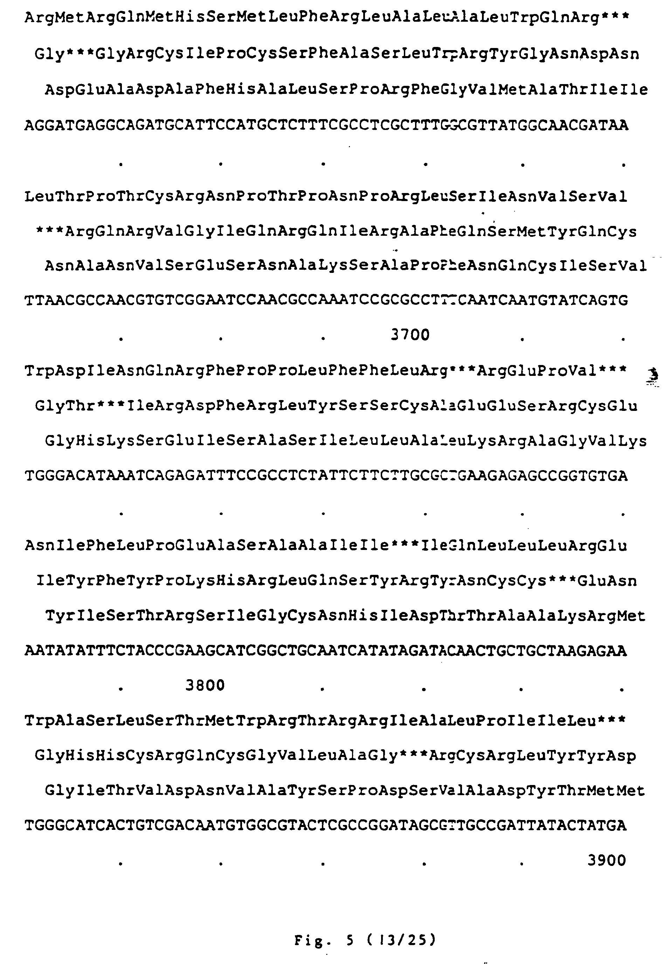

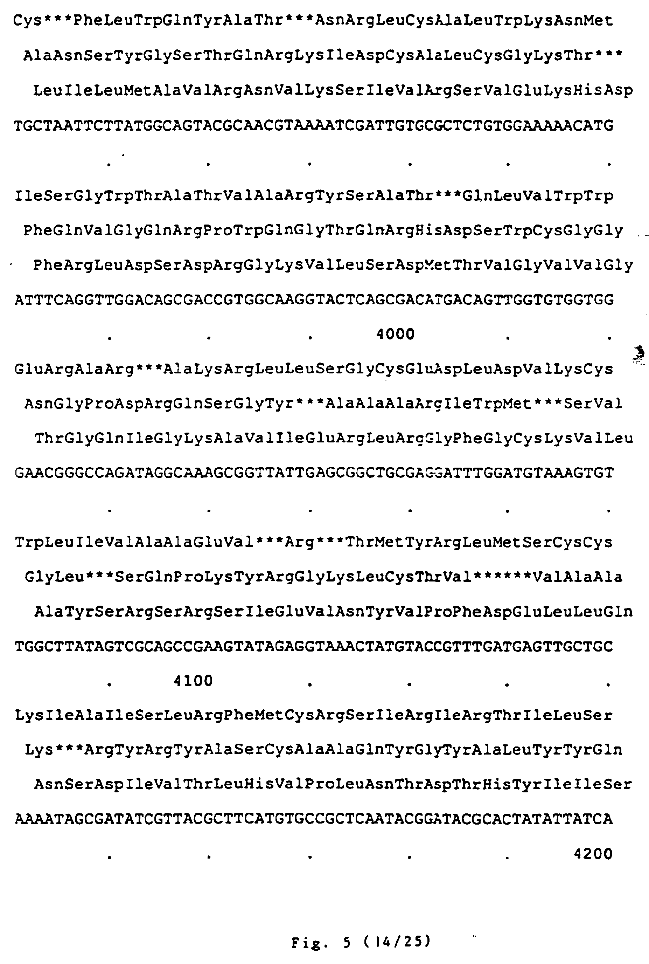

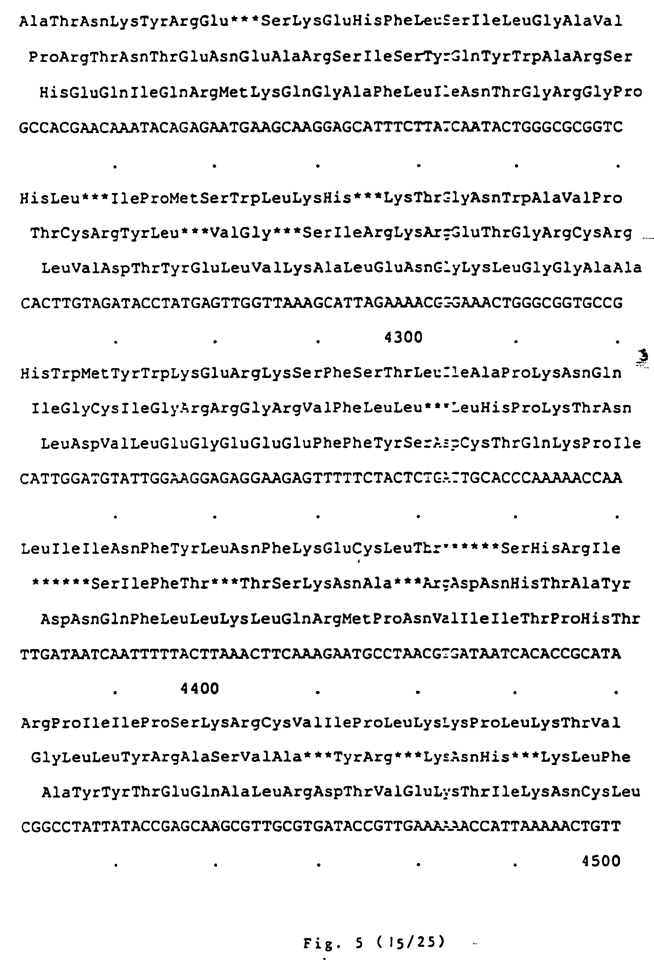

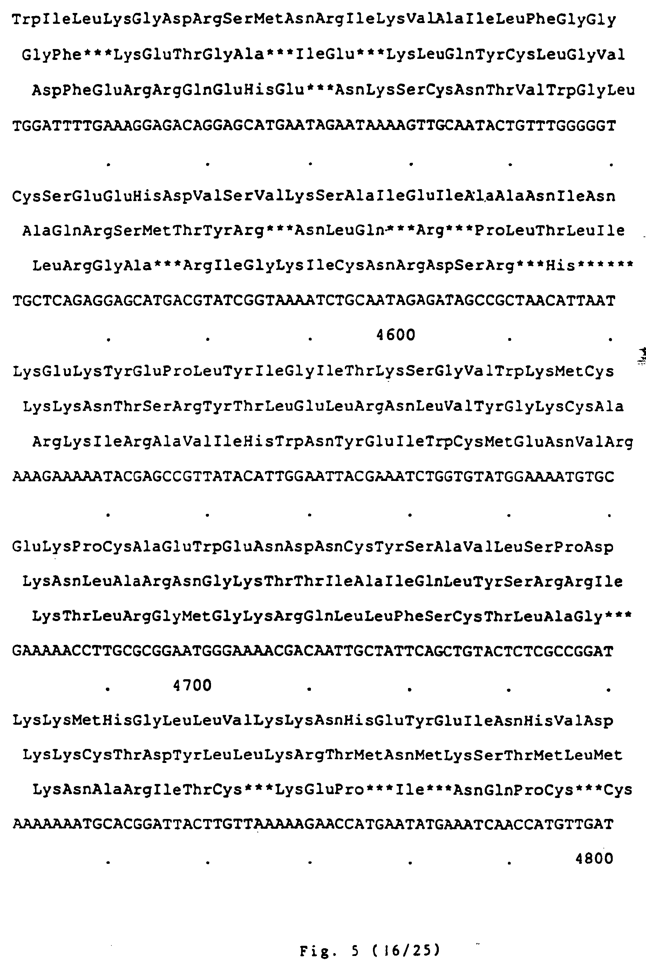

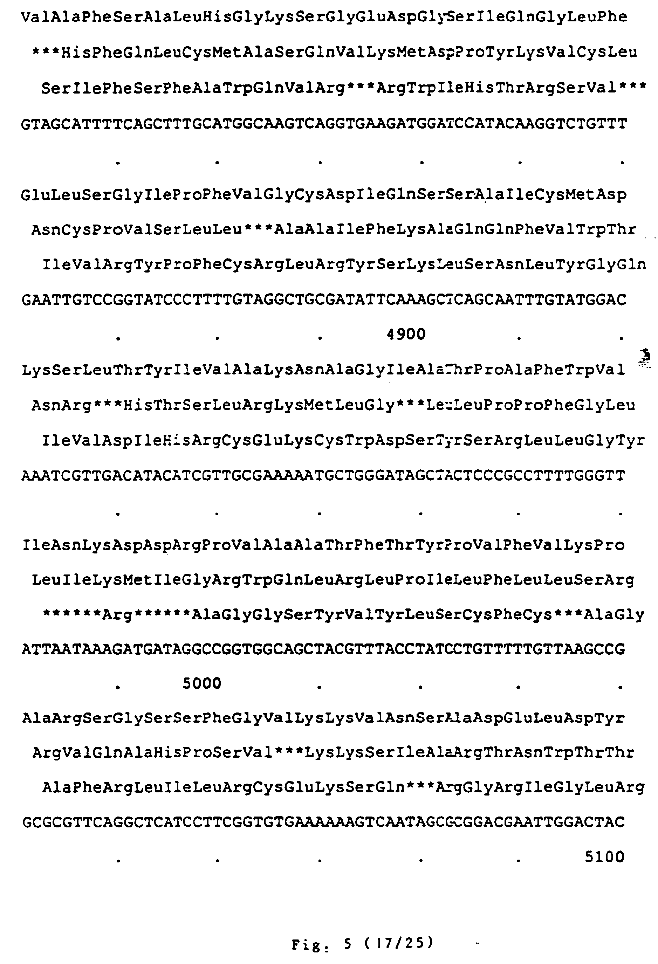

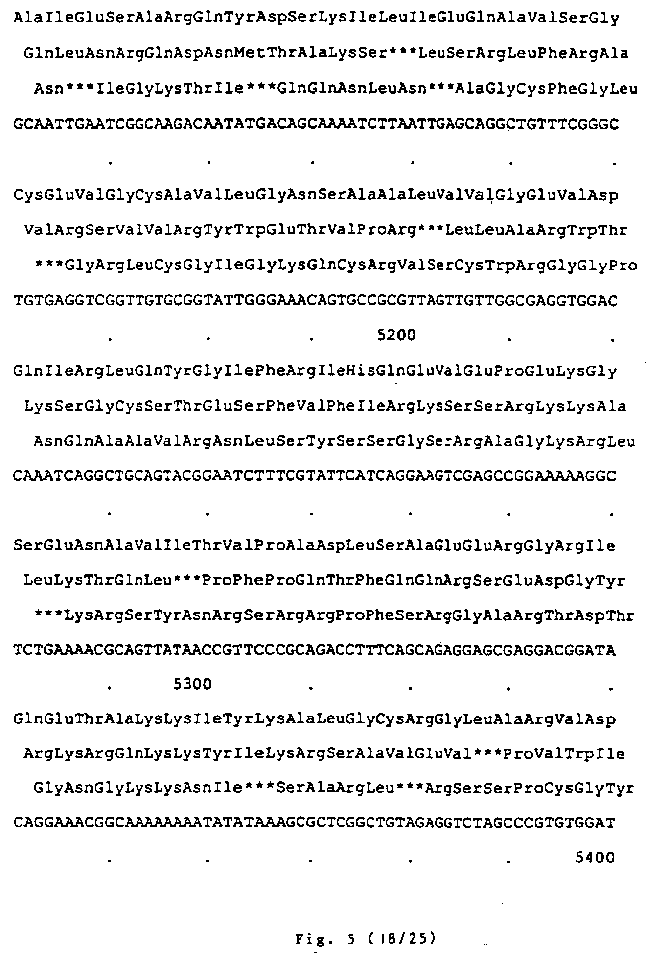

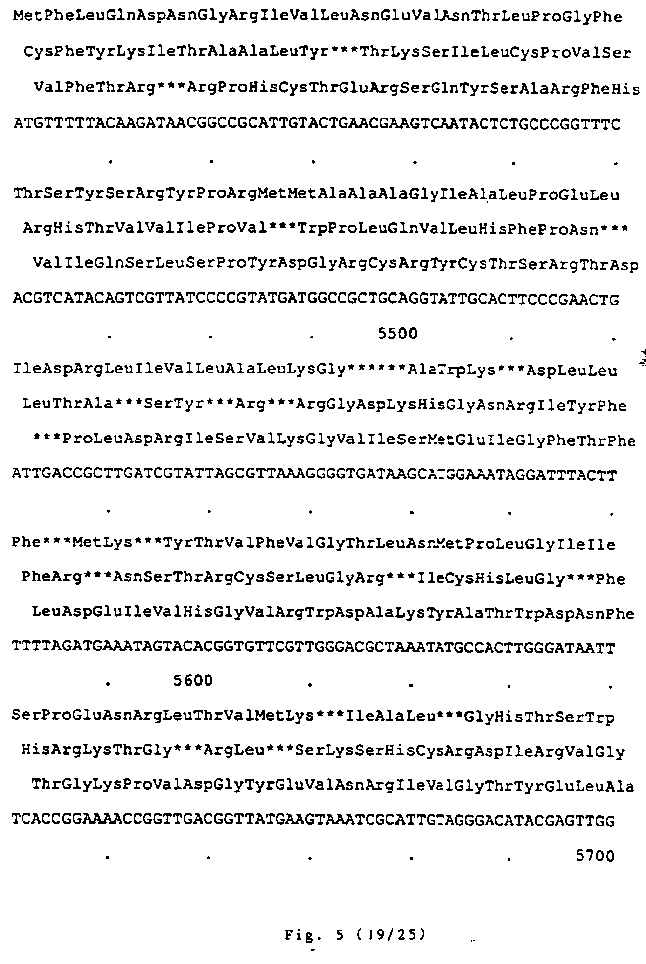

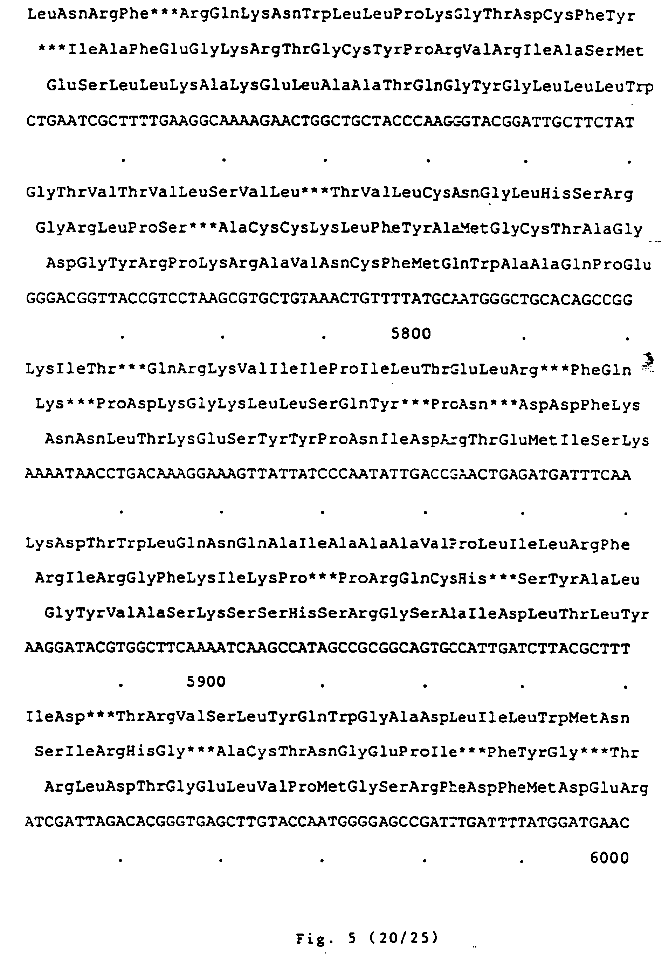

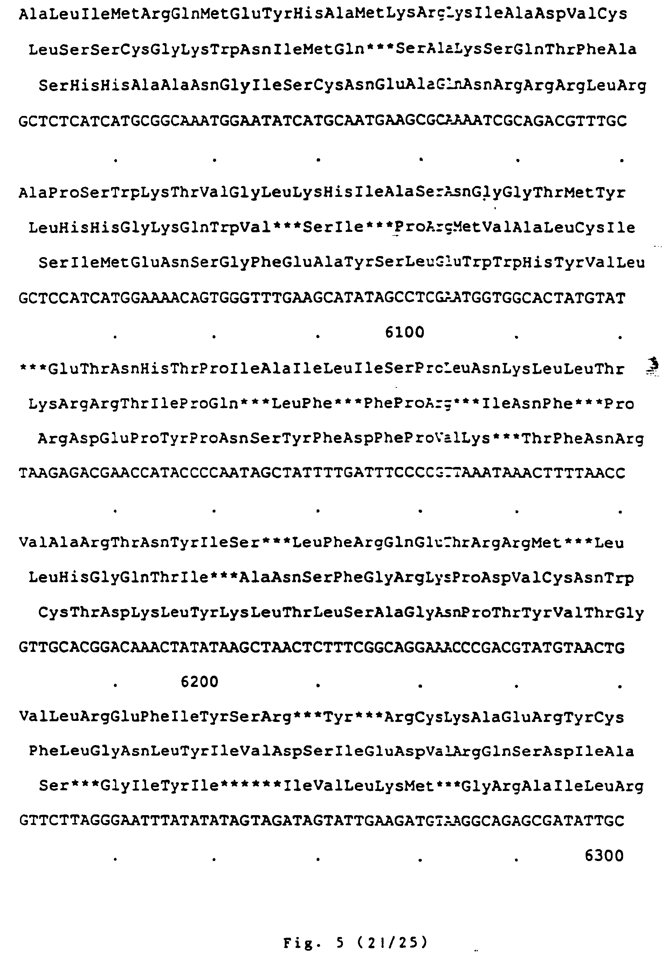

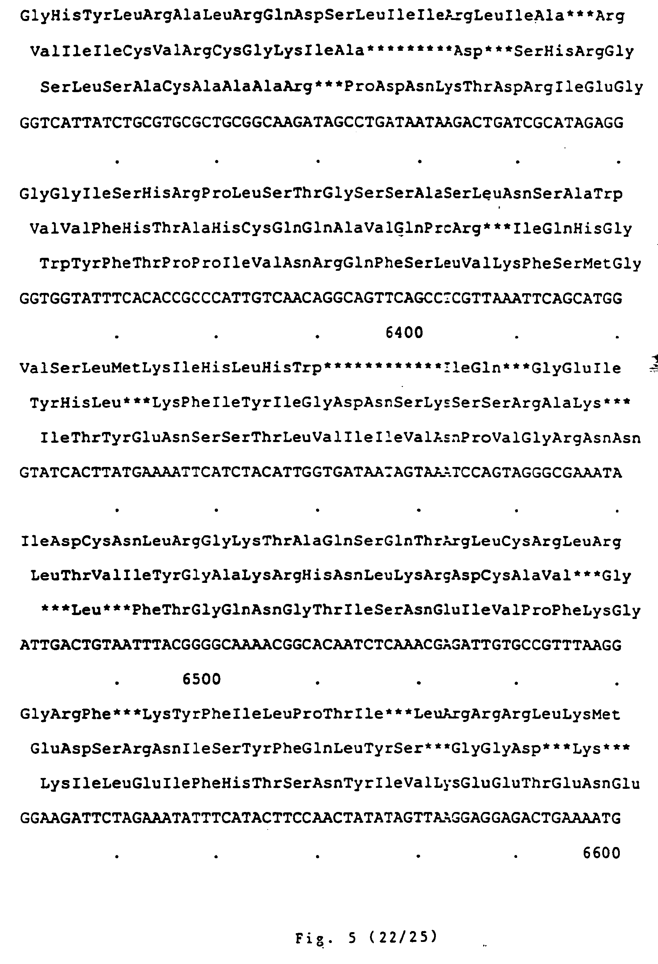

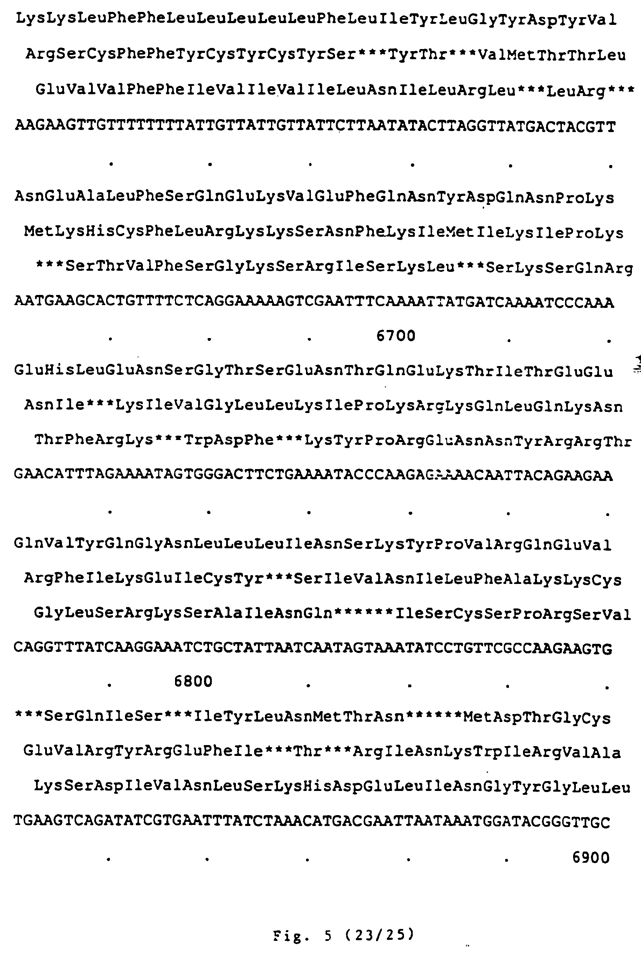

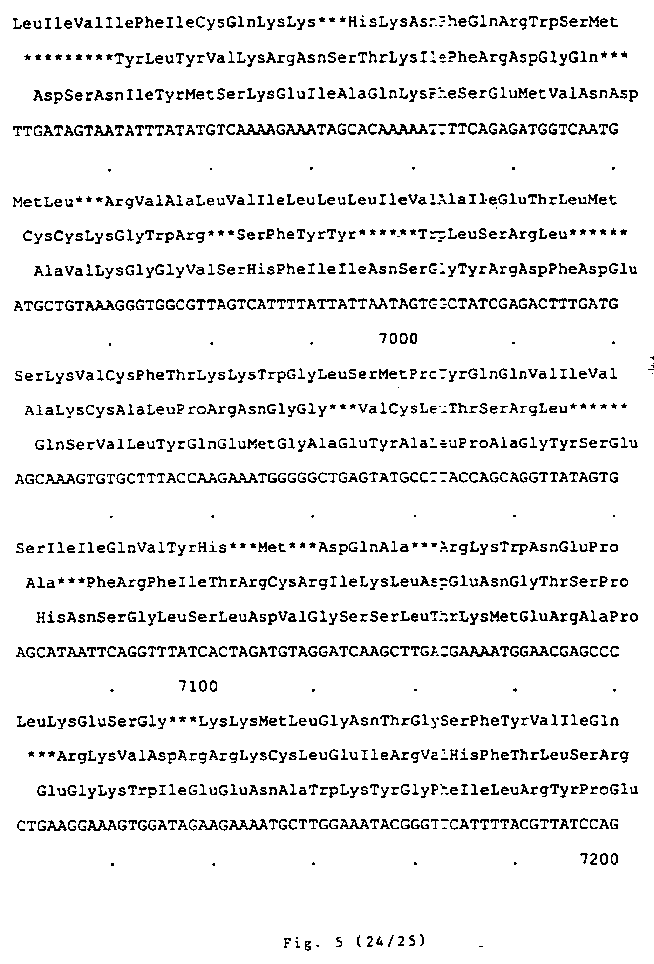

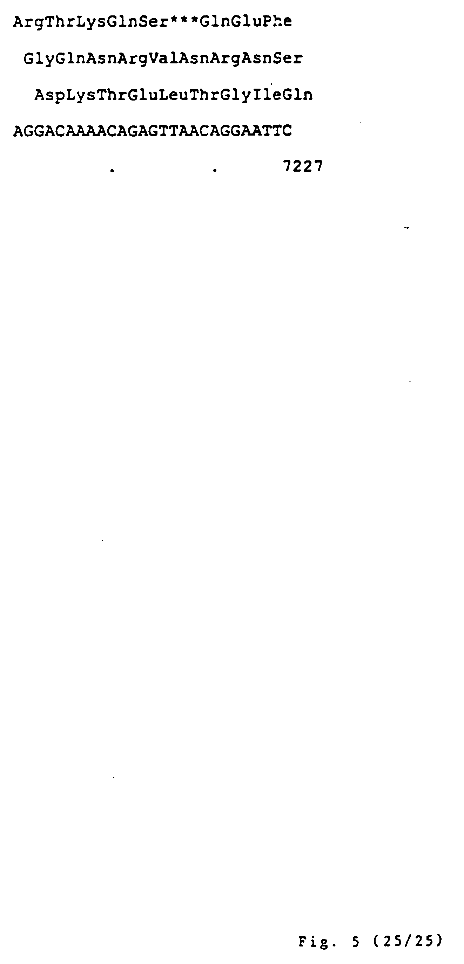

Entre aussi dans le cadre de l'invention une séquence modifiée par rapport à SEQ ID NO 6, caractérisée en ce qu'elle est dépourvue des séquences flanquantes. Ces séquences flanquantes sont les enchaînements représentés dans les pages suivantes et délimitées comme suit:
- enchaînement en amont de la séquence codant pour R: entre les bases 1 et 1476 de la séquence de la figure 5,
- enchaînement entre la séquence codant pour la protéine senseur S et ORF1: entre les bases 3347 et 3500 de la séquence de la figure 5,
- enchaînement en aval de la séquence codante ORF3:entre les bases 6168 et 7227 de la séquence de la figure 5.

La séquence désignée par SEQ ID NO 6 est encore caractérisée par le fragment comportant les sites de restriction dans l'ordre suivant :
BglIII - EcoRI - BamHI - EcoRI

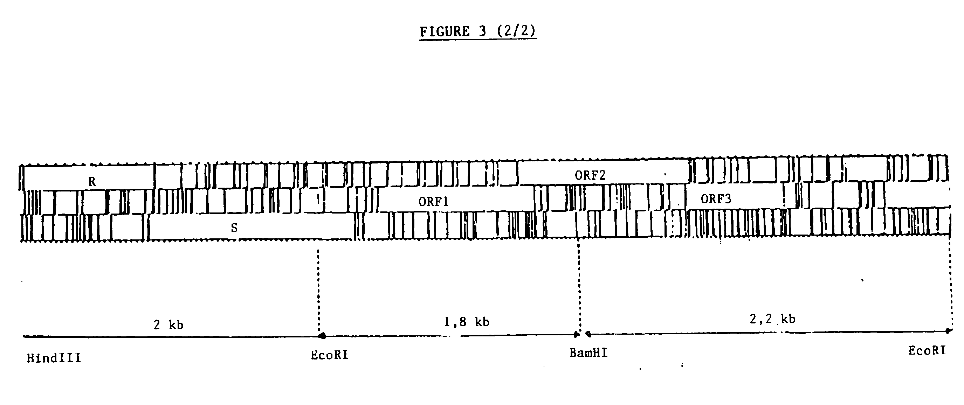

La localisation des protéines de régulation et de résistance est illustrée figure 3.

Les inventeurs ont identifié en amont et en aval des gènes vanR, vanS, vanH, vanA et vanX nécessaires ou associés à l'expression de la résistance à des glycopeptides à un niveau donné, dès gènes codant pour une transposase et une résolvase (en amont du groupe précité) et des gènes vanY et vanZ, en aval de ce groupe. Les gènes de transposase et de résolvase seraient impliqués dans des fonctions de transposition et le gène vanY codant pour une D,D-carboxy peptidase serait impliqué dans le métabolisme du peptidoglycane, et pourrait contribuer à la résistance aux glycopeptides chez E. faecium BM4147 même si vanR, vanS, vanH, vanA et vanX portés par un plasmide à nombre élevé de copies, confèrent seuls une résistance de haut niveau.

Notons que la séquence codante de la transposase est localisée sur le brin (-) de la séquence ID NO 22 qui code pour vanR, vanS, vanH, vanA, vanX, vanY, vanZ et la résolvase.

L'invention vise non seulement les séquences d'ADN identifiées dans la liste des séquences mais aussi les séquences d'ADN complémentaires et les séquences d'ARN correspondantes. L'invention concerne en outre les séquences équivalentes des précédentes, soit en terme d'expression de protéines, de polypeptides ou de leurs fragments décrits plus haut, soit en terme de capacité à détecter, par exemple par les techniques de polymérisation en chaîne des souches de bactéries à Gram-positif, présentant une résistance aux antibiotiques de la famille des glycopeptides tels que la vancomycine ou la teicoplanine.

Font également partie de l'invention, des séquences recombinantes caractérisées en ce qu'elles comprennent l'une des séquences nucléotidiques ci-dessus.

L'invention concerne aussi un vecteur recombinant caractérisé en ce qu'il comprend l'une des séquences nucléotidiques ci-dessus, en un site non essentiel pour sa réplication, sous le contrôle d'éléments de régulation susceptibles d'intervenir dans l'expression, de la résistance à des antibiotiques de la famille des glycopeptides, notamment la vancomycine ou la teicoplanine, chez un hôte déterminé.

Des vecteurs recombinants particulièrement avantageux pour la mise en oeuvre de l'invention sont les vecteurs suivants : pAT214 contenant le fragment EcoRV-SacII de 1761 bp contenant une séquence nucléotidique codant pour la protéine VanA ; dans ces vecteurs les séquences de l'invention sont avantageusement placées sous le contrôle de promoteurs tels que le promoteur lac.

L'invention vise aussi un hôte cellulaire recombinant comprenant une séquence nucléotidique telle que décrite précédemment ou un vecteur décrit ci-dessus, dans des conditions permettant l'expression de la résistance à des antibiotiques de la famille des glycopeptides, notamment la résistance à la vancomycine ou/et à la teicoplanine, cet hôte étant par exemple choisi parmi les bactéries, notamment les cocci à Gram-positif.

Pour certaines applications on peut aussi utiliser les levures, les champignons, les cellules d'insectes ou de mammifères.

L'invention vise également une sonde nucléotidique caractérisée en ce qu'elle est capable d'hybrider avec une séquence décrite précédemment, cette sonde étant le cas échéant marquée. Ces sondes peuvent être ou non, spécifiques des protéines de résistance aux glycopeptides.

Des marqueurs utilisables pour les besoins de l'invention sont les marqueurs radioactifs connus ainsi que d'autres marqueurs tels que les marqueurs enzymatiques ou des marqueurs chémoluminescents.

Des sondes ainsi marquées peuvent être utilisées dans des tests d'hybridation pour détecter une résistance aux glycopeptides chez des bactéries à Gram-positif. Dans ce cas, on pourra mettre en oeuvre des conditions de faible stringence.

Des sondes nucléotidiques selon l'invention peuvent être caractérisées en ce qu'elles sont spécifiques chez des bactéries à Gram-positif des séquences codant pour une protéine de résistance à des glycopeptides notamment à la vancomycine et/ou à la teicoplanine, ces sondes pouvant en outre être universelles parmi ces séquences.

Par ces sondes spécifiques, on entend tout oligonucléotide hybridant avec une séquence nucléotidique codant pour l'une des protéines selon l'invention, telle que décrite dans les pages précédentes, et ne présentant pas de réaction d'hybridation croisée ou d'amplification (PCR) avec des séquences présentes chez l'ensemble des souches sensibles.

Le caractère universel des oligonucléotides utilisables en PCR, est déterminé par leur capacité de promouvoir spécifiquement l'amplification d'une séquence nucléotidique impliquée dans la résistance chez une souche quelconque de bactérie à Gram-positif, résistante aux antibiotiques de la famille des glycopeptides.

La taille des sondes nucléotidiques selon l'invention peut varier en fonction de l'utilisation recherchée. Pour les oligonucléotides utilisables en PCR on aura recours à des fragments de longueur usuelle dans cette technique. Pour réaliser des sondes, on peut prendre toute partie des séquences de l'invention, par exemple des sondes fragments de 200 nucléotides.

Selon un mode de réalisation particulier de l'invention, une sonde nucléotidique est choisie pour sa spécificité vis à vis d'une séquence nucléotidique codant pour une protéine nécessaire à l'expression chez des bactéries à Gram-positif d'un haut niveau de résistance à des antibiotiques de la famille des glycopeptides, en particulier à la vancomycine et à la teicoplanine.

A titre d'exemple des sondes intéressantes pourront être choisies dans la partie intragénique du gène vanA.

D'autres sondes avantageuses pour la réalisation de l'invention, sont caractérisées par leur caractère universel, selon la définition précédente, mais non spécifique des gènes de résistance. Elles peuvent être utilisées aussi comme amorces de PCR, et sont par exemple :

V1 : GGX GAA GAT GGX TCX TTX CAA GGX
           G   C     AG  C     G
                         A

V2 : AAT ACX ATX CCX GGX TTT AC
       C     T             C
             C

V1 et V2 hybrident avec vanA et vanC et sont susceptibles de conduire à la détection chez d'autres micro-organismes, de protéines associées à la résistance aux glycopeptides.

D'autres sondes particulières de l'invention ont le caractère spécifique d'une séquence nucléotidique codant pour une protéine nécessaire à l'expression chez des bactéries à Gram-positif d'un bas niveau de résistance à des antibiotiques de la famille des glycopeptides, en particulier à la vancomycine chez des bactéries à Gram-positif.

On mentionne également que des sondes oligonucléotidiques qui seraient issues de la séquence du gène vanA codant pour la protéine VanA peuvent être utilisées pour rechercher indifféremment une résistance de haut niveau ou de bas niveau.

D'une façon particulièrement préférée, on caractérise une sonde de l'invention en ce qu'elle hybride, avec une séquence nucléotidique chromosomique ou non chromosomique d'une souche à Gram-positif résistante à des glycopeptides, notamment la vancomycine et/ou la teicoplanine, en particulier en ce qu'elle hybride avec une séquence nucléotidique chromosomique ou non chromosomique d'une souche de cocci à Gram-positif, par exemple une souche d'entérocoque et de préférence E. faecium 4147 ou E. gallinarum.

Pour différencier des souches à haut niveau de résistance, de souches à bas niveau de résistance on pourra réaliser un test d'hybridation en mettant en oeuvre des conditions de forte stringence.

Les oligonucléotides de l'invention peuvent être obtenus à partir des séquences de l'invention, par coupure avec des enzymes de restriction, ou par synthèse chimique selon les méthodes classiques.

L'invention vise par ailleurs des anticorps polyclonaux ou monoclonaux, caractérisés en ce qu'ils reconnaissent le ou les polypeptides décrits ci-dessus ou une séquence d'acides aminés décrite ci-dessus.

Ces anticorps peuvent être obtenus selon les méthodes classiques de production d'anticorps. En particulier pour la préparation des anticorps monoclonaux on aura recours à la méthode de Kolher et Milstein selon laquelle on prépare des anticorps monoclonaux par fusion cellulaire entre des cellules de myélome et des cellules spléniques de souris préalablement immunisées avec un polypeptide ou une composition selon l'invention, conformément au procédé classique.

Les anticorps de l'invention sont avantageusement utilisables pour la détection de la présence de protéines caractéristiques d'une résistance aux glycopeptides, en particulier à la vancomycine et à la teicoplanine.

Des anticorps particulièrement intéressants sont les anticorps polyclonaux ou monoclonaux dirigés contre la protéine VanA ou VanC. De tels anticorps permettent avantageusement de détecter des souches de bactéries, en particulier des cocci à Gram-positif présentant une résistance à haut niveau vis à vis des antibiotiques de la famille des glycopeptides. Le cas échéant, une étape de lyse préalable des cellules de l'échantillon soumis à la détection, est mise en oeuvre préalablement à la mise en contact de l'échantillon avec les anticorps.

Pour réaliser cette détection, on aura avantageusement recours à des anticorps marqués par exemple par une substance radioactive ou autre.

Entrent donc dans le cadre de l'invention, des tests pour la détection chez les bactéries à Gram-positif, d'une résistance aux glycopeptides, notamment des tests mettant en oeuvre les techniques ELISA.

Un kit pour le diagnostic in vitro de la présence de souches à Gram-positif, résistantes aux glycopeptides, en particulier à la vancomycine et/ou à la teicoplanine, ces souches appartenant en particulier aux cocci à Gram-positif par exemple des entérocoques, par exemple E. faecium ou E. gallinarum, caractérisé en ce qu'il comprend :
- des anticorps répondant à la définition ci-dessus, le cas échéant marqués,
- un réactif pour la détection d'une réaction immunologique du type anticorps-antigène,
- le cas échéant des réactifs pour effectuer la lyse de cellules de l'échantillon testé.

Les moyens mis au point par les inventeurs présentent par ailleurs l'avantage tout à fait intéressant d'être adaptés pour la réalisation d'un test ou d'un kit rapide et fiable de détection de souches à Gram-positif, résistantes aux glycopeptides par la réaction de polymérisation en chaîne (PCR). Un tel test permet d'améliorer la sensibilité des tests existants qui restent peu fiables et peut permettre dans certains cas la détection de l'ensemble des représentants de la famille des gènes codant pour des protéines de résistance aux glycopeptides chez des bactéries à Gram-positif.

La réalisation d'un test par la méthode d'amplification des gènes de ces protéines est faite par la technique PCR ou par la technique IPCR (IPCR : abréviation de réaction de polymérisation en chaîne inverse).

La technique IPCR permet l'amplification des régions NH₂ et COOH terminales des gènes que l'on souhaite détecter.

Certaines amorces particulières permettent d'amplifier les gènes des souches résistantes à bas niveau. Ces amorces sont par exemple choisies dans la séquence codant pour la protéine de résistance VanA.

A titre d'exemple les séquences suivantes sont utilisables comme amorces pour la préparation de sondes pour la détection d'une amplification par la méthode PCR ou IPCR.

V1 : GGX GAA GAT GGX TCX TTX CAA GGX
           G   C     AG  C     G
                         A

V2 : AAT ACX ATX CCX GGX TTT AC
       C      T            C
              C

X représente l'une des bases A,T,C ou G ou encore correspond dans tous les cas à l'inosine.

L'invention vise naturellement les sondes complémentaires des oligonucléotides précédemment décrits ainsi que éventuellement les sondes ARN qui leur correspondent.

Un kit pour le diagnostic in vitro de la présence de souches de bactéries à Gram-positif, résistantes aux glycopeptides, en particulier résistantes à la vancomycine et/ou à la teicoplanine, ces souches appartenant en particulier aux cocci à Gram-positif, notamment en ce qu'il s'agit de souches d'entérocoques, par exemple E. faecium ou E. gallinarum, est caractérisé en ce qu'il comprend :
- une sonde nucléotidique répondant aux définitions ci-dessus et le cas échéant,
- des oligonucléosides triphosphates en quantité suffisante pour permettre l'amplification de la séquence recherchée,
- un tampon d'hybridation,
- un agent de polymérisation d'ADN.

L'invention vise aussi un procédé de détection in vitro de la présence de souches à Gram-positif, résistantes aux glycopeptides, en particulier à la vancomycine et/ou à la teicoplanine, ces souches appartenant en particulier à la famille des cocci à Gram-positif, notamment en ce qu'il s'agit de souches d'entérocoques, par exemple E. faecium ou E.gallinarum, caractérisé en ce qu'il comprend :
a) la mise en contact d'un échantillon biologique susceptible de contenir les souches résistantes, avec une amorce constituée par une séquence nucléotidique ci-dessus décrite, ou toute partie d'une séquence précédemment décrite, capable d'hybrider, avec une séquence nucléotidique recherchée nécessaire à l'expression de la résistance aux glycopeptides, cette séquence étant utilisée comme matrice, en présence des 4 différents nucléosides triphosphates, et d'un agent de polymérisation, dans des conditions d'hybridation telles que pour chaque séquence nucléotidique ayant hybridé avec une amorce, un produit d'élongation de chaque amorce complémentaire de la matrice est synthétisé,
b) la séparation de la matrice et du produit d'élongation obtenu, ce dernier pouvant alors également se comporter comme une matrice,
c) la répétition de l'étape a) de façon à obtenir une quantité détectable des séquences nucléotidiques recherchées,
d) la détection du produit d'amplification des séquences nucléotidiques.

La détection des produits d'élongation de la séquence recherchée peut être réalisée par une sonde identique aux amorces mises en oeuvre pour effectuer la technique PCR ou IPCR, ou encore par une sonde différente de ces amorces, cette sonde étant le cas échéant marquée.

Des détails concernant la mise en oeuvre des techniques PCR, peuvent être obtenus à partir des demandes de brevets EP 0229701 et EP 0200362.

D'autres avantages et caractéristiques de l'invention apparaissent dans les exemples qui suivent et dans les figures.

### FIGURES

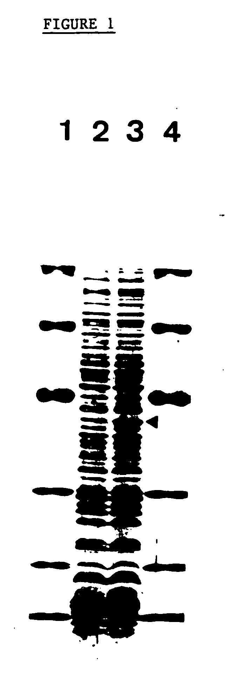

- Figure 1 : électrophorèse sur gel de polyacrylamide SDS (SDS-PAGE) des protéines des fractions membranaires lignes 1 et 4, standards de poids moléculaires ; ligne 2, E. faecium BM4147 mis en culture en l'absence de vancomycine; ligne 3, BM4147 mis en culture avec 10 *µ* g/ml de vancomycine. La tête de flèche indique la position de la protéine VANA.
- Figure 2 :
   A : Cartes de restriction des insérats des plasmides pAT213 et pAT214. Le vecteur et l'insérat d'ADN sont distingués par des segments clairs et sombres respectivement. La flèche ouverte représente le gène vanA.
   B : Stratégie pour le séquençage nucléotidique pour l'insérat de 1761 bp dans le plasmide pAT214. Les flèches indiquent la direction et l'étendue des réactions de séquençage par la méthode didéoxy. L'amorce synthétique d'oligonucléotides
      (5' ATGCTCCTGTCTCCTTTC 3' OH) est complémentaire de la séquence entre les positions 361 et 378. Seuls les sites de restriction pertinents sont donnés.
- Figure 3 : position des séquences R,S,ORF1,ORF2,ORF3.

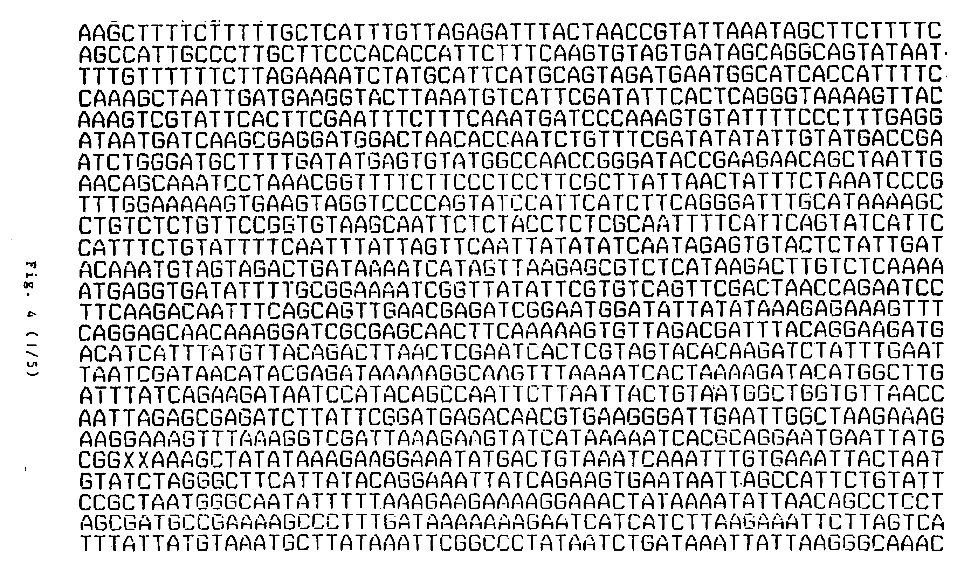

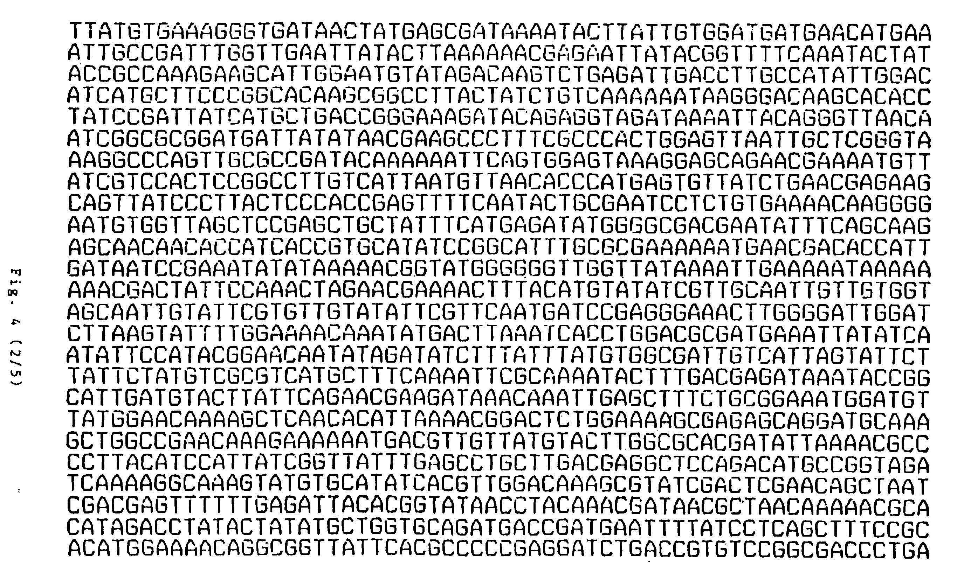

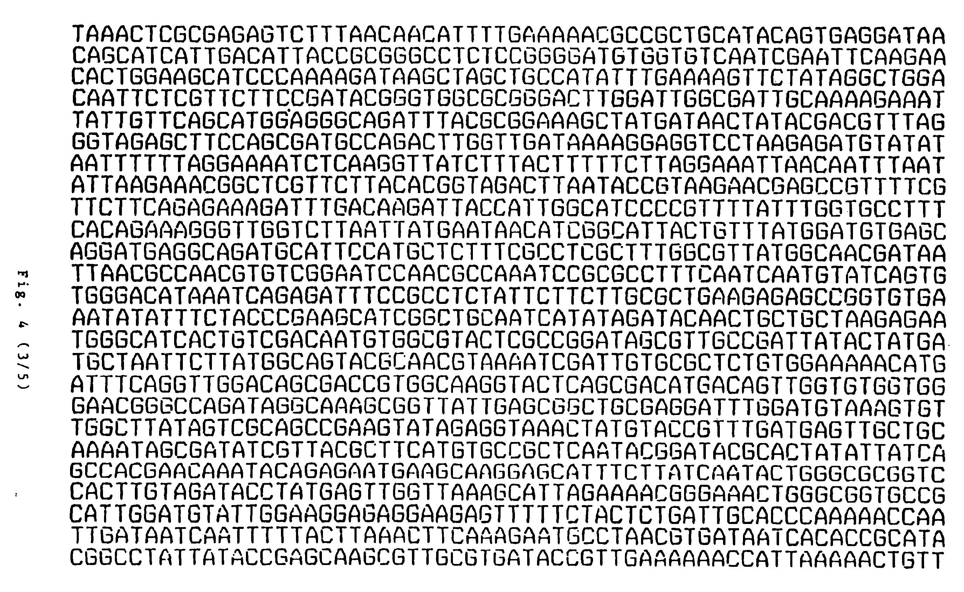

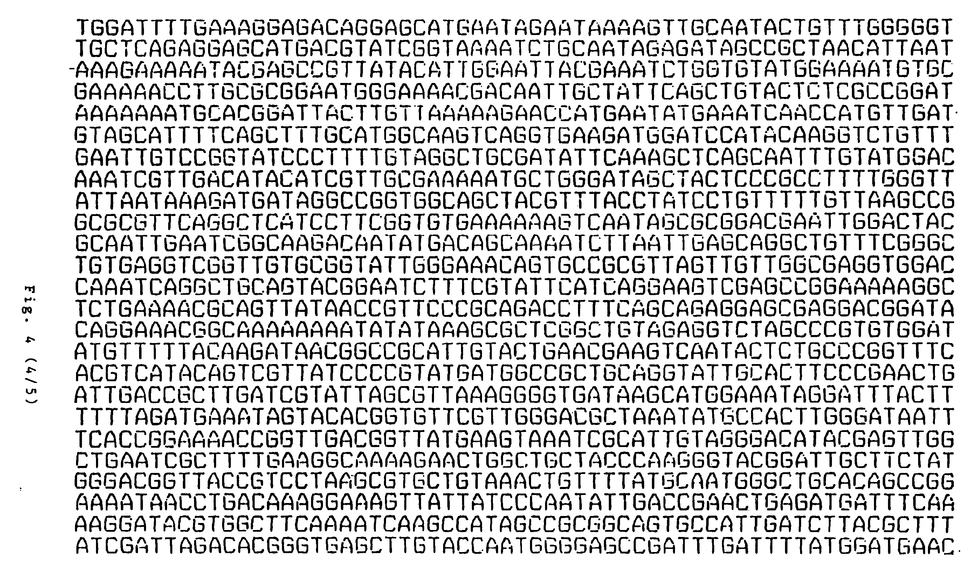

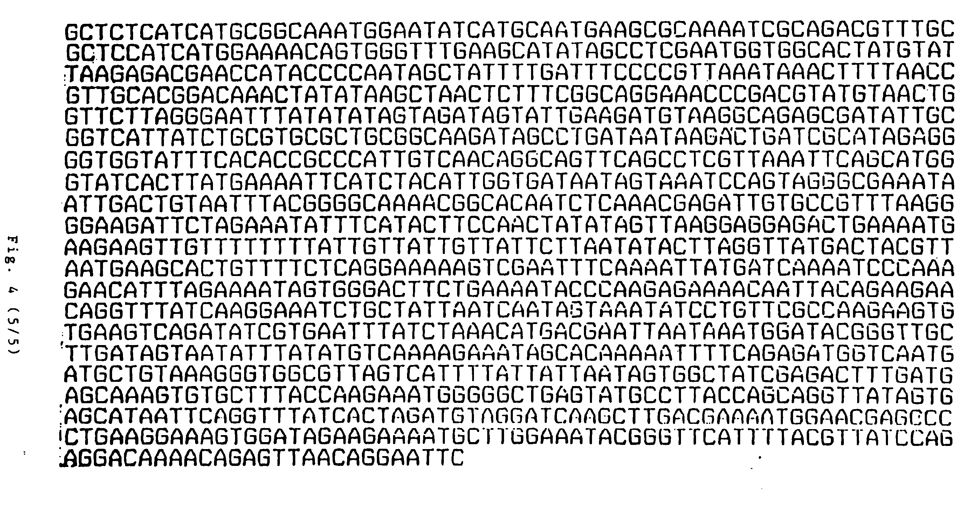

- Figure 4 : représentation de SEQ ID N0 6
- Figure 5 : représentation de SEQ ID N0 6 et de la protéine correspondante.
- Figure 6 : séquence du gène vanA et la protéine correspondante.
- Figure 7 :
   (a) : Localisation des gènes vanR, vanS, vanH, vanA, vanX, vanY, vanZ, du gène de la transposase et du gène de la résolvase, ainsi que des séquences terminales inversées répétées de 38 bp à l'extrémité du transposon.
   (b) : Cartographie des plasmides (A) Polylinker de pAT29 et des dérivés construits dans cette étude. La flèche marquée P2 indique la position et l'orientation du promoteur P2 de aphA-3 (Caillaud et al, 1987, Mol. Gen. Genet. 207:509-513). (B) Insérat de pAT80. Les rectangles blancs indiquent l'ADN de pAT29 mais ne sont pas représentés à l'échelle. Les rectangles se terminant par une flèche indiquent les séquences codantes. Les flèches en traits pleins, verticales et horizontales indiquent respectivement la position et l'orientation du gène apha-1 dans les dérivés de pAT80. sites de rstriction : Ac, AccI ; B, BamHI ; Bg, BglII ; Bs, BssHII ; E, EcoRI ; H, HindIII ; Hc, HincII ; K, KpnI ; P, PstI ; S, SmaI ; SI, SacI, SII, SacII ; Sa, SalI ; Sp, SphI ; Xb, XbaI. (C) Insérats dans pAT86, pAT87, pAT88 et pAT89. Les insérats sont représentés par des traits pleins et les vecteurs correspondants sont indiqués entre parenthèses.

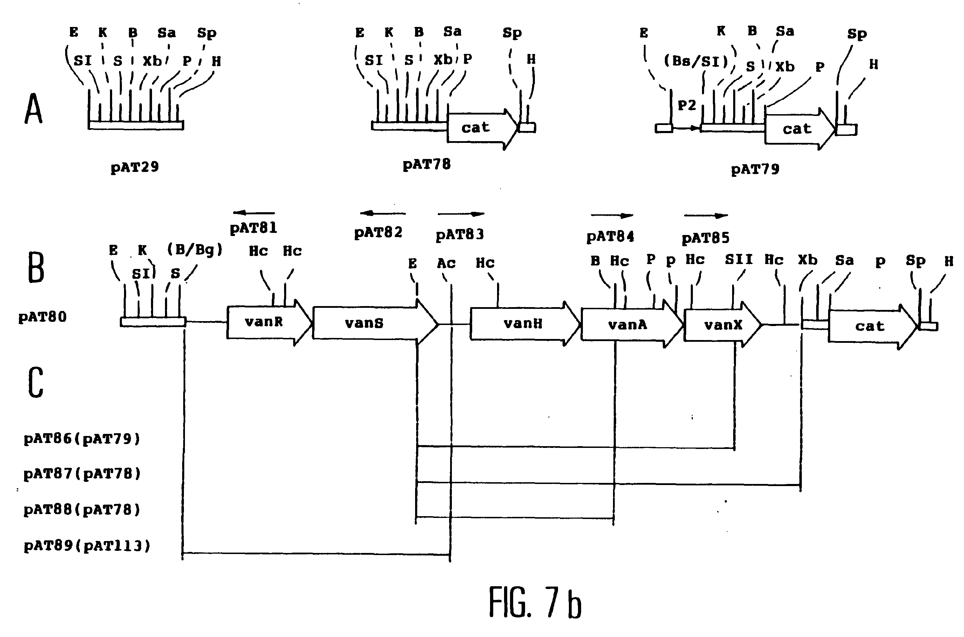

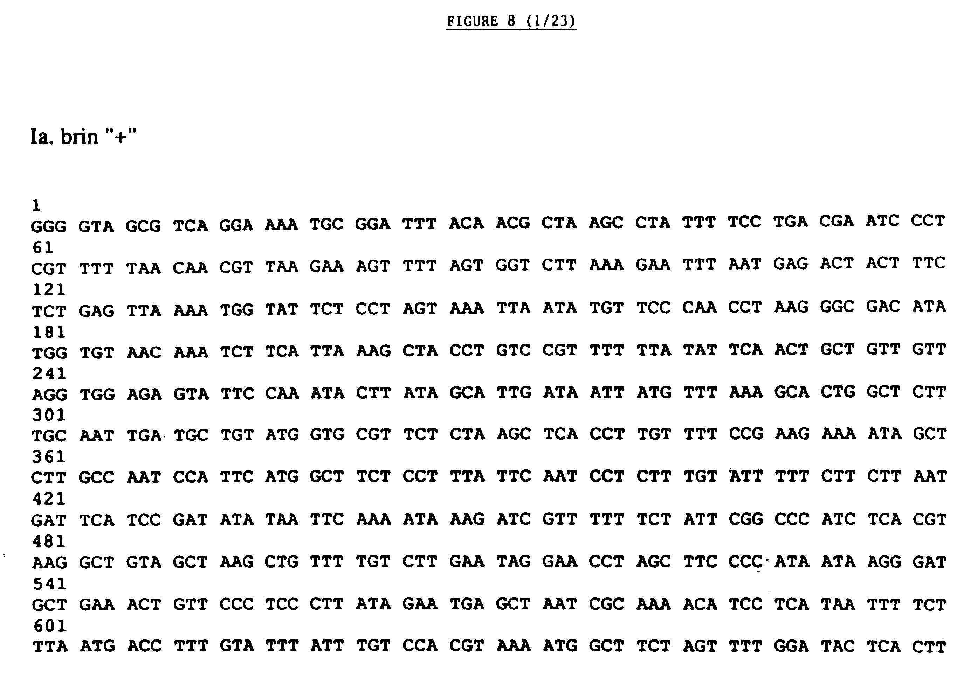

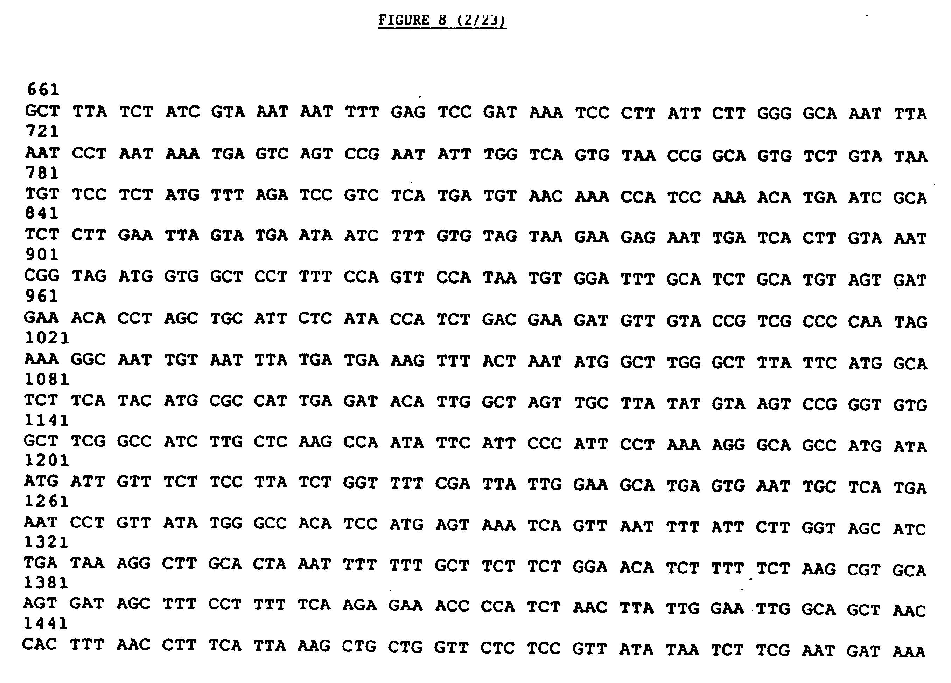

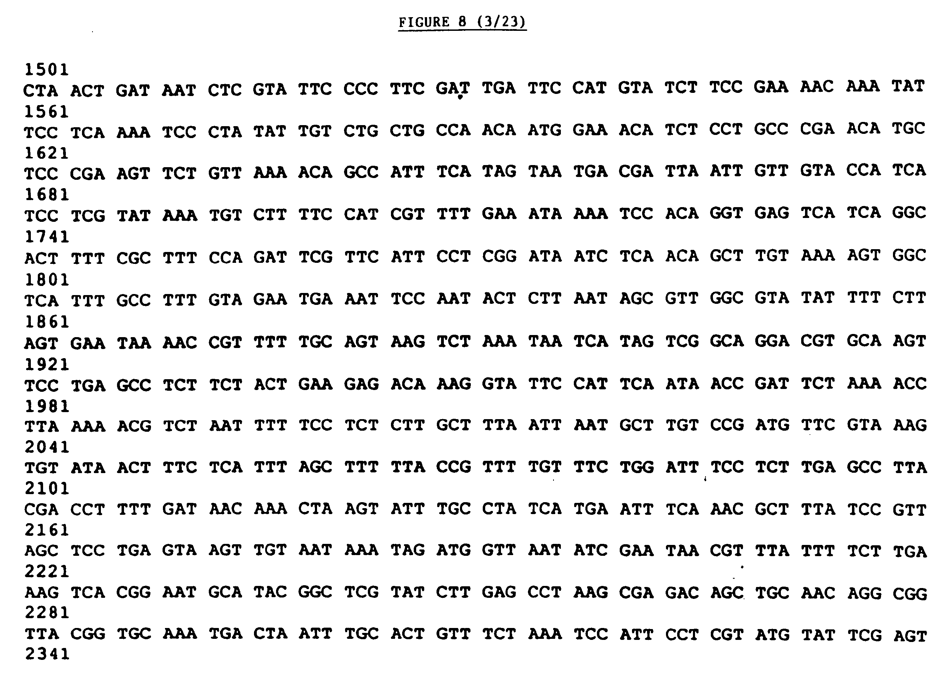

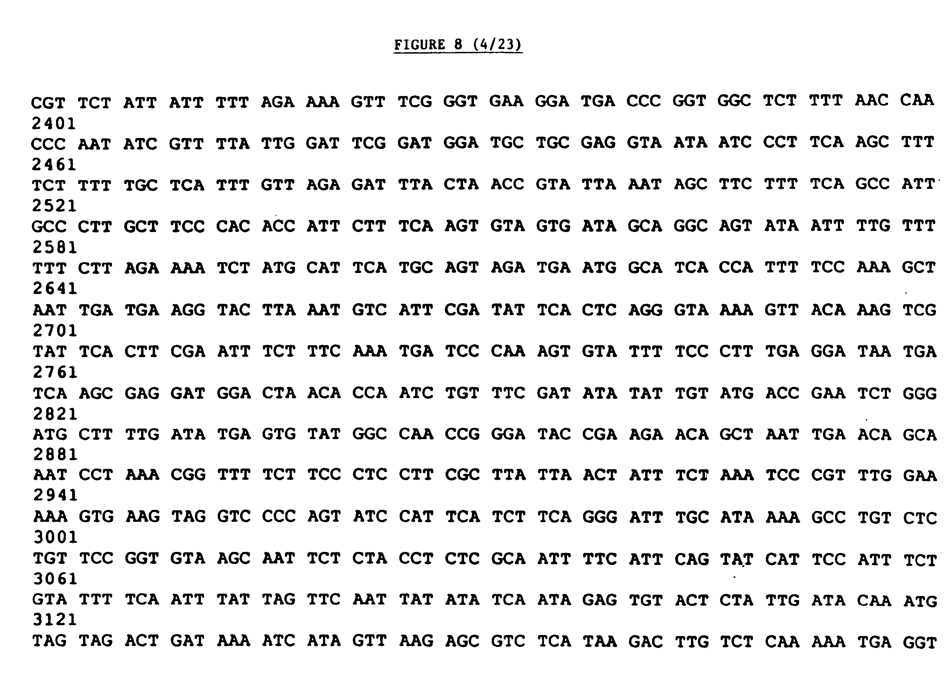

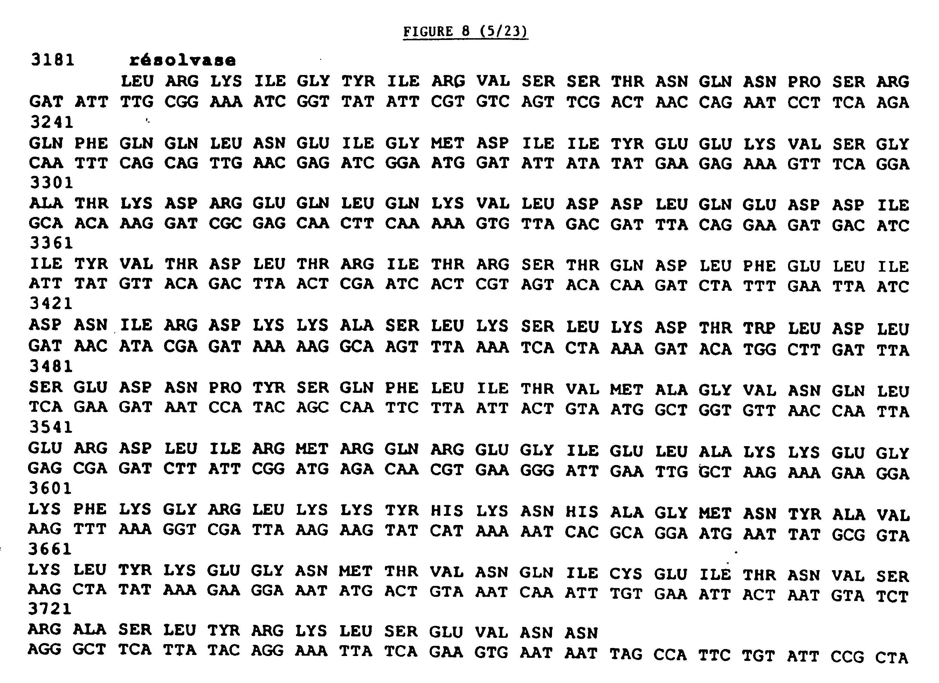

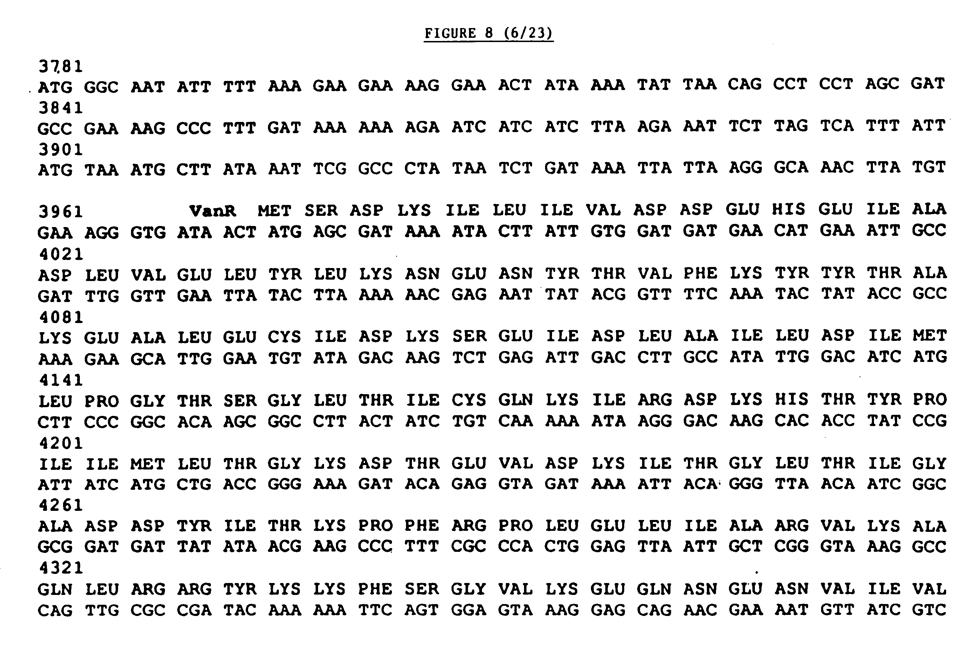

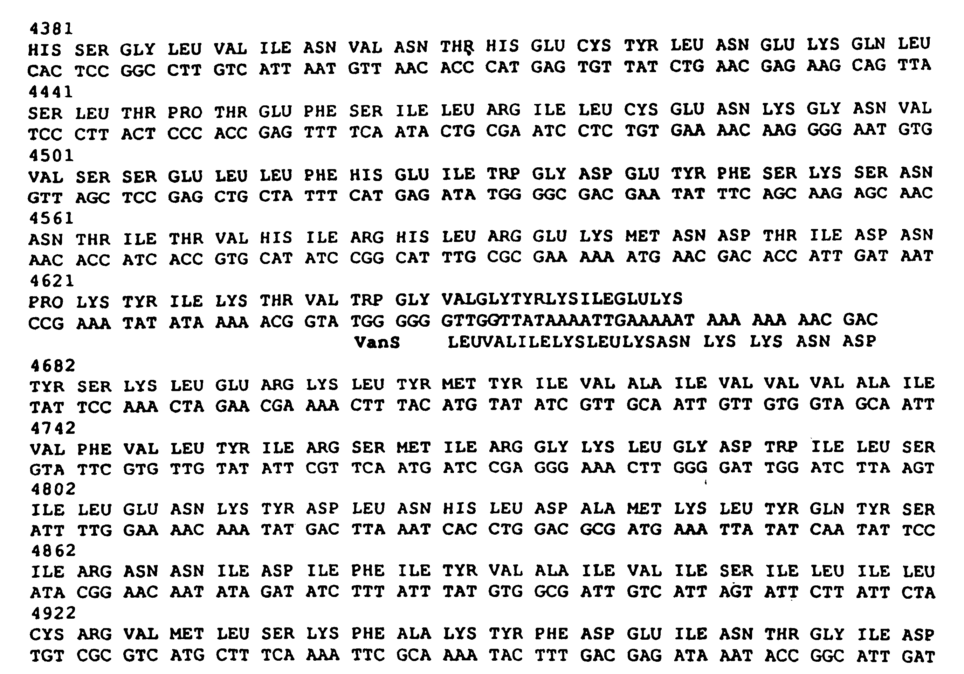

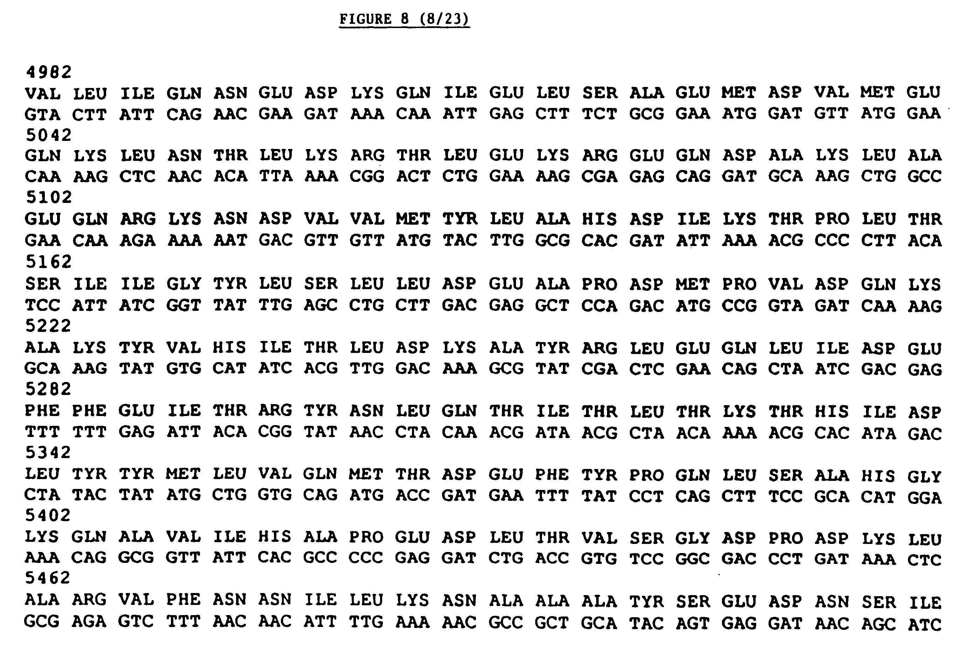

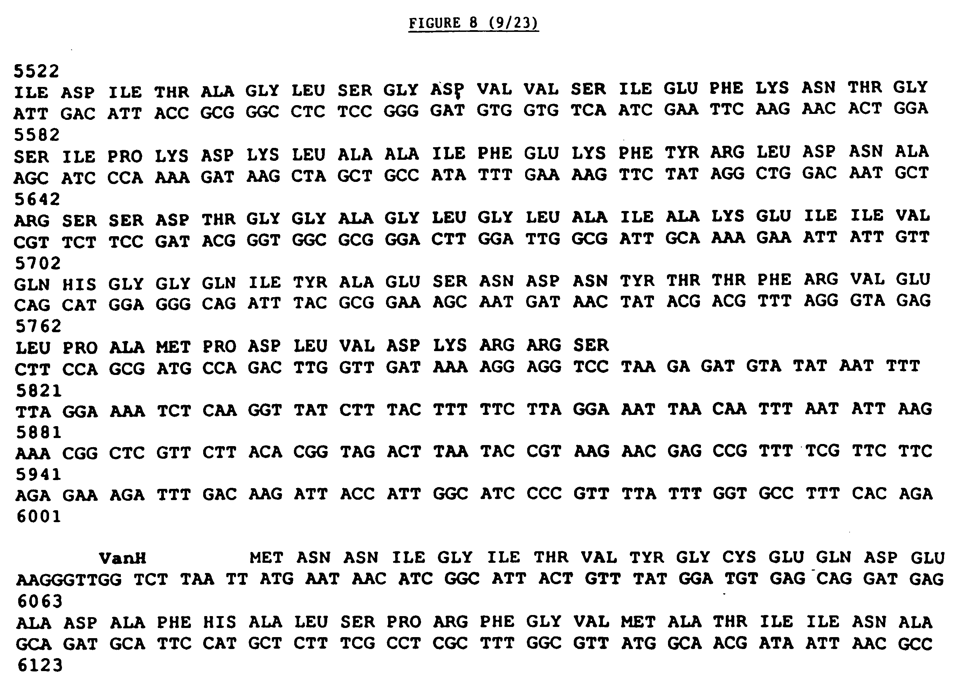

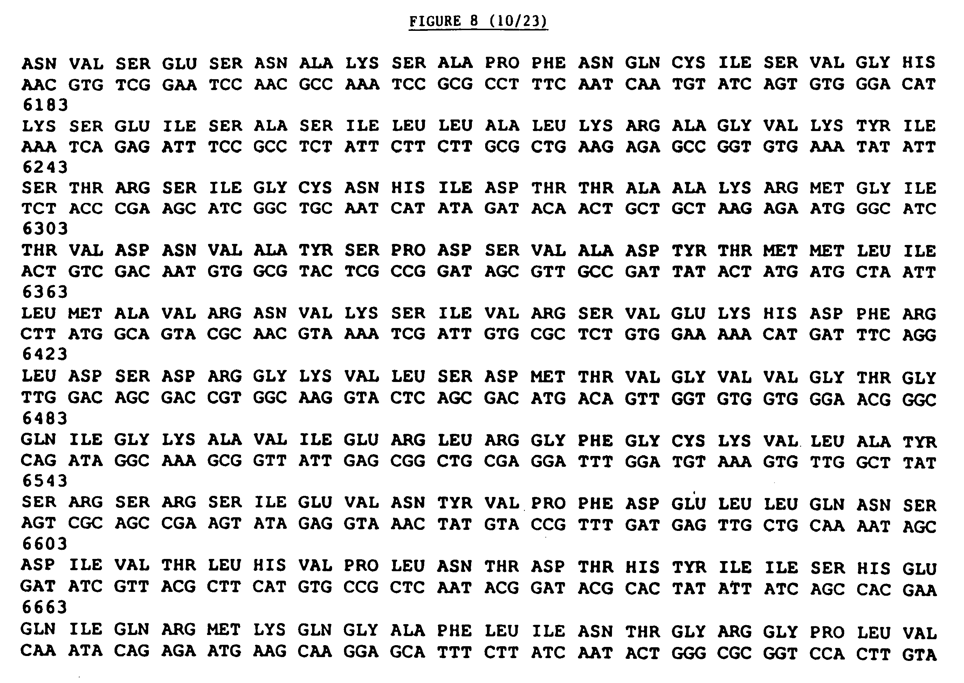

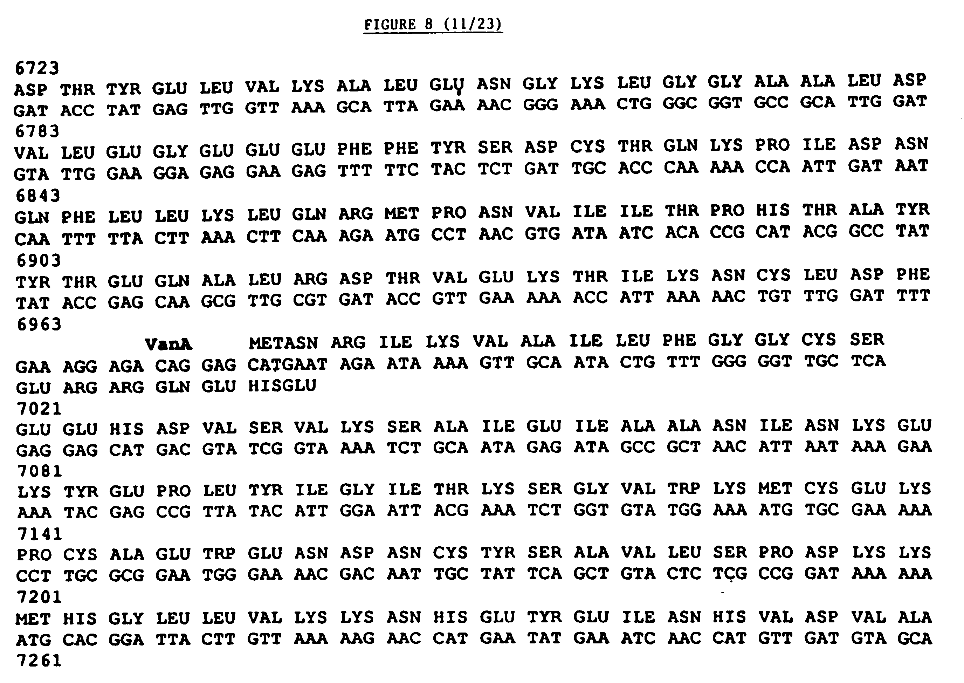

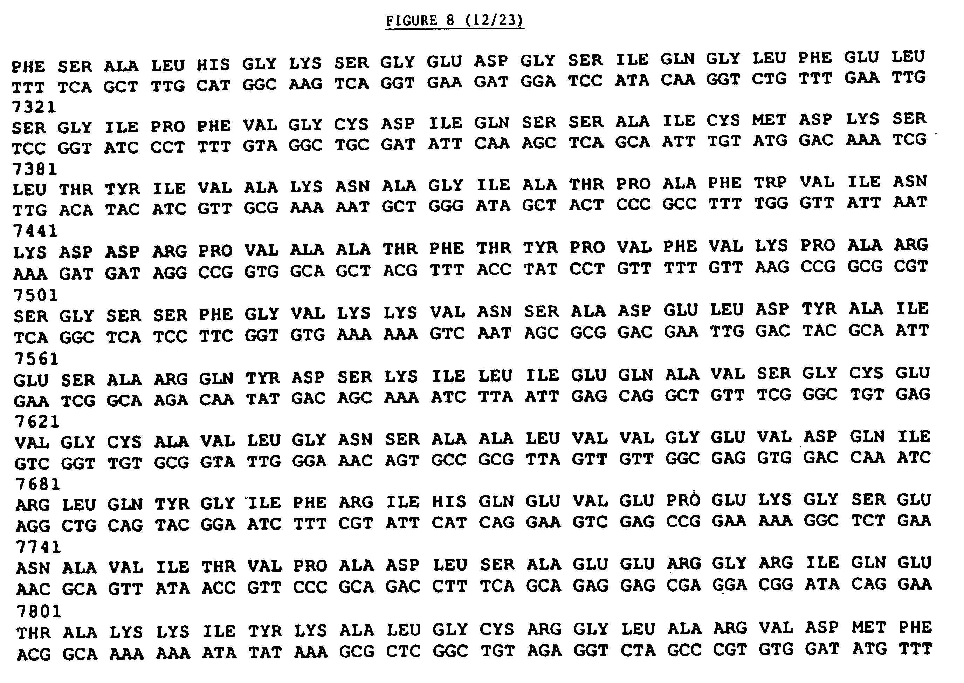

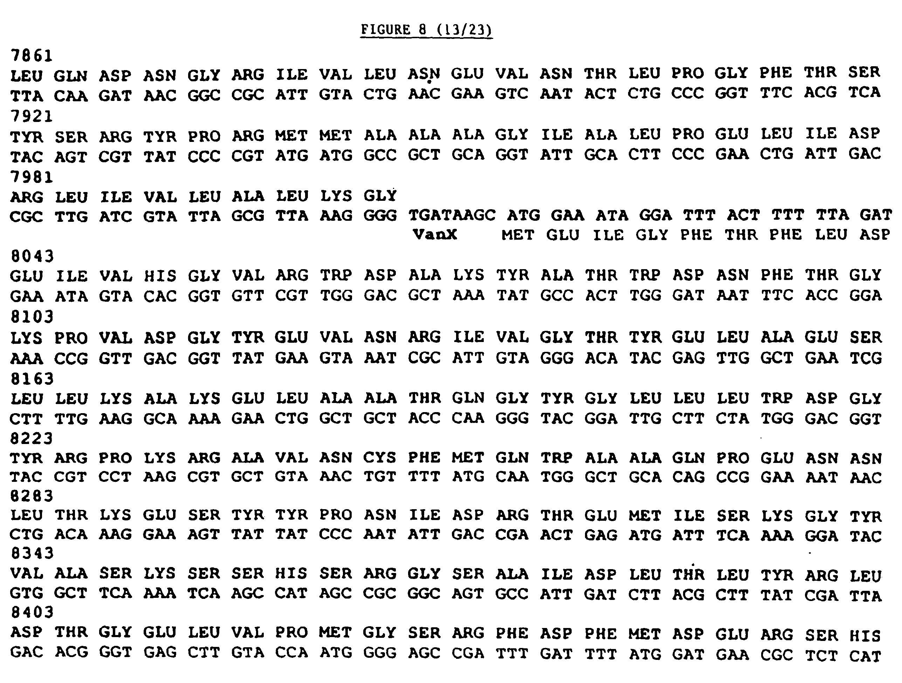

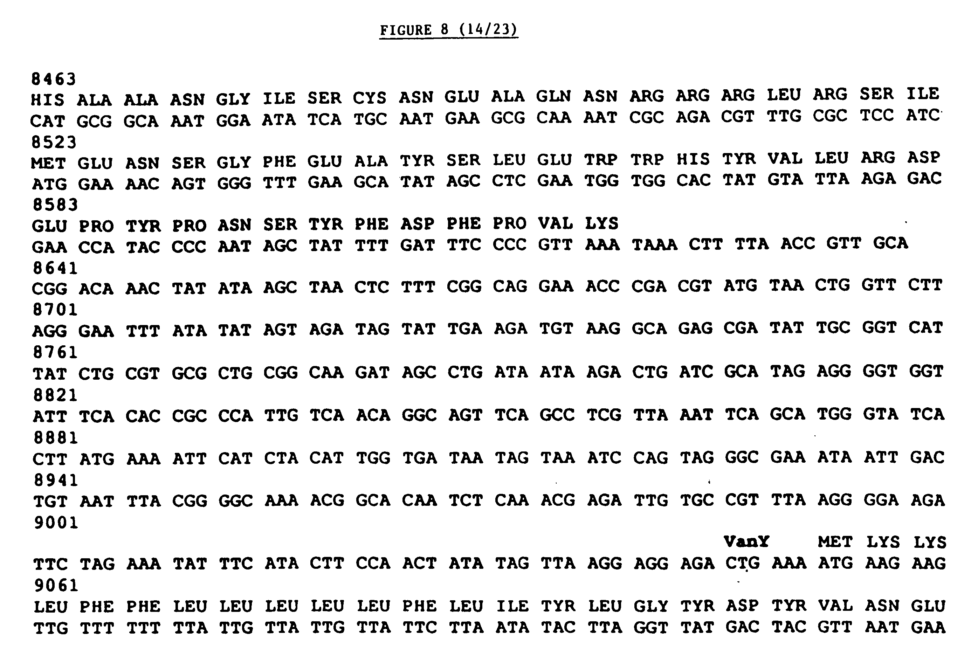

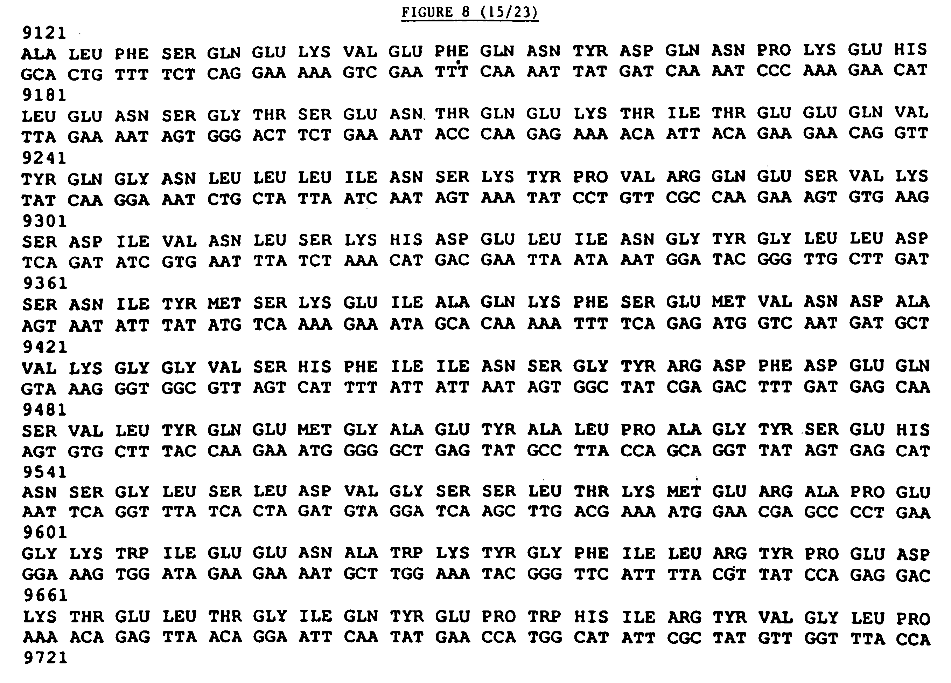

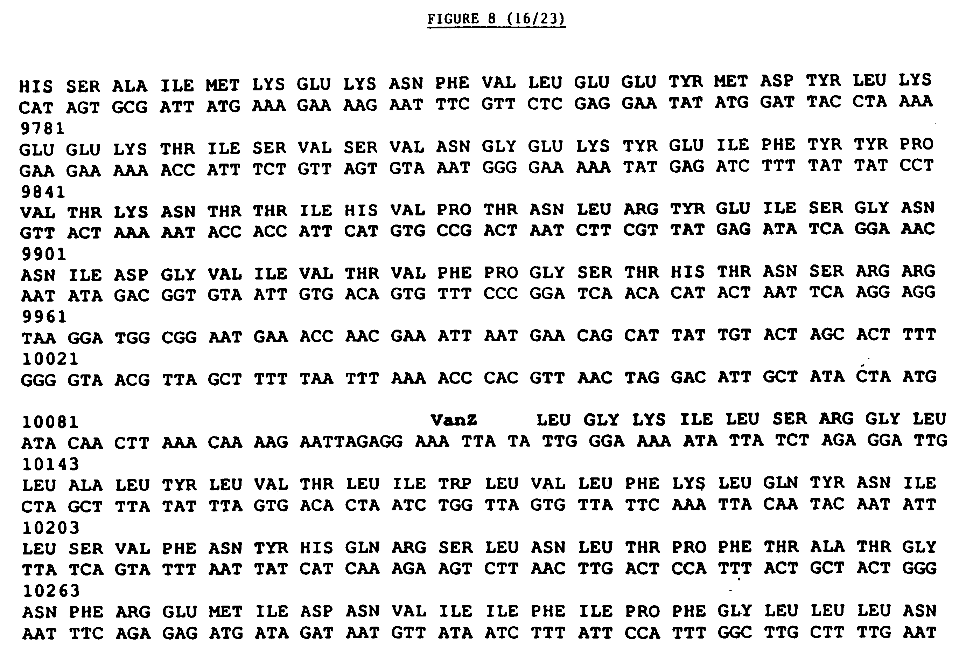

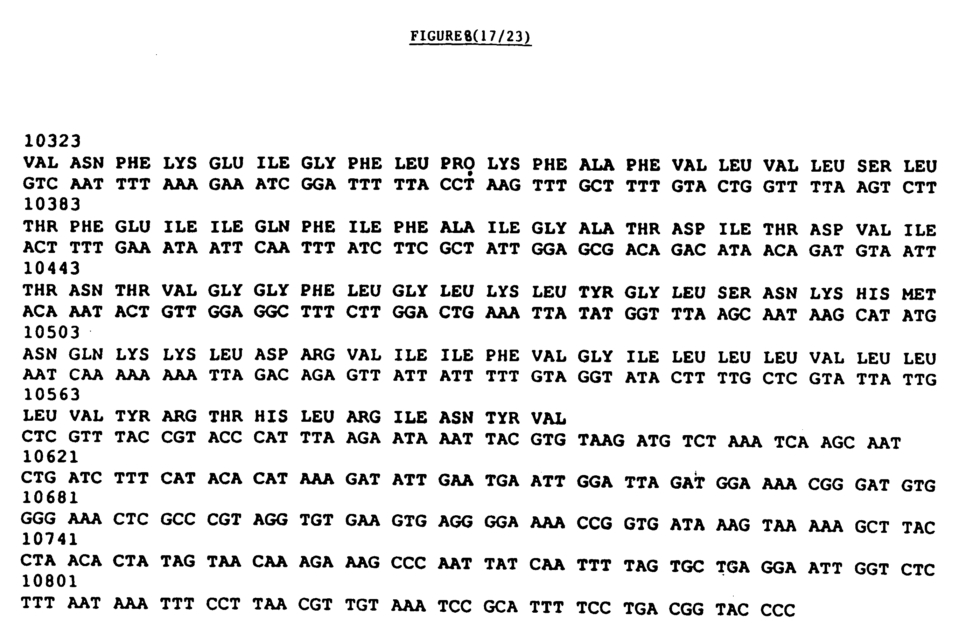

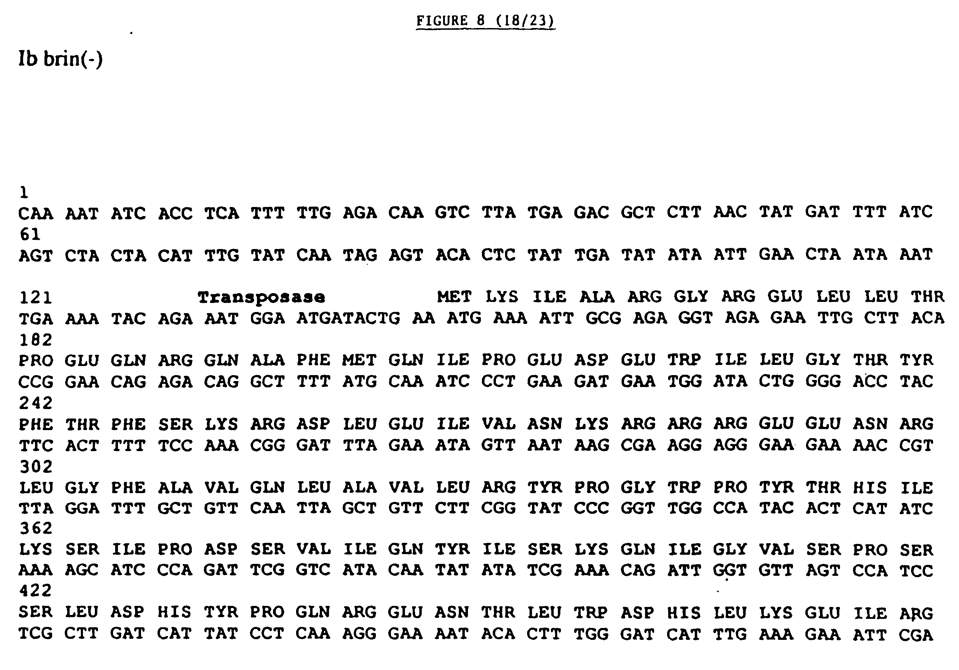

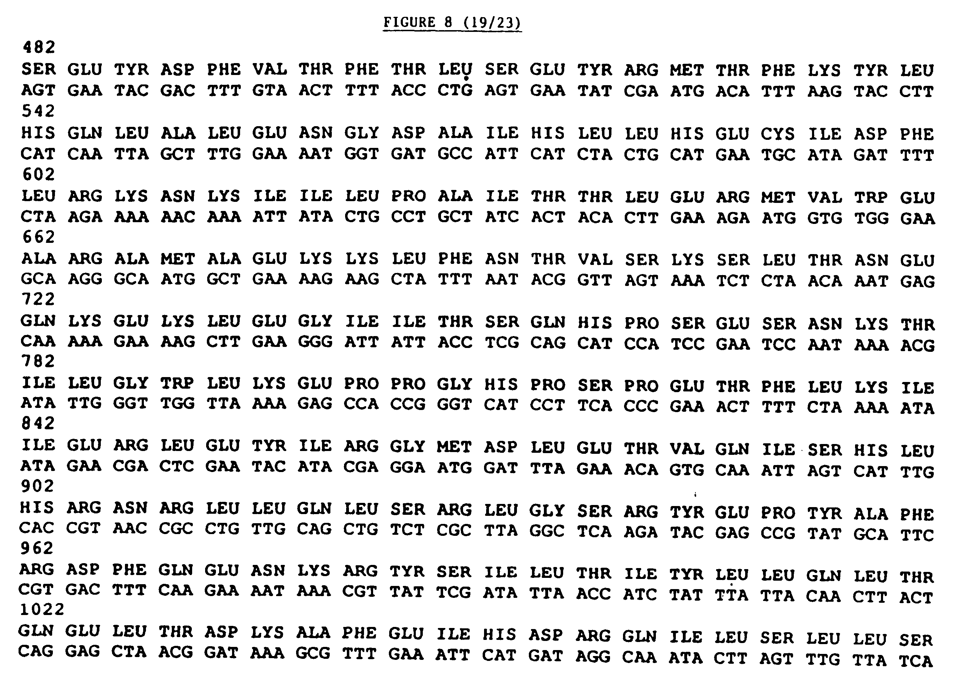

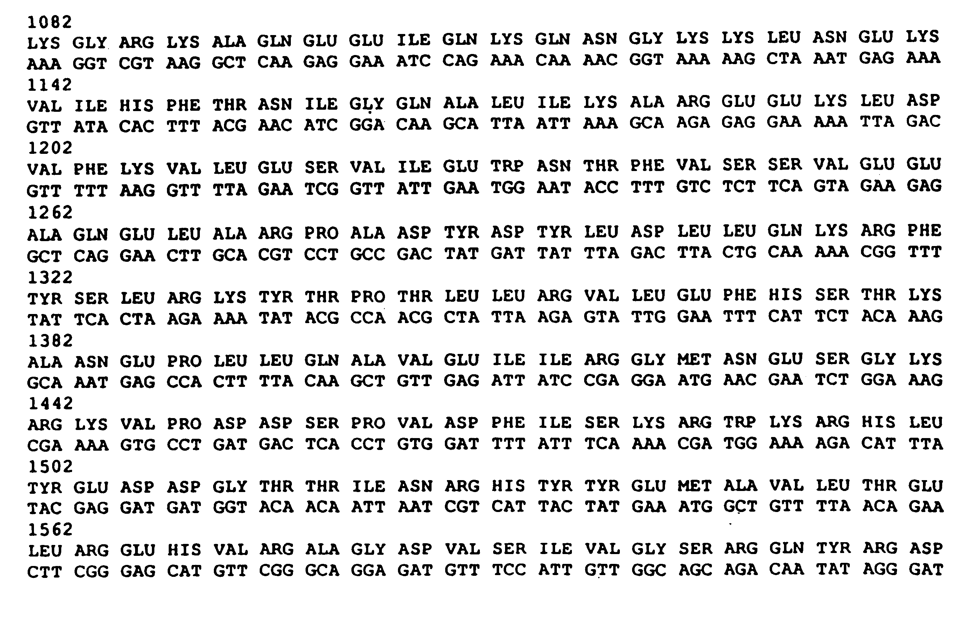

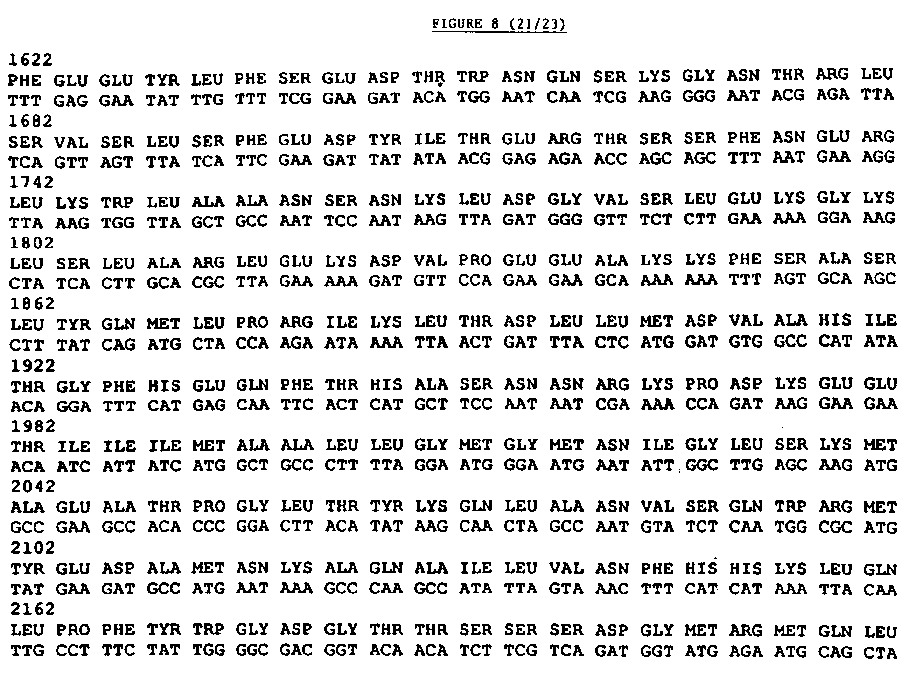

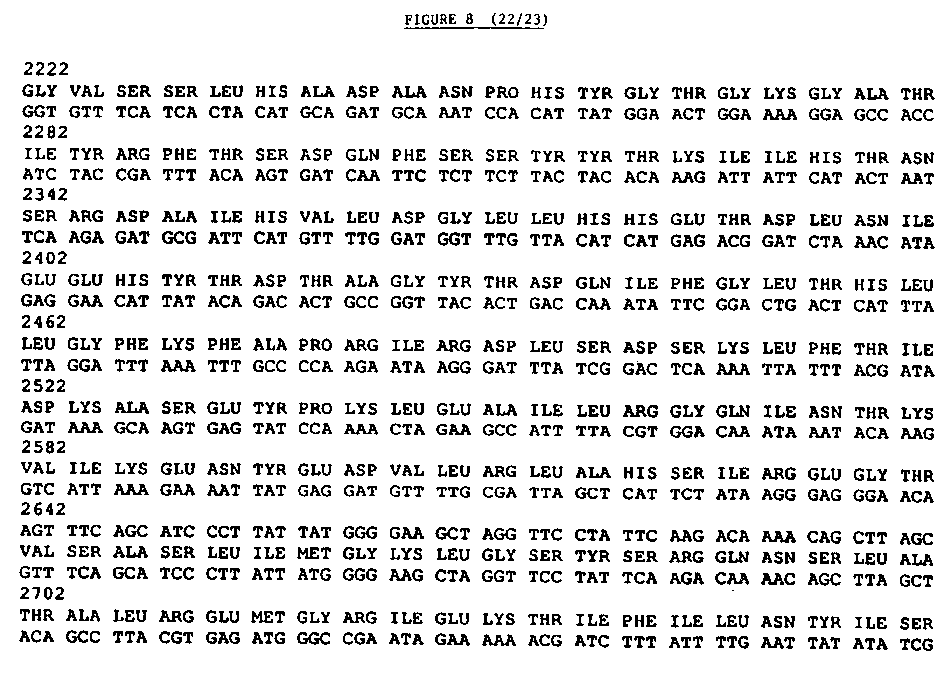

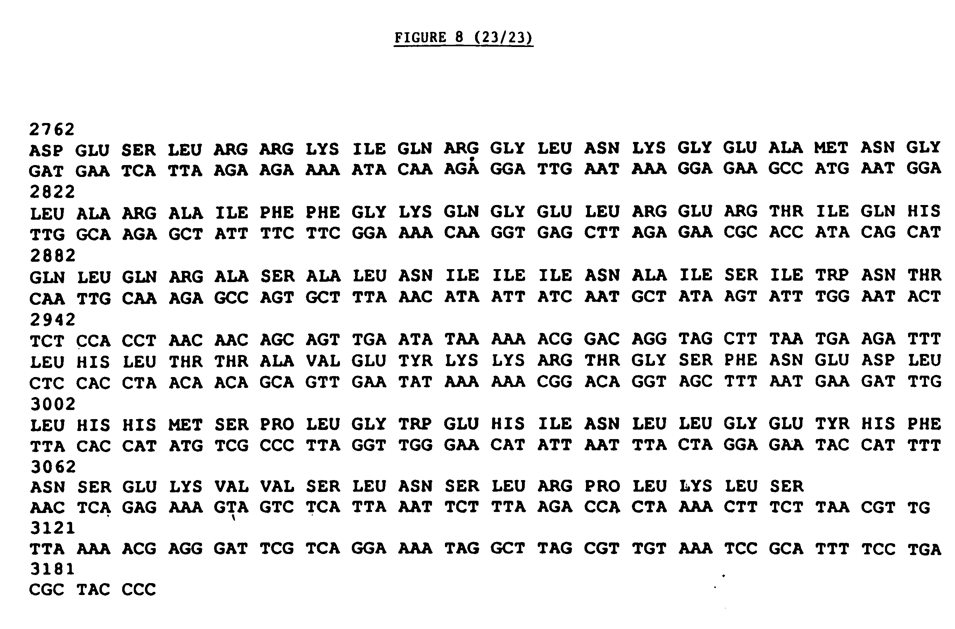

- Figure 8 : séquence nucléotidique du transposon représenté à la figure 7 et séquence -d'acides aminés des protéines correspondantes. La séquence nucléotidique est présentée pour le brin (+) et pour le brin (-) (correspondant à la séquence complémentaire du brin (+) des positions 1 à 3189) sur lequel est localisée la séquence codante de la transposase.

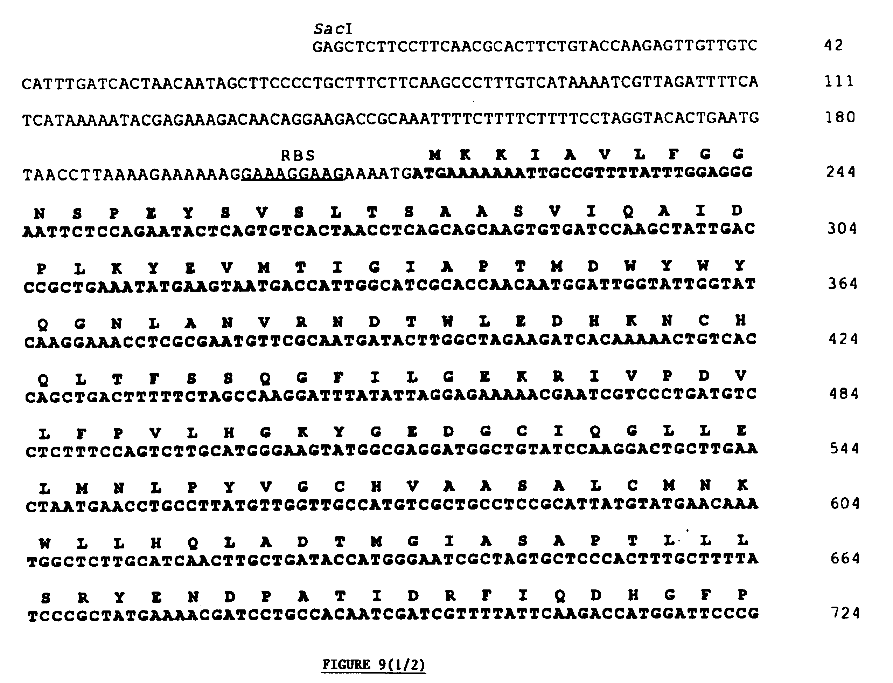

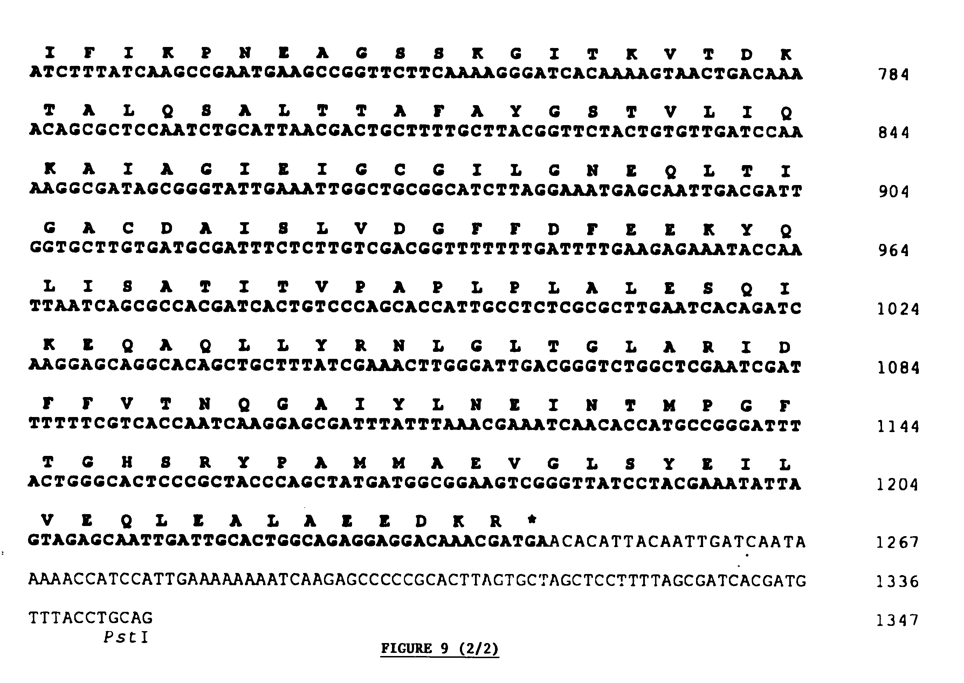

- Figure 9 : séquence nucléotidique du fragment de 1347 bp SacI-PstI du plasmide pAT216 contenant le gène vanC. La numérotation commence à la première base G du site de restriction SacI. La séquence RBS potentielle en amont du codon d'initiation de traduction ATG à la position 215 est soulignée. Le codon STOP (TGA) est indiqué par *. La région codante de vanC et la séquence d'acides aminés déduite sont indiquées en caractères gras. Des clones séquentiels se chevauchant ont été générés par des fragments de restriction de sous-clonage de pAT216 dans le bactériophage M13mp10 (Amersham, Angleterre). L'amorce universelle (New England Biolabs Beverly MA) a été utilisée pour séquencer l'insérat des phages recombinants. Le séquençage a été réalisé par la méthode enzymatique de di-desoxy nucléotide (Sanger et al, 1977 PNAS 74: 5463-5467) en utilisant la polymérase d'ADN T7 (Sequenase US B CORP, Cleveland, OH) et le [α-³⁵S]dATP (Amersham, England). Les produits des réactions ont été chargés sur des gels de polyacrylamide dénaturant à 6%.

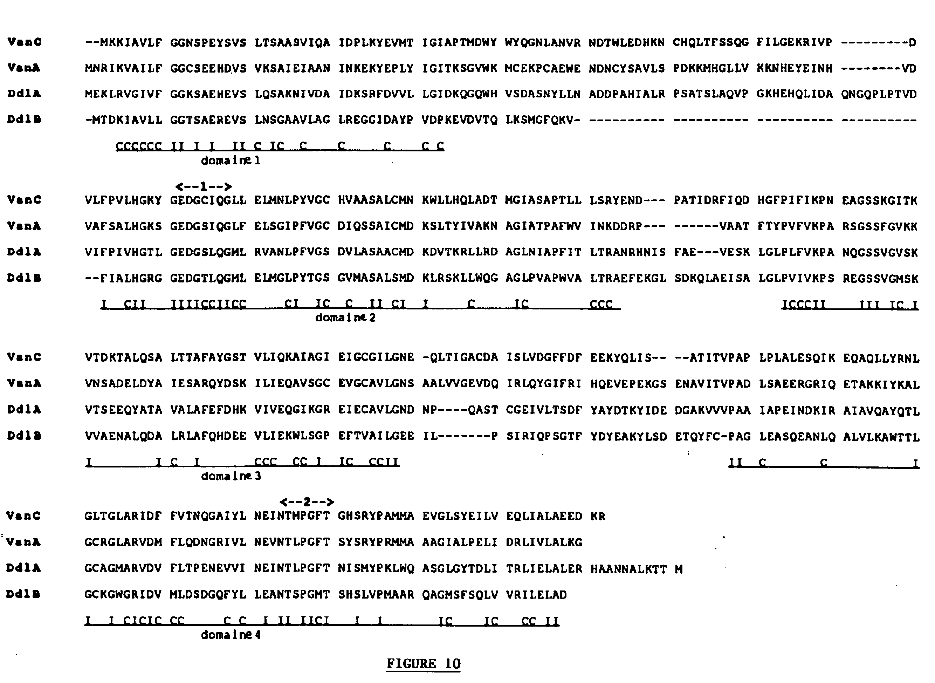

- Figure 10 : alignement des séquences d'acides aminés de VanC, VanA, Dd1A et Dd1B. Les acides aminés identiques (I) et les substitutions conservatives (C) dans les 4 séquences sont indiqués dans l'alignement. Pour classifier les substitutions conservatives, les acides aminés ont été groupés comme suit : RK, LFPMVI, STQNC, AGW, H, ED et Y. Les régions de forte homologie correspondant aux domaines 1, 2, 3 et 4 sont soulignées. Les séquences correspondant aux peptides 1 et 2 sont indiquées par les flèches.

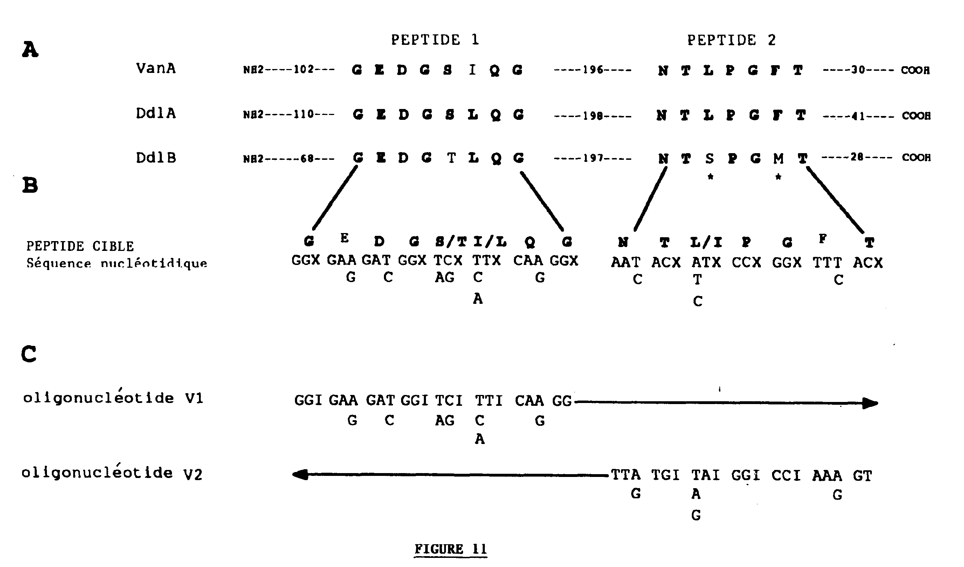

- Figure 11 : description des oligonucléotides V1 et V2
   (A) : Séquence d'acides aminés des peptides 1 et 2 de VanA et des D-Ala-D-Ala ligases. Le nombre d'acides aminés entre l'extrémité N-terminale et le peptide 1, entre les peptides 1 et 2 et le peptide 2 et l'extrémité C-terminale est indiqué. Les acides aminés identiques entre au moins 2 des 3 séquences, sont représentés en caractères gras.
   (B) : Peptides cible et séquence nucléotidique déduite. X représente une base quelconque de l'ADN. Le peptide 2 dans DdlB diffère du peptide cible au niveau de 2 positions (*).
   (C) : Séquence nucléotidique de V1 et de V2. Des nucléotides alternés et la déoxyinosine (I) qui peuvent correspondre à toute base d'ADN, ont été utilisés aux positions pour lesquelles les séquences nucléotidiques codant pour les peptides cible varient. Les flèches donnent la direction de la synthèse d'ADN. Les oligonucléotides ont été synthétisés par la méthode au méthoxy-phospharamidite avec un appareil Biosystem ADN 380B (Applied Biosystem, Foster City, Ca). L'ADN a été isolé à partir de lysats bactériens par extraction avec le bromure d'hexadécyl triméthyl ammonium (Inst. Biotechnologies, Inc, New Haven, CO) (Le Bouguénec et al, 1990, J. Bacteriol. 172:727-734) et utilisé comme matrice pour l'amplification par PCR avec un système de chauffage contrôlé "Intelligent Heating Block" IBH101 (Hybarid ltd, GB), selon la description de Mabilat et al (1990, Plasmid 23:27-34). Les produits d'amplification ont été révélés par électrophorèse sur gel à 0,8%, après coloration au bromure d'éthidium.

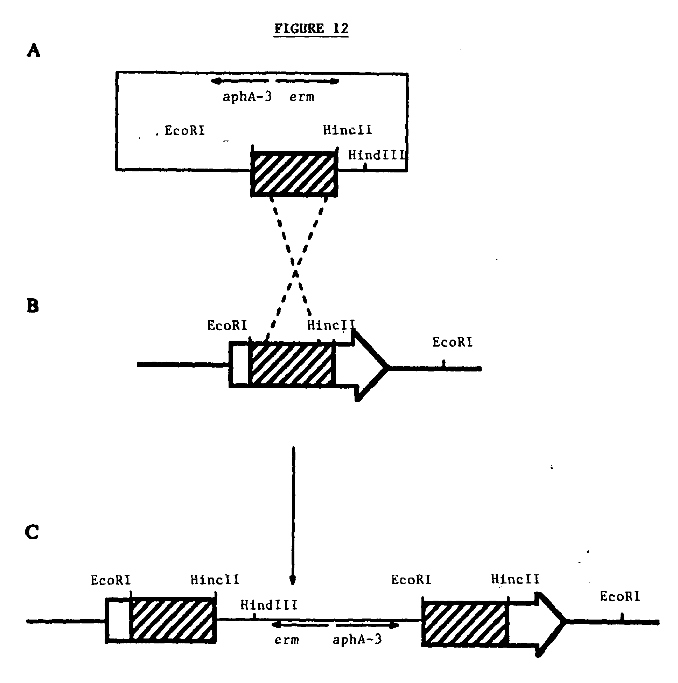

- Figure 12 : inactivation par insertion de vanC. Le gène vanC est représenté par une flèche ouverte et le fragment interne de 690 bp EcoRI-HinCII est hachuré. En trait fin on a représenté l'ADN de pAT114 ; en trait gras l'ADN chromosomique de PM4174 ; les flèches indiquent les gènes de résistance aux antibiotiques : aphA-3 est le gène codant pour la 3'-aminoglycoside phosphotransférase ; erm est le gène codant pour la ER^{R} méthyl transférase.
   (A) : Le plasmide pAT217 a été construit par ligature du fragment EcoRI-HinCII de pAT216 avec le vecteur suicide pAT114 (Trieu-Cuot et al, 1991, Gène 106:21-27) digéré avec EcoRI et SmaI.
   (B) : Région vanC de l'ADN chromosomique de BM4174.
   (C) : Région vanC après intégration de pAT217.

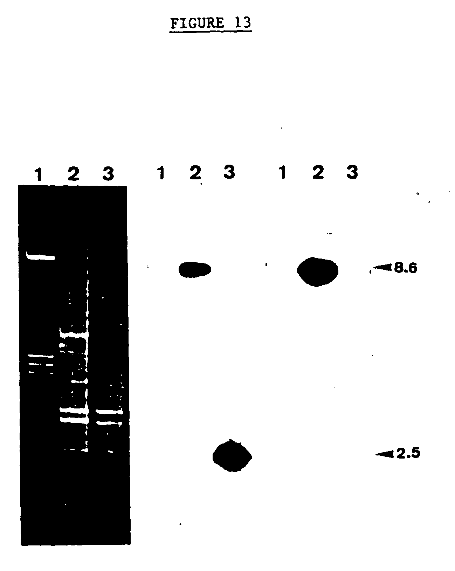

- Figure 13 : analyse Southern blot de l'intégration de pAT217 dans le gène vanC de BM4174.
   (partie gauche) : ADN total de BM4175 (ligne 2) et BM4174 (ligne 3) digéré avec EcoRI et résolu par électrophorèse sur gel d'agarose à 1%. L'ADN du bactériophage lambda digéré avec PstI a été utilisé comme standard de masse moléculaire (ligne 1). L'ADN a été transféré sous vide sur une membrane Nytran (Schleicher et Schül, Allemagne) en utilisant un appareil Trans-Vac TE80 (Höfer Scientific Instruments, San Francisco, CA) et lié à la membrane par l'intermédiaire d'une lumière UV. L'hybridation a été réalisée avec la sonde C (Middle) ou la sonde aphA-3 spécifique de pAT114 (Lambert et al, 1985, Annales de l'Institut Pasteur/Microbiol. 136(b): 135-150).
   (partie droite) : les sondes ont été marquées avec le ³²P par translation de coupure. Les masses moléculaires (kb) sont indiquées.

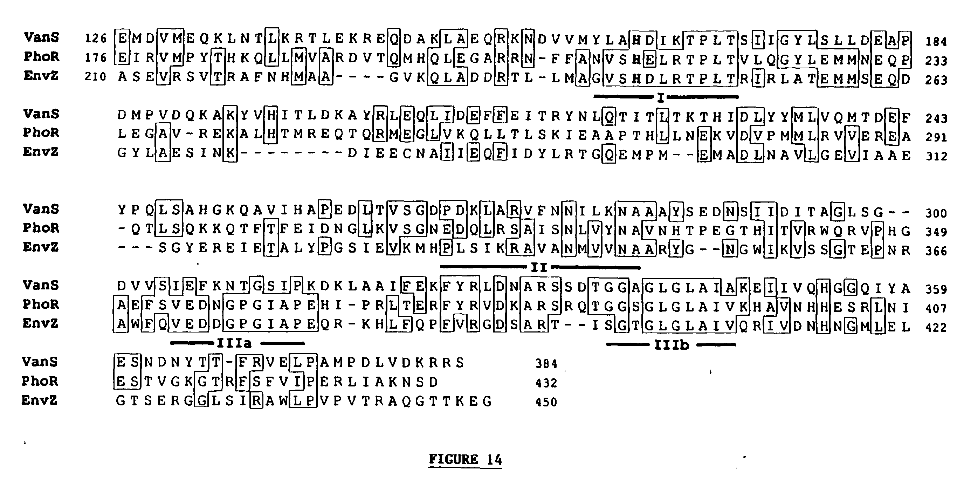

- Figure 14 _{:} alignement des séquences d'acides aminés déduites de VanS à partir de E. faecium BM4147 et de PhoR et EnvZ de E.coli. Les nombres sur la gauche se réfèrent à la position du premier acide aminé dans l'alignement. Les nombres sur la droite se réfèrent à la position du dernier acide aminé de la ligne correspondante. Les acides aminés identiques sont encadrés. Les pointillés indiquent des trous introduits pour optimiser la similitude. Les traits indiquent les positions des motifs conservés d'acides aminés dans d'autres HPK. Les résidus histidine en gras dans le motif 1 sont des sites potentiels d'autophosphorylation.

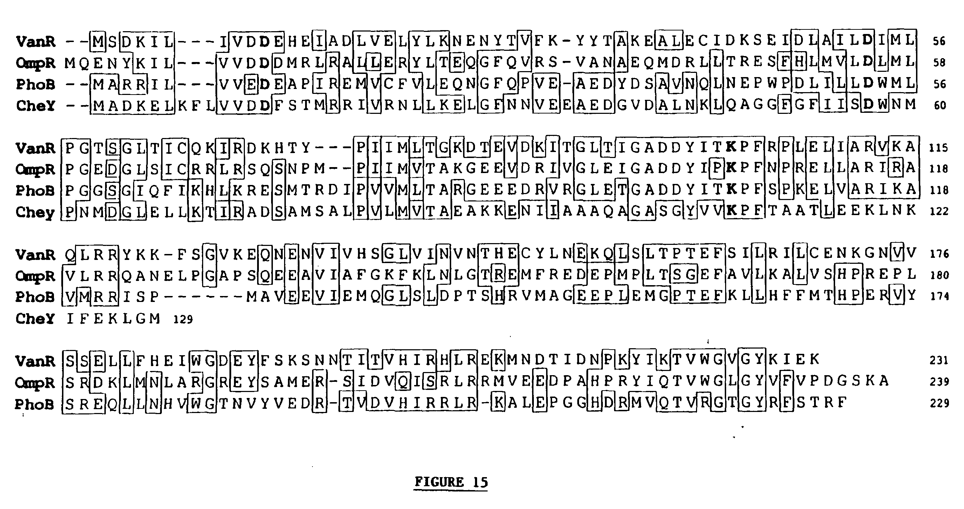

- Figure 15 : alignement des séquences d'acides aminés déduites de VanR de E. faecium BM4147, OmpR et PhoB de E.coli ainsi que de CheY de Salmonella typhimurium. Les nombres sur la droite indiquent la position du dernier acide aminé de la ligne correspondante. Les acides aminés indentiques sont encadrés. Les pointillés indiquent les trous introduits pour optimiser les similitudes. Les résidus en caractère gras correspondent aux acides aminés fortement conservés dans les domaines effecteurs, d'autres RR. Le résidu aspartique 57 de CheY est phosphorylé par la HPK associée CheA.

### I - IDENTIFICATION DE vanA

### Matériels et méthodes pour l'indentification et la caractérisation du gène vanA

### Souches bactériennes et plasmides

L'origine des plasmides utilisés est donnée dans le tableau ci-après.

| Souche ou plasmide | Source ou Référence |
|---|---|
| Escherichia Coli | |
| JM83 | Messing (1979) |
| AR1062 | Rambach et Hogness (1977) |
| JM103 | Hannshan (1983) |
| ST640 | Lugtenberg et van Schijndel van-Dam (1973) |

| Enterococcus faecium | |
|---|---|
| BM4147 | Leclercq et al (1988) |
| Plasmide pUC18 | Norrander et al (1983) |
| pAT213 | Brisson-Noël et al (1990) |
| pAT214 | Décrit dans ce texte |

### Préparation des membranes d'enterocoque

Enterococcus faecium BM4147 a été cultivé jusqu'à l'obtention d'une densité optique (DO₆₀₀) de 0,7 dans 500ml de bouillon coeur-cervelle (milieu broth BHI). L'induction a été réalisée avec 10 µg/ml de vancomycine (Eli Lilly Indianapolis Ind). Les étapes ultérieures ont été réalisées à 4°C. Les cellules ont été récupérées par centrifugation pendant 10 minutes à 6000g, lavées dans un tampon TE (0,01 M TRIS-HC1, 0,002 M EDTA, pH 7,0) et lysées avec des billes de verre (100 µm de diamètre) dans un appareil Braun pendant 2 minutes. Les débris cellulaires ont été séparés par centrifugation pendant 10 minutes à 6000g. Les membranes ont été collectées par centrifugation pendant 1 heure à 65000g et resuspendues dans 0,5ml de tampon TE.

### Préparation des minicellules

Des plasmides ont été introduits par transformation dans la souche E.coli AR1062 préparée sous forme de sac bactérien. Les sacs bactériens ont été récupérés sur des gradients de sucrose et les protéines ont été marquées avec 50 µCi de [³⁵S]L-methionine (Amersham, Grande-Bretagne) selon la méthode de Rambach et Hogness (1977, P.N.A.S. USA, 74; 5041-5045).

### Préparation des fractions membranaires et des fractions cytoplasmiques de E.coli

E.coli JM83 et des souches dérivées ont été mises en culture dans un milieu BHI jusqu'à l'obtention d'une densité optique (DO₆₀₀) de 0,7, lavées et suspendues dans un tampon TE. La suspension cellulaire a été traitée par ultrasons (phonolysée) pendant 20 secondes avec des doses de 50 W dans un appareil de fragmentation de cellules dans un appareil à ultrasons Branson B7 et les cellules intactes ont été éliminées par centrifugation pendant 10 minutes à 6000g. Le surnageant a été fractionné en fractions membranaire et cytoplasmique par centrifugation pendant 1 heure à 100000g.

### Electrophorèse sur gel de polyacrylamide SDS (SDS-PAGE)

Les protéines des fractions bactériennes ont été séparées par SDS-PAGE dans des gels à gradient linéaire de polyacrylamide (7,5% - 15%) (Laemmli 1970, Nature 227 : 680-685). L'electrophorèse a été réalisée pendant 1 heure à 200 V puis 3 heures à 350 V. Les gels ont été colorés avec du bleu de Coomassie. Les protéines des extraits ont été séparées dans des gels de 10% de polyacrylamide et visualisées par autoradiographie.

### Purification de la bande protéique et détermination de la séquence N-terminale

Les protéines des fractions membranaires d'une culture induite de E.faecium BN4147 ont été séparées par SDS-PAGE. Le gel a été electrotransféré pendant 1 heure à 200 mA sur une membrane de polyvinylidène difluorure (Immobilon Transfer, Millipore) en utilisant un appareil de transfert (Electrophoresis Unit LKB 2117 Multiphor II) selon les recommandations du fabricant. Les protéines transférées ont été colorées avec le rouge de Ponceau. La portion de membrane portant la protéine intéressante a été coupée, centrée sur un filtre en teflon et placée dans la cartouche du bloc d'un séquenceur (Séquenceur Applied Biosystems modèle 470A). La protéine a été séquencée par la dégradation automatisée de Edman (1967, Eur. J.Biochem 1; 80-81).

### Construction de plasmides

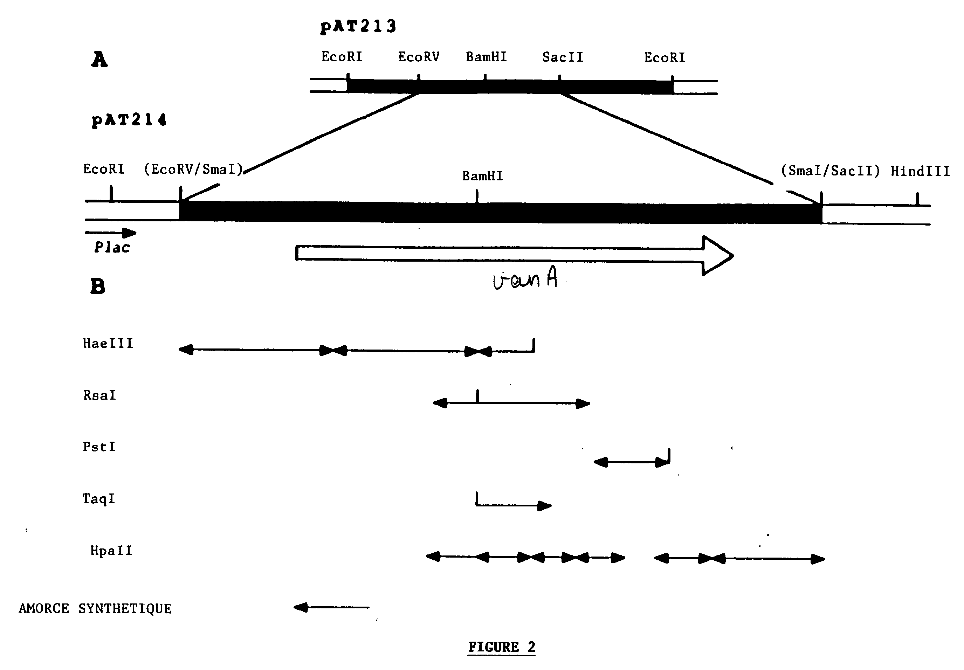

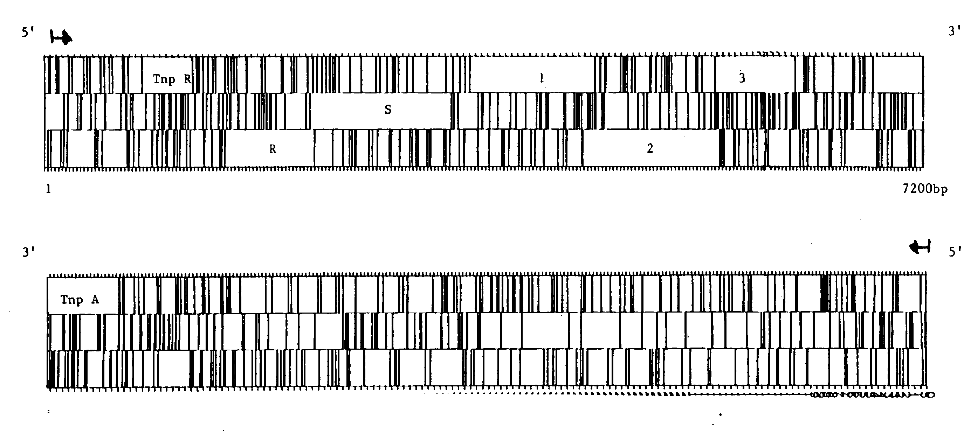

Le plasmide pAT213 (Brisson-Noël et al, 1990, Antimicrob Agents chemother, 34; 924-927) consiste en un fragment d'ADN EcoRI de 4,0 kb du plasmide pIP816 d'entérocoque cloné au site EcoRI d'un vecteur navette gram-positif-gram-négatif pAT187 (Trieu-Cuot et al, 1987, FEMS Microbiol Lett 48; 289-294). Pour construire pAT214, le fragment d'ADN de 1761 bp EcoRV-SacII de pAT213 a été purifié, traité avec le fragment de Klenow de l'ADN polymérase I de E.coli et ligué à l'ADN de pUC18 préalablement digéré avec SmaI et déphosphorylé (figure 2). Le clonage (Maniatis et al, 1982 Cold Spring Harbor Laboratory Press) a été réalisé avec des endonucléases de restriction (Boehringer Mannheim et Pharmacia) avec la ligase ADN T4 (Pharmacia) et la phosphatase alcaline (Pharmacia) selon les recommandations du fabricant.

### Sous-clonage dans M13 et séquence nucléotidique

Les fragments d'ADN de restriction ont été sous-clonés dans le polylinker des formes de réplication des dérivés mp18 et mp19 du bactériophage M13 (Norrander et al, 1983, Gene 26; 101-106), obtenus auprès de Pharmacia P-L Biochemicals. E.coli JM103 a été transfecté avec des phages recombinants et de l'ADN simple brin a été préparé. Le séquençage nucléotidique a été réalisé par la méthode enzymatique des di-desoxy nucléotides (Sanger et al, 1977, P.N.A.S USA 74; 5463-5467) en utilisant une ADN T7 polymérase (Sequenase, United States Biochemical Corporation, Cleveland Ohio) et de l'[α³⁵S]dATP (Amersham, Grande-Bretagne). Les produits des réactions ont été révélés dans des gels de polyacrylamide contenant un tampon dénaturant à 6%.

### Analyse informatique et données sur la séquence

La séquence d'ADN complète a été assemblée en utilisant les programmes d'ordinateur DBCOMP et DBUTIL (Staden, 1980, Nucleic Acids Res 8; 3673-3694). La banque de données de protéines, PSEQIP de l'Institut Pasteur a été criblée en utilisant un alogorythme développé par Claverie (1984, Nucleic Acids Res 12; 397-407). Les alignements entre des paires de séquences d'acides aminés ont été construits en utilisant l'algorythme de Wilbur et al (1983, P.N.A.S USA 80; 726-730). La signification statistique de l'homologie a été évaluée avec l'algorythme de Lipman et Pearson (1985, Science 227; 1435-1440).

Pour chaque comparaison 20. séquences d'acides aminés ont été utilisées pour calculer les moyennes et les déviations standard des résultats aléatoires.

### Tests de complémentation génétique

Les plasmides ont été introduits par transformation dans E.coli ST640, un mutant sensible à la température avec une ligase non modifiée D-ala-D-ala (Lugtenberg et al 1973, J. Bacteriol 110 ; 26-34). Les transformants ont été sélectionnés à 30°C sur des plaques contenant 100 µg/ml d'ampicilline et la présence de l'ADN plasmidique de la taille recherchée et les cartes de restriction ont été vérifiés. Des colonies uniques poussées à 30°C dans un milieu BHI broth contenant de l'ampicilline ont été placées à la fois sur un milieu agar BHI contenant 100 µg/ml d'ampicilline et dans 50 µM d'isopropyle-1-thio-β-D-glacto-pyranoside (IPTG) et les plaques ont été incubées à une température permissive de 30°C ou non permissive de 42°C. Le test de complémentation était considéré comme positif si les colonies étaient présentes après 18 heures d'incubation à 42°C.

### RESULTATS

### Identification de la protéine VanA et séquence N-terminale

Les fractions membranaires des E. faecium BM4147 mis en culture d'une part dans des conditions d'induction, et d'autre part en l'absence d'induction, ont été analysées par SDS-PAGE. La seule différence détectable associée avec l'exposition à des concentrations sous-inhibitrices de vancomycine a été l'intensification marquée d'une bande qui correspondait à une protéine de poids moléculaire estimé d'environ 40 kDa. Dans les cellules induites et dans les cellules non induites, la bande protéique représente la même protéine puisque cette bande est absente des membranes d'un dérivé de BM4147 qui a perdu le plasmide pIP816. La protéine inductible, désignée par VanA a été purifiée après SDS-PAGE et une dégradation automatisée de Edman a été réalisée sur un échantillon de 50 pmol. Neuf acides aminés de la séquence N-terminale de VanA ont été identifiés : Met Asn Arg Ile Lys Val Ala Ile Leu.

### Sous-clonage du gène vanA

L'insérat de 4,0 kb du plasmide pAT213 porte le déterminant de la résistance aux glycopeptides de E. faecium BM4147. Divers fragments de restriction de cet insérat ont été sous-clonés dans pUC18 et les plasmides recombinants spécifiques de vanA dans E.coli ont été identifiés par analyse SDS-PAGE des protéines des fractions cytoplasmique et membranaire ou des extraits de sacs bactériens. Cette approche a été utilisée car E.coli est intrinsèquement résistant au glycopeptide. L'insérat EcoRV-SacII du plasmide pAT214 (figure 2) code pour un polypeptide unique de 40 kDa qui migre conjointement avec VanA, issu des préparations membranaires de E. faecium BM4147.

### Séquence nucléotidique de l'insérat dans pAT214 et identification de la séquence codante vanA

La séquence nucléotidique de l'insérat EcoRV-SacII de 1761 bp dans pAT214 a été déterminée sur les deux brins de l'ADN selon la stratégie décrite à la figure 2. La localisation des codons de terminaison (TGA, TAA, TAG) dans trois cadres de lecture sur chaque brin d'ADN a montré la présence d'un unique cadre de lecture ouvert (ORF) ayant une taille suffisante pour coder pour la protéine VanA. Ce cadre de lecture ORF est localisé entre le codon TAA en position 281 et le codon TAG en position 1406. La séquence d'acides aminés déduite de ORF a été comparée avec celle de l'extrémité N-terminale de VanA. Les neufs acides aminés identifiés par le séquencage protéique sont codés par la séquence nucléotidique débutant avec le codon ATG (méthionine) en position 377 (figure 3). Ce codon d'initation de traduction est précédé par une séquence (TGAAAGGAGA), caractéristique d'un site de liaison au ribosome (RBS) de bactéries à Gram-positif qui est complémentaire pour les 8 bases de l'ARNr de la sous-unité 16S de Bacillus subtilis dans sa partie (3'OH UCUUUCCUCC 5') (Moran et al, 1982, Mol. Gen. Genet. 186; 339-346). Dans ce cadre ORF, il n'y a pas d'autre codon d'initiation ATG ou GTG entre les positions 281 et 377. La séquence de 1029 bp qui s'étend du codon ATG en position 377 au codon TGA en position 1406 code pour une protéine contenant 343 résidus d'acides aminés. Le poids moléculaire calculé de cette protéine est 37400 Da ce qui est en accord avec l'estimation de 40 kDa obtenue par l'analyse SDS-PAGE.

### Homologie des séquences d'acides aminés de VanA et des enzymes ligases D-ala-D-ala

Le criblage de la banque de données de protéines, PSEQIP a montré l'existence d'une homologie de séquences entre VanA et les ligases D-ala-D-ala de E.coli (ECOALA, Robinson et al, 1986, J. Bacteriol 167; 809-817) et de Salomella typhimurium (DALIG, Daub et al, 1988, Biochemistry 27; 3701-3708). Le pourcentage de similarité calculé par paires de protéines était compris entre 28% et 36% pour les acides aminés identiques et entre 48% et 55% en tenant compte des acides aminés homologues. VanA et DALIG sont plus étroitement liées. La signification statistique de ces similarités a été évaluée en alignant VANA et des séquences contenant la même composition en acides aminés que DALIG ou ECOALA (Lipman et Pearson, 1985, Science 227; 1435-1440).

### Test de complémentation génétique pour l'activité de ligase D-ala-D-ala

La souche E.coli ST640 est un mutant thermosensible présentant une activité ligase D-ala-D-ala déficiente (Lugtenberg et al, 1973, J. Bacteriol 113 : 96-104).

Les plasmides pUC18 et pAT214 ont été introduits par transformation dans E.coli ST640. Les souches ST640 et ST640 (pUC18) ont poussé normalement uniquement à la température permissive (30°C) alors que E.coli ST640 (pAT214) a poussé à la fois à la température permissive et à la température non permissive (42°C).

Ce test montre que VANA est fonctionnellement apparentée aux D-Ala-D-Ala ligases dans E.coli et est probablement capable de catalyser la même réaction de ligation que DALIG.

### II - Système de régulation à deux composants VanS-VanR, pour le contrôle de la synthèse de depsipeptides de précurseur de peptidoglycanes

### MATERIELS ET METHODES

### Souches, plasmides et conditions de culture

Les fragments de restriction de pIP816 (Tra-, Mob⁺, Vm^{r}) ont été clones dans des dérivés du vecteur pAT29 qui constitue un vecteur navette entre les bactéries gram-positives et gram-négatives (oriR pAMβ1, oriR pUC, oriT RK2, spc, lacZα) (Trieu-Cuot et al, 1990, Nucleic Acids Res. 18:4296). Ce vecteur a été construit par les inventeurs et utilisé pour transformer la souche E.coli JM103 (Δ(lac-proAB), supE, thi, strA, sbcB15, endA, hspR4, F traD36, proAB, LacI^{q}, lacZAM15) (Messing et al, 1983, Methods Enzymol. 101:20-78). L'ADN plasmidique a été préparé par un protocole de lyse alcaline à petite échelle (Sambrook et al, 1982, Molecular cloning, a laboratory manual. Cold Spring Harbor Laboratory, Cold Spring Harbor NY) et introduit par électrotransformation (Cruz-Rodz A.L. et al, 1990, Mol. Gen. Genet. 224: 152-154) dans E. faecalis JH2-2 (Fus^{R}, Rif^{R}) (Jacob A.E. et al, 1974, J. Bacteriol. 117:360-372), en utilisant un appareil Gene Pulser (Bio-Rad Laboratories, Richmond, Californie). Les profils de restriction des plasmides purifiés à partir de E. faecalis et de E.coli ont été comparés pour détecter d'éventuels réarrangements d'ADN.

Le plasmide intégratif pAT113 (Mob⁺, Em^{R}, Km^{R}, oriR PACYC184, attTn1545, LacZα) (Trieu-Cuot et al, Gene 106:21-27) porte les extrémités jointes du transposon Tn1545. Ce vecteur ne se réplique pas dans les bactéries gram-positives mais s'intègre au chromosome de l'hôte par recombinaison illégitime médiée par l'intégrase de Tn1545 ou de Tn916 (Trieu-Cuot et al précité). Les plasmides intégratifs ont été introduits par électrotransformation dans E. faecalis BM4148 (souche JH2-2::Tn916). Cette souche est modifiée par le transposon Tn916 décrit par Franque A.E. et al (1981, J. Bacteriol. 145: 494-502).

Les cultures ont été réalisées dans un bouillon coeur-cervelle (BHI - Brain Heart Infusion Broth) ou sur agar à 37°C. La méthode de Steers et al (Antibiot. Chemother. Basel. 9:307-311) a été utilisée pour déterminer les concentrations minimales d'inhibition (MICs) des antibiotiques sur un milieu gélosé Mueller-Hinton agar.

### Techniques d'ADN recombinant

Le clivage de l'ADN avec des endonucléases de restriction (Boehringer Manheim and Pharmacia), la purification des fragments d'ADN de restriction à partir des gels d'agarose, la conversion des extrémités cohésives en extrémités franches avec le fragment de Klenow de l'ADN polymérase I de E.coli (Boehringer Manheim), la déphosphorylation des extrémités de l'ADN avec la phosphatase intestine de veau (Boehringer Manheim), la ligature des fragments d'ADN avec la T4 DNA ligase (Amersham) ont été réalisés selon les méthodes standard de Sambrook et al (1982, Molecular Cloning, a laboratory Manual. Cold Spring Harbor Laboratory. Cold Spring Harbor NY).

### Construction de plasmide

L'origine des vecteurs et des insérats utilisés pour les plasmides recombinants construits ici est la suivante:
(i) vecteur pAT78 pour la reconnaissance de promoteur : l'ADN amplifié du gène cat de chloramphénicol acétyltransférase du plasmide pC194 de Staphylococcus aureus (Horinouchi et al, 1982, J. Bacteriol. 150:815-825) a été inséré entre les sites de restriction PstI et SphI du vecteur navette pAT29. L'amplification par la réaction de polymérisation en chaîne a été effectuée au moyen des amorces A1 et A2 qui ont été synthétisées par la méthode au méthoxy phosphoramidite (Mabilat et al, 1990, Plasmid 23:27-34). La séquence de l'amorce A1 (5'GCTGCAGATAAAAATTTAGGAGG) est composée d'un site de reconnaissance PstI (souligné) et de 18 bases (positions 6 à 23) de pC194 qui incluent le site de liaison au ribosome (RBS ; AGGAGG positions 18 à 23) du gène cat. La séquence de l'amorce A2 (5'CGCATGCTATTATAAAA GCCAGTC) contient le site de clivage SphI (souligné) et est complémentaire (positions 8 à 24) à 17 bases de l'extrémité 3' du gène cat. Le triplet ATT aux positions 9 à 11 correspond au codon stop TAA de cat. Les fragments d'ADN amplifiés avec les amorces A1 et A2 consistent donc en une phase ouverte de lecture (orf) et en un site de liaison au ribosome pour CAT (positions 1234 à 1912 selon la numérotation de Horinouchi et al (1982, J. Bacteriol. 150:815-825) flanqués par les sites PstI et SphI. La position 1234 est localisée à l'intérieur de la boucle de la structure secondaire de l'ARNm qui bloque la traduction en l'absence de chloramphénicol. Ainsi la séquence amplifiée ne contient pas le promoteur cat ni la séquence complémentaire du RBS qui est essentielle pour la régulation de traduction Ambulos, N.P et al, 1984, Gène 28:171-176).
(ii) vecteur d'expression pAT79 : le fragment de 243 bp ClaI-BssHII portant le promoteur P2 du gène aphA-3 du plasmide d'entérocoque pJH1 (Caillaud et al, 1987, Mol. Gen. Genet. 207: 509-513) a été inséré entre les sites de restriction EcoRI et SacI de pAT78.
(iii) plasmide pAT80 et ses dérivés : le fragment de 5,5 kb BglII-XbaI de pIP816 a été inséré entre les sites BamHI et XbaI de pAT78. Le plasmide résultant dénommé par pAT80 a été partiellement digéré avec HincII et ligaturé avec le fragment EcoRV contenant un gène apparenté au gène apha-I du transposon Tn903 (Oka A. et al, 1981, J. Mol. Biol. 147:217-226). Ce fragment contient le gène aphA-I qui code pour la 3' aminoglycoside phosphotransférase de type I conférant la résistance à la kanamycine. L'insertion de aphA-I a été réalisée à trois sites différents dans pAT80, générant les plasmides pAT81, pAT83 et pAT85. Les cassettes BamHI et EcoRI contenant aphA-I ont été insérées aux sites BamHI (pour former le plasmide pAT84) et EcoRI (pour former le plasmide pAT82) de pAT80.
(iv) plasmide pAT86, pAT87, pAT88 et pAT89 : le plasmide pAT86 a été construit par clonage du fragment de 2,803 bp, EcoRI-SacII de pAT80 codant pour VanH et VanA, au niveau d'un site SmaI de pAT79. pAT87 a été obtenu en insérant le fragment de 3,4 kb EcoRI-XbaI de pAT80 en amont du gène cat du vecteur de détection de promoteur pAT78. Le plasmide pAT88 résultait de la ligature de pAT78 digéré avec EcoRI et BamHI avec le fragment EcoRI-BamHI de 1,731 bp de pAT80. Le fragment BglII-AccI (positions 1 à 2356) de pAT80 a été inséré dans le polylinker du vecteur intégratif pAT113, générant pAT89.

### Sous-clonage dans M13 et séquençage

Les fragments d'ADN de restriction ont été sous-clonés dans un polylinker de dérivés réplicatifs du bactériophage M13, ces dérivés étant appelés mp18 et mp19 (Norrander et al, 1983, Gène 26:101-106). E.coli JM103 a été transfecté avec les phages recombinants et un ADN simple brin a été préparé. Le séquençage des nucléotides a été réalisé selon les conditions décrites par Sanger et al (Proc. Natl. Acad. Sci. USA, 1977, 74: 5463-5467) en utilisant la polymérase d'ADN T7 modifiée (Sequenase, United States, Biochemical Corporation Cliveland OH) et [α-35S]dATP (Amersham). Les produits des réactions ont été résolus sur des gels de gradients dans un tampon de polyacrylamide à 6%.

### Test enzymatique

Les dérivés JH2-2 de E. faecalis ont été cultivés à une densité optique OD₆₀₀ de 0,7 dans un milieu BHI broth complété avec de la spectinomycine (300 µg/ml). Les cellules ont été traitées avec une lysozyme, lysée par sonication et les débris cellulaires ont été centrifugés pendant 45 minutes à 100000g selon la description de Courvalin et al (1978, Antimicrob. Agents Chemother. 13:716-725). La formation de 5-thio-2-nitrobenzoate a été mesurée à 37°C en présence et en l'absence de chloramphénicol et l'activité CAT spécifique a été exprimée en micromole par minute et par milligramme de protéines (Shaw et al, 1975, Methods Enzymol. 43:737-755).

### RESULTATS

Les gènes vanH et vanA de pIP816 ont été clonés dans un plasmide pAT79 sous le contrôle du promoteur hétérologue P2 (Caillaud et al, 1987, Mol. Gen. Genet. 207: 509-513) et le plasmide pAT86 formé ne conférait pas à la souche E. faecalis JH2-2 la résistance à la vancomycine. Ces gènes ne sont donc pas suffisants pour la synthèse de peptidoglycane en l'absence de l'antibiotique. Différents fragments de restriction de pIP816 ont été clonés dans le vecteur pAT78. Le fragment BglII-XbaI de 5,5 kb de pAT80 est le plus petit fragment obtenu qui conférait la résistance à la vancomycine.

### Séquence nucléotidique des gènes vanR et vanS

La séquence de l'insérat dans pAT80 a été déterminée sur les deux brins de l'ADN à partir du site BglII jusqu'au codon d'initiation de traduction ATG de VanH. Deux phases ouvertes de lecture (orf) ont été mises en évidence à l'intérieur de la séquence de 2475 bp : la première phase ouverte de lecture s'étend du nucléotide 386 au nucléotide 1123 ; en position 431 on trouve une séquence caractéristique des séquences RBS de bactéries gram-positives, 6 paires de base en amont du codon d'initiation de traduction ATG (TGAAAGGGTG) ; les autres codons d'initiation de traduction dans cette orf ne sont pas précédés de ce type de séquence. La séquence de 693 bp à partir du codon ATG au niveau 431 jusqu'au codon TAA à la position 1124 est susceptible de coder pour une protéine de 231 acides aminés avec une masse moléculaire de 26,612 Da et qui est désignée par VanR.

Pour la deuxième phase ouverte de lecture (du nucléotide 1089 au nucléotide 2255) la séquence d'acides aminés déduite à partir du premier codon d'initiation en phase (TTG en position 1104) coderait pour une protéine de 384 acides aminés ayant une masse moléculaire de 43,847 Da et désignée par VanS. Les codons TTG en position 1116 et ATG en position 1164 sont des codons d'initiation de traduction en phase précédée par des séquences avec une faible complémentarité avec la terminaison 3'OH de la sous-unité 16S de l'ARN de B. subtilis (GGGGGGTTGG-N8-TTG et AGAACGAAAA-N6-ATG respectivement).

Entre le dernier codon de vanS et le codon d'initiation de traduction ATG de vanH on remarque une séquence de 217 bp qui contient une séquence inversée répétée de 17 bp. Cette séquence ne fonctionne pas comme un terminateur de transcription fort.

La comparaison des séquences obtenues avec des bases de données a montré que les motifs d'acides aminés conservés identifiés par Stock et al (1989, Microbiol. Rev. 53:450-490) dans le domaine kinase de 16 HPK (Histidine Protein Kinase) étaient détectés dans la partie C-terminale de VanS. VanS présente deux groupes d'acides aminés hydrophobes dans la région N-terminale. Le résidu Histidine 164 de VanS est aligné avec le résidu His216 de PhoR (Makino et al, 1986, J. Mol. Biol. 192:549-556) et His 243 de EnvZ (Comeau et al, 1985, 164:578-584) qui sont des sites présumés d'autophosphorylation chez ces protéines.

De même les acides aminés 1 à 122 de VanR présentent des similitudes avec les domaines effecteurs de régulateurs de réponse RR (Response Regulators). L'acide aspartique 53 de VanR pourrait être un site de phosphorylation puisque ce résidu est aligné avec Asp 57 de Che Y qui est phosphorylé par HPK associé à CheA et correspond à une position invariante dans d'autres protéines de type RR (Stock et al précité). VanR pourrait appartenir à la sous-classe OmpR-PhoB de RR qui active l'initiation de transcription médiée par l'ARN polymérase comportant le facteur σ70S de E.coli (Stock et al précité).

### Inactivation par insertion des gènes van

Des cassettes de résistance à la kanamycine insérées dans le groupe de gènes van, dans le plasmide pAT80 ont montré ceci : l'insertion dans vanR supprime la résistance à la vancomycine et au chloramphénicol ; VanR est un activateur de transcription nécessaire pour l'expression des gènes de résistance à la vancomycine. L'inactivation de vanS conduit à une réduction de deux fois la concentration minimale inhibitrice (MIC) de chloramphénicol et à une réduction de trois fois de l'activité CAT spécifique mais la concentration minimale inhibitrice de vancomycine reste inchangée. Donc VanS est nécessaire pour obtenir un fort niveau de transcription des gènes de résistance à la vancomycine bien qu'il ne soit pas requis pour l'expression du phénotype de résistance à la vancomycine.

Des dérivés de pAT80 portant des insertions dans vanH (pAT83), vanA (pAT84) ou dans la région de 1,0 kb en aval de vanA (pAT85), ont permis d'obtenir une résistance au chloramphénicol mais pas à la vancomycine. Ce phénotype dissocié correspond à l'inactivation de gènes codant pour des enzymes qui synthétisent les précurseurs depsipeptidiques nécessaires pour l'assemblage des parois bactériennes en présence de vancomycine.

En aval du gène vanA, on a mis en évidence au niveau d'une séquence de 365 bp après le codon TGA de vanA et avant le site SacII, la présence d'un orf inactivé dans pAT85 et qui contient un codon d'initiation ATG en phase, précédé d'une séquence RBS-like. Cette séquence code pour une protéine nécessaire à la résistance au glycopeptide, désignée par VanX et qui comprend au maximum 330 acides aminés environ.

### Trans-activation de la transcription des gènes van

Le plasmide intégratif pAT89 codant pour VanR et VanS a été introduit dans le chromosome de E. faecalis BM4138. Le plasmide pAT87 portant les gènes vanH, vanA et vanX clonés en amont du gène cat dépourvu de promoteur de pAT78, a conféré la résistance à la vancomycine à cette souche mais pas à E. faecalis JH2-2. Le niveau d'expression du gène cat de pAT87 dans les souches BM4138::pAT89 et JH2-2 a indiqué que VanR active la transcription du gène reporteur localisé à l'extrémité 3' du groupe de gènes van. Des niveaux similaires de synthèse CAT ont été observés pour pAT88 qui porte une fusion de transcription entre les parties 5' de vanA et le gène cat. Ces résulats montrent que dans E. faecalis BM4138::pAT89 (pAT87) VanR et VanS codés par le chromosome activent en trans la transcription de vanA, vanH et vanX de pAT87 permettant l'obtention d'une résistance à la vancomycine.

On a par ailleurs remarqué que l'expression du gène était essentiellement constitutive lorsque vanR et vanS étaient portés par un plasmide multicopie pAT80 et faiblement inductible par la vancomycine lorsque les gènes pour les protéines de régulation étaient présents sur le chromosome de l'hôte.

### III- Caractérisation de la séquence du gène vanC d'Enterococcus gallinarum BM4174

### Définition et utilisation d'amorces universelles pour l'amplification de gènes codant pour des ligases D-Ala-D-Ala et des protéines apparentées impliquées dans la résistance à la vancomycine

La protéine VanA nécessaire à l'expression d'un haut niveau de résistance aux glycopeptides dans E. faecium BM4147 partage environ 28 à 36% de similitude en acides aminés avec les ligases D-Ala-D-Ala de E.coli mais possède une spécificité de substrats différente de celle de ces ligases. Des peptides désignés par 1 et 2 qui sont conservés dans les séquences des ligases Dd1A et DdlB (Zawadzke, 1991 Biochemistry 30:1673-1682) de E.coli et dans la protéine VanA ont été sélectionnés pour synthétiser les amorces universelles destinées à amplifier des fragments internes de gènes codant pour des D-Ala-D-Ala ligases ou enzymes apparentées. Les peptides cibles GEDG(S/T) (I/L)QG et NT(I/L)PGFT ont été traduits en retour comme le montre la figure IV.1, pour obtenir des oligonucléotides dégénérés V1 et V2. Comme les peptides 1 et 2 de VanA, Dd1A et DdlB sont séparés par des séquences d'acides aminés de longueur similaire, la taille prédite pour le produit d'amplification était d'environ 640 bp.

Une amplification par PCR avec l'ADN de E.coli JM83 et de E. faecium BM4147 a conduit à amplifier des produits correspondant à la taille attendue, qui ont ensuite été purifiés et clonés dans le bactériophage M13mp10 (Norrander et al, 1983, Gene 26:101-106). Le séquençage de l'insérat obtenu avec E.coli JM83 a indiqué que le produit de PCR était un fragment interne de ddlA. Une sonde générée à partir d'un phage recombinant obtenu avec le fragment d'amplification de BM4147 a été utilisée pour l'analyse Southern d'un ADN de BM4147 et de BM4147-1 qui est un dérivé de BM4147 sensible à la vancomycine et qui est dépourvu du plasmide pIP816 (Leclercq et al, 1988, N. Engl. J. Med. 319:157-161). La sonde hybridait avec le fragment d'ADN EcoRI de 4 kb de BM4147 mais pas avec l'ADN de E. faecium BM4147-1. Comme le gène vanA est porté par le fragment EcoRI de 4 kb de pIP816, ces résultats indiquent que les amorces permettent également l'amplification d'une partie de vanA. Ainsi les oligonucléotides V1 et V2 peuvent amplifier des fragments de gènes codant pour des différentes protéines apparentées aux D-Ala-D-Ala ligases, et ce dans des espèces différentes.

### Amplification, clonage et séquençage du gène vanC

Une amplification par PCR a été réalisée sur l'ADN total de. E. gallinarum BM4174 et le produit d'amplification obtenu, d'environ 640 bp a été cloné dans le bactériophaghe M13mp10. L'ADN simple brin isolé à partir du phage recombinant a été utilisé pour construire une sonde C (Hu et al, 1982, Gene 17:2171-2177). En analyse Southern la sonde hybridait avec un fragment PstI de 1,7 kb de BM4174 mais pas avec l'ADN de BM4147 et BM4147-1.

L'ADN de BM4174 a été digéré avec PstI et des fragments de 1,5 et 2 kb ont été purifiés par électrophorèse sur gel d'agarose et clonés dans pUC18 (Norrander et al, 1983, précité). Les plasmides recombinants ont été introduits dans E.coli JM83 par transformation et criblés par hybridation sur colonies (Sambrook et al, 1989, Molecular cloning, a laboratory manual. Cold Spring Harbor Laboratory Press, Cold Spring Harbor, NY) en utilisant la sonde C. Une homologie a été détectée avec un transformant hébergeant un plasmide, appelé pAT216, qui contenait un insérat PstI de 1,7 kb. La séquence de la partie de 1347 bp SacI-PstI de l'insérat de pAT216 a été mise en évidence sur les deux brins d'ADN. La localisation des codons de terminaison dans les trois cadres de lecture de chaque brin d'ADN a révélé la présence d'une phase ORF localisée entre les codons TGA aux positions 47 et 1244. Le codon d'initiation de transcription ATG en position 215 est précédé par une séquence GAAAGGAAGA caractéristique des séquences RBS complémentaire de l'ARN de la sous- unité 16S de B. subtilis (Moran et al, 1982, Mol. Gen. Genet. 186:339-346). La séquence de 1029 bp qui s'étend du codon ATG en position 215 au codon TGA en position 1244 pourrait coder une protéine de 343 acides aminés ayant une masse moléculaire calculée de 37504Da désignée par VanC. Une similitude de séquence a été détectée entre VanC, VanA et les ligases D-Ala-D-Ala de E.coli. En particulier quatre domaines de forte homologie préalablement trouvés entre VanA et les D-Ala-D-Ala ligases d'entérobactéries sont également présents dans VanC. Le pourcentage d'acides aminés identiques calculé pour ces protéines prises deux à deux était entre 29 et 38%. L'alignement des quatre séquences a révélé la présence de 57 acides aminés invariants qui incluent les résidus conservés des peptides 1 et 2 utilisés pour définir les sondes oligonucléotidiques V1 et V2.

### Inactivation par insertion du gène vanC

Pour évaluer la contribution de vanC à la résistance à la vancomycine chez E. gallinarum BM4174, le gène vanC a été inactivé par insertion. Un fragment de 690 bp EcoRI-HincII, interne à vanC a été clone dans pAT114 qui ne se réplique pas dans les bactéries gram-positives. Le plasmide pAT217 résultant a été introduit dans BM4174 par électrotransformation (Cruz-Rodz et al, 1990, Mol. Gen. Genet. 224:152-154) et les clones supposés résulter d'une recombinaison homologue conduisant à l'intégration de pAT217 dans vanC ont été sélectionnés sur de l'érythromycine. Le clone BM4175 a été comparé à BM4174 par hybridation Southern en utilisant la sonde C et aphA-3 spécifique de pAT114. Les deux sondes hybridaient avec le fragment EcoRI de 8,6 kb de BM4175. La sonde C hybridait avec un fragment de 2,5 kb de BM4174 alors qu'aucun signal n'était observé avec la sonde aphA-3. Les résultats indiquent que le plasmide pAT217 de 6,1 kb était intégré dans le gène vanC. La détermination de la concentration minimum inhibitrice de vancomycine pour BM4174 (16 mg/l) et BM4175 (2 mg/l) a indiqué que l'inactivation par insertion dans vanC abolit la résistance à la vancomycine.

VanC est donc requise pour la résistance à la vancomycine. On peut donc penser que cette protéine synthétise un dipeptide ou un depsipeptide qui est incorporé dans les précurseurs de peptidoglycanes et n'est pas reconnu par la vancomycine.

Les séquences qui font l'objet de l'invention sont données dans les pages suivantes après la liste des séquences contenant la description de ces séquences. Dans la liste des séquences, les protéines sont repérées par rapport à la position des bases nucléotidiques correspondant aux acides aminés des extrémités des protéines.

### Liste des séquences

(contenues dans les séquences I (Ia, Ib), II présentées ci-après ou dans la séquence de la figure 5).

### Séquences d'acides aminés

SEQ ID NO 1 (VanH) : séquence de la première protéine de résistance, correspondant à la séquence d'acides aminés de la phase de lecture ouverte n° 3 , commençant à la base 3501 et se terminant à la base 4529, contenant la séquence codante du gène vanH entre les bases 3564 et 4529, par rapport à la séquence de la figure 5 ou correspondant à la séquence entre les positions des nucléotides 6018 et 6983 de la séquence Ia.
SEQ ID NO 2 (VanA) : séquence de la protéine VanA, correspondant à la séquence d'acides aminés de la phase de lecture ouverte n°1, commençant à la base 4429 et se terminant à la base 5553, par rapport à la séquence de la figure 5 ou correspondant à la séquence entre les positions des nucléotides 6977 et 7807 de la séquence Ia.
SEQ ID NO 3 (VanX) : séquence de la troisième protéine de résistance, correspondant à la séquence d'acides aminés de la phase de lecture ouverte n° 3, commençant à la base 5526 et se terminant à la base 6167, par rapport à la séquence de la figure 5 ou correspondant à la séquence entre les positions des nucléotides 7816 et 8621 de la séquence Ia.
SEQ ID NO 4 (VanR) : séquence de la protéine de régulation R, correspondant à la séquence d'acides aminés de la phase de lecture n°1, commençant à la base 1477 et se terminant à la base 2214, par rapport à la séquence de la figure 5 ou correspondant à la séquence entre les positions des nucléotides 3976 et 4668 de la séquence Ia.
SEQ ID NO 5 (VanS) : séquence de la protéine senseur S, correspondant à la séquence d'acides aminés de la phase de lecture ouverte n°2, commençant à la base 2180 et se terminant à la base 3346, par rapport à la séquence de la figure 5 ou correspondant à la séquence entre les positions des nucléotides 4648 et 5800 de la séquence la.
SEQ ID NO 16 : séquence de la transposase correspondant aux acides aminés compris entre les nucléotides 150 et 3112 de la séquence Ib.
SEQ ID NO 17 : séquence de la résolvase comprenant les acides aminés situés entre les positions de nucléotides 3187 et 3759 de la séquence Ia.
SEQ ID NO 18 : séquence VanY comprenant les acides aminés situés entre les positions des nucléotides 9046 à 9960 dans la séquence Ia.
SEQ ID NO 19 : séquence VanZ comprenant les acides aminés situés entre les positions des nucléotides 10116 et 10598 dans la séquence Ia.
SEQ ID NO 20 : séquence VanC d'acides aminés représentée sur la liste II.

### - Séquences nucléotidiques

SEQ ID NO 6 : séquence nucléotidique contenant la séquence codant pour les 5 protéines, ainsi que les séquences flanquantes, représentée à la figure 5.
SEQ ID NO 7 : séquence contenant la séquence codant pour les 3 protéines de résistance, ainsi que les séquences flanquantes et commençant à la base 3501 et se terminant à la base 6167, représentée à la figure 5.
SEQ ID NO 8 : séquence du gène van A, commençant à la base 4429 et se terminant à la base 5553, de la séquence présentée à la figure 5, ou correspondant à la séquence nucléotidique située entre les nucléotides 6977 et 7807 de la séquence Ia.
SEQ ID NO 9 : séquence codant pour la première protéine de résistance appelée VanH, commençant à la base 3501 et se terminant à la base 4529, en particulier la séquence vanH dont la séquence codante est localisée entre les bases 3564 et 4529, de la séquence présentée à la figure 5, ou correspondant à la séquence nucléotidique située entre les nucléotides 6018 et 6983 de la séquence Ia.
SEQ ID NO 10 : séquence codant pour la troisième protéine de résistance VanX, commençant à la base 5526 et se terminant à la base 6167, de la séquence présentée à la figure 5, ou correspondant à la séquence nucléotidique située entre les nucléotides 7816 et 8621 de la séquence Ia.
SEQ ID NO 11 : séquence du transposon codant pour la transposase, la résolvase, VanR, VanS, VanH, VanA, VanX, VanY et VanZ et contenant la séquence invsersée répétée de 38 pb à ses extrémités N- et C-terminales, et correspondant à la séquence Ia.
SEQ ID NO 12 : séquence codant pour la transposase, commençant à la base 150 et se terminant à la base 3112 de la séquence Ib.
SEQ ID NO 13 : séquence codant pour la résolvase, commençant à la base 3187 et se terminant à la base 3759 de la séquence Ia.
SEQ ID NO 14 : séquence codant pour VanY commençant à la base 9046 et se terminant à la base 9960 de la séquence Ia.
SEQ ID NO 15 : séquence codant pour VanZ commençant à la base 10116 et se terminant à la base 10598 de la séquence Ia.
SEQ ID NO 21 : séquence codant pour VanC, représentée sur la liste II en correspondance avec la protéine VanC.
SEQ ID NO 22 : séquence complète la du transposon de E. faecium commençant à la base 1 et se terminant à la base 10851.
SEQ ID NO 23 : séquence codant pour la protéine VanR, commençant à la base 3976 et se terminant à la base 4668 de la séquence Ia.
SEQ ID NO 24 : séquence codant pour la protéine VanS, commençant à la base 4648 et se terminant à la base 5800 de la séquence Ia.

I. Séquence nucléotidique du transposon et traduction.

Ia. brin "+"

1
GGG GTA GCG TCA GGA AAA TGC GGA TTT ACA ACG CTA AGC CTA TTT TCC TGA CGA ATC CCT
61
CGT TTT TAA CAA CGT TAA GAA AGT TTT AGT GGT CTT AAA GAA TTT AAT GAG ACT ACT TTC
121
TCT GAG TTA AAA TGG TAT TCT CCT AGT AAA TTA ATA TGT TCC CAA CCT AAG GGC GAC ATA
181
TGG TGT AAC AAA TCT TCA TTA AAG CTA CCT GTC CGT TTT TTA TAT TCA ACT GCT GTT GTT
241
AGG TGG AGA GTA TTC CAA ATA CTT ATA GCA TTG ATA ATT ATG TTT AAA GCA CTG GCT CTT
301
TGC AAT TGA TGC TGT ATG GTG CGT TCT CTA AGC TCA CCT TGT TTT CCG AAG AAA ATA GCT
361
CTT GCC AAT CCA TTC ATG GCT TCT CCT TTA TTC AAT CCT CTT TGT ATT TTT CTT CTT AAT
421
GAT TCA TCC GAT ATA TAA TTC AAA ATA AAG ATC GTT TTT TCT ATT CGG CCC ATC TCA CGT
481
AAG GCT GTA GCT AAG CTG TTT TGT CTT GAA TAG GAA CCT AGC TTC CCC ATA ATA AGG GAT
541
GCT GAA ACT GTT CCC TCC CTT ATA GAA TGA GCT AAT CGC AAA ACA TCC TCA TAA TTT TCT
601
TTA ATG ACC TTT GTA TTT ATT TGT CCA CGT AAA ATG GCT TCT AGT TTT GGA TAC TCA CTT

661
GCT TTA TCT ATC GTA AAT AAT TTT GAG TCC GAT AAA TCC CTT ATT CTT GGG GCA AAT TTA
721
AAT CCT AAT AAA TGA GTC AGT CCG AAT ATT TGG TCA GTG TAA CCG GCA GTG TCT GTA TAA
781
TGT TCC TCT ATG TTT AGA TCC GTC TCA TGA TGT AAC AAA CCA TCC AAA ACA TGA ATC GCA
841
TCT CTT GAA TTA GTA TGA ATA ATC TTT GTG TAG TAA GAA GAG AAT TGA TCA CTT GTA AAT
901
CGG TAG ATG GTG GCT CCT TTT CCA GTT CCA TAA TGT GGA TTT GCA TCT GCA TGT AGT GAT
961
GAA ACA CCT AGC TGC ATT CTC ATA CCA TCT GAC GAA GAT GTT GTA CCG TCG CCC CAA TAG
1021
AAA GGC AAT TGT AAT TTA TGA TGA AAG TTT ACT AAT ATG GCT TGG GCT TTA TTC ATG GCA
1081
TCT TCA TAC ATG CGC CAT TGA GAT ACA TTG GCT AGT TGC TTA TAT GTA AGT CCG GGT GTG
1141
GCT TCG GCC ATC TTG CTC AAG CCA ATA TTC ATT CCC ATT CCT AAA AGG GCA GCC ATG ATA
1201
ATG ATT GTT TCT TCC TTA TCT GGT TTT CGA TTA TTG GAA GCA TGA GTG AAT TGC TCA TGA
1261
AAT CCT GTT ATA TGG GCC ACA TCC ATG AGT AAA TCA GTT AAT TTT ATT CTT GGT AGC ATC
1321
TGA TAA AGG CTT GCA CTA AAT TTT TTT GCT TCT TCT GGA ACA TCT TTT TCT AAG CGT GCA
1381
AGT GAT AGC TTT CCT TTT TCA AGA GAA ACC CCA TCT AAC TTA TTG GAA TTG GCA GCT AAC
1441
CAC TTT AAC CTT TCA TTA AAG CTG CTG GTT CTC TCC GTT ATA TAA TCT TCG AAT GAT AAA

1501
CTA ACT GAT AAT CTC GTA TTC CCC TTC GAT TGA TTC CAT GTA TCT TCC GAA AAC AAA TAT
1561
TCC TCA AAA TCC CTA TAT TGT CTG CTG CCA ACA ATG GAA ACA TCT CCT GCC CGA ACA TGC
1621
TCC CGA AGT TCT GTT AAA ACA GCC ATT TCA TAG TAA TGA CGA TTA ATT GTT GTA CCA TCA
1681
TCC TCG TAT AAA TGT CTT TTC CAT CGT TTT GAA ATA AAA TCC ACA GGT GAG TCA TCA GGC
1741
ACT TTT CGC TTT CCA GAT TCG TTC ATT CCT CGG ATA ATC TCA ACA GCT TGT AAA AGT GGC
1801
TCA TTT GCC TTT GTA GAA TGA AAT TCC AAT ACT CTT AAT AGC GTT GGC GTA TAT TTT CTT
1861
AGT GAA TAA AAC CGT TTT TGC AGT AAG TCT AAA TAA TCA TAG TCG GCA GGA CGT GCA AGT
1921
TCC TGA GCC TCT TCT ACT GAA GAG ACA AAG GTA TTC CAT TCA ATA ACC GAT TCT AAA ACC
1981
TTA AAA ACG TCT AAT TTT TCC TCT CTT GCT TTA ATT AAT GCT TGT CCG ATG TTC GTA AAG
2041
TGT ATA ACT TTC TCA TTT AGC TTT TTA CCG TTT TGT TTC TGG ATT TCC TCT TGA GCC TTA
2101
CGA CCT TTT GAT AAC AAA CTA AGT ATT TGC CTA TCA TGA ATT TCA AAC GCT TTA TCC GTT
2161
AGC TCC TGA GTA AGT TGT AAT AAA TAG ATG GTT AAT ATC GAA TAA CGT TTA TTT TCT TGA
2221
AAG TCA CGG AAT GCA TAC GGC TCG TAT CTT GAG CCT AAG CGA GAC AGC TGC AAC AGG CGG
2281
TTA CGG TGC AAA TGA CTA ATT TGC ACT GTT TCT AAA TCC ATT CCT CGT ATG TAT TCG AGT
2341
CGT TCT ATT ATT TTT AGA AAA GTT TCG GGT GAA GGA TGA CCC GGT GGC TCT TTT AAC CAA

2401
CCC AAT ATC GTT TTA TTG GAT TCG GAT GGA TGC TGC GAG GTA ATA ATC CCT TCA AGC TTT
2461
TCT TTT TGC TCA TTT GTT AGA GAT TTA CTA ACC GTA TTA AAT AGC TTC TTT TCA GCC ATT
2521
GCC CTT GCT TCC CAC ACC ATT CTT TCA AGT GTA GTG ATA GCA GGC AGT ATA ATT TTG TTT
2581
TTT CTT AGA AAA TCT ATG CAT TCA TGC AGT AGA TGA ATG GCA TCA CCA TTT TCC AAA GCT
2641
AAT TGA TGA AGG TAC TTA AAT GTC ATT CGA TAT TCA CTC AGG GTA AAA GTT ACA AAG TCG
2701
TAT TCA CTT CGA ATT TCT TTC AAA TGA TCC CAA AGT GTA TTT TCC CTT TGA GGA TAA TGA
2761
TCA AGC GAG GAT GGA CTA ACA CCA ATC TGT TTC GAT ATA TAT TGT ATG ACC GAA TCT GGG
2821
ATG CTT TTG ATA TGA GTG TAT GGC CAA CCG GGA TAC CGA AGA ACA GCT AAT TGA ACA GCA
2881
AAT CCT AAA CGG TTT TCT TCC CTC CTT CGC TTA TTA ACT ATT TCT AAA TCC CGT TTG GAA
2941
AAA GTG AAG TAG GTC CCC AGT ATC CAT TCA TCT TCA GGG ATT TGC ATA AAA GCC TGT CTC
3001
TGT TCC GGT GTA AGC AAT TCT CTA CCT CTC GCA ATT TTC ATT CAG TAT CAT TCC ATT TCT
3061
GTA TTT TCA ATT TAT TAG TTC AAT TAT ATA TCA ATA GAG TGT ACT CTA TTG ATA CAA ATG
3121
TAG TAG ACT GAT AAA ATC ATA GTT AAG AGC GTC TCA TAA GAC TTG TCT CAA AAA TGA GGT

3181        résolvase
        LEU ARG LYS ILE GLY TYR ILE ARG VAL SER SER THR ASN GLN ASN PRO SER ARG
GAT ATT TTG CGG AAA ATC GGT TAT ATT CGT GTC AGT TCG ACT AAC CAG AAT CCT TCA AGA
3241
GLN PHE GLN GLN LEU ASN GLU ILE GLY MET ASP ILE ILE TYR GLU GLU LYS VAL SER GLY
CAA TTT CAG CAG TTG AAC GAG ATC GGA ATG GAT ATT ATA TAT GAA GAG AAA GTT TCA GGA
3301
ALA THR LYS ASP ARG GLU GLN LEU GLN LYS VAL LEU ASP ASP LEU GLN GLU ASP ASP ILE
GCA ACA AAG GAT CGC GAG CAA CTT CAA AAA GTG TTA GAC GAT TTA CAG GAA GAT GAC ATC
3361
ILE TYR VAL THR ASP LEU THR ARG ILE THR ARG SER THR GLN ASP LEU PHE GLU LEU ILE
ATT TAT GTT ACA GAC TTA ACT CGA ATC ACT CGT AGT ACA CAA GAT CTA TTT GAA TTA ATC
3421
ASP ASN ILE ARG ASP LYS LYS ALA SER LEU LYS SER LEU LYS ASP THR TRP LEU ASP LEU
GAT AAC ATA CGA GAT AAA AAG GCA AGT TTA AAA TCA CTA AAA GAT ACA TGG CTT GAT TTA
3481
SER GLU ASP ASN PRO TYR SER GLN PHE LEU ILE THR VAL MET ALA GLY VAL ASN GLN LEU
TCA GAA GAT AAT CCA TAC AGC CAA TTC TTA ATT ACT GTA ATG GCT GGT GTT AAC CAA TTA
3541
GLU ARG ASP LEU ILE ARG MET ARG GLN ARG GLU GLY ILE GLU LEU ALA LYS LYS GLU GLY
GAG CGA GAT CTT ATT CGG ATG AGA CAA CGT GAA GGG ATT GAA TTG GCT AAG AAA GAA GGA
3601
LYS PHE LYS GLY ARG LEU LYS LYS TYR HIS LYS ASN HIS ALA GLY MET ASN TYR ALA VAL
AAG TTT AAA GGT CGA TTA AAG AAG TAT CAT AAA AAT CAC GCA GGA ATG AAT TAT GCG GTA
3661
LYS LEU TYR LYS GLU GLY ASN MET THR VAL ASN GLN ILE CYS GLU ILE THR ASN VAL SER
AAG CTA TAT AAA GAA GGA AAT ATG ACT GTA AAT CAA ATT TGT GAA ATT ACT AAT GTA TCT
3721
ARG ALA SER LEU TYR ARG LYS LEU SER GLU VAL ASN ASN ASN
AGG GCT TCA TTA TAC AGG AAA TTA TCA GAA GTG AAT AAT TAG CCA TTC TGT ATT CCG CTA

3781
ATG GGC AAT ATT TTT AAA GAA GAA AAG GAA ACT ATA AAA TAT TAA CAG CCT CCT AGC GAT
3841
GCC GAA AAG CCC TTT GAT AAA AAA AGA ATC ATC ATC TTA AGA AAT TCT TAG TCA TTT ATT
3901
ATG TAA ATG CTT ATA AAT TCG GCC CTA TAA TCT GAT AAA TTA TTA AGG GCA AAC TTA TGT

3961 VanR MET SER ASP LYS ILE LEU ILE VAL ASP ASP GLU HIS GLU ILE ALA
GAA AGG GTG ATA ACT ATG AGC GAT AAA ATA CTT ATT GTG GAT GAT GAA CAT GAA ATT GCC
4021
ASP LEU VAL GLU LEU TYR LEU LYS ASN GLU ASN TYR THR VAL PHE LYS TYR TYR THR ALA
GAT TTG GTT GAA TTA TAC TTA AAA AAC GAG AAT TAT ACG GTT TTC AAA TAC TAT ACC GCC
4081
LYS GLU ALA LEU GLU CYS ILE ASP LYS SER GLU ILE ASP LEU ALA ILE LEU ASP ILE MET
AAA GAA GCA TTG GAA TGT ATA GAC AAG TCT GAG ATT GAC CTT GCC ATA TTG GAC ATC ATG
4141
LEU PRO GLY THR SER GLY LEU THR ILE CYS GLN LYS ILE ARG ASP LYS HIS THR TYR PRO
CTT CCC GGC ACA AGC GGC CTT ACT ATC TGT CAA AAA ATA AGG GAC AAG CAC ACC TAT CCG
4201
ILE ILE MET LEU THR GLY LYS ASP THR GLU VAL ASP LYS ILE THR GLY LEU THR ILE GLY
ATT ATC ATG CTG ACC GGG AAA GAT ACA GAG GTA GAT AAA ATT ACA GGG TTA ACA ATC GGC
4261
ALA ASP ASP TYR ILE THR LYS PRO PHE ARG PRO LEU GLU LEU ILE ALA ARG VAL LYS ALA
GCG GAT GAT TAT ATA ACG AAG CCC TTT CGC CCA CTG GAG TTA ATT GCT CGG GTA AAG GCC
4321
GLN LEU ARG ARG TYR LYS LYS PHE SER GLY VAL LYS GLU GLN ASN GLU ASN VAL ILE VAL
CAG TTG CGC CGA TAC AAA AAA TTC AGT GGA GTA AAG GAG CAG AAC GAA AAT GTT ATC GTC

4381
HIS SER GLY LEU VAL ILE ASN VAL ASN THR HIS GLU CYS TYR LEU ASN GLU LYS GLN LEU
CAC TCC GGC CTT GTC ATT AAT GTT AAC ACC CAT GAG TGT TAT CTG AAC GAG AAG CAG TTA
4441
SER LEU THR PRO THR GLU PHE SER ILE LEU ARG ILE LEU CYS GLU ASN LYS GLY ASN VAL
TCC CTT ACT CCC ACC GAG TTT TCA ATA CTG CGA ATC CTC TGT GAA AAC AAG GGG AAT GTG
4501
VAL SER SER GLU LEU LEU PHE HIS GLU ILE TRP GLY ASP GLU TYR PHE SER LYS SER ASN
GTT AGC TCC GAG CTG CTA TTT CAT GAG ATA TGG GGC GAC GAA TAT TTC AGC AAG AGC AAC
4561
ASN THR ILE THR VAL HIS ILE ARG HIS LEU ARG GLU LYS MET ASN ASP THR ILE ASP ASN
AAC ACC ATC ACC GTG CAT ATC CGG CAT TTG CGC GAA AAA ATG AAC GAC ACC ATT GAT AAT
4621
PRO LYS TYR ILE LYS THR VAL TRP GLY VALGLYTYRLYSILEGLULYS
CCG AAA TAT ATA AAA ACG GTA TGG GGG GTTGGTTATAAAATTGAAAAAT AAA AAC GAC
VanS LEUVALILELEULYSLEULYSASN LYS ASN ASP
4682
TYR SER LYS LEU GLU ARG LYS LEU TYR MET TYR ILE VAL ALA ILE VAL VAL VAL ALA ILE
TAT TCC AAA CTA GAA CGA AAA CTT TAC ATG TAT ATC GTT GCA ATT GTT GTG GTA GCA ATT
4742
VAL PHE VAL LEU TYR ILE ARG SER MET ILE ARG GLY LYS LEU GLY ASP TRP ILE LEU SER
GTA TTC GTG TTG TAT ATT CGT TCA ATG ATC CGA GGG AAA CTT GGG GAT TGG ATC TTA AGT
4802
ILE LEU GLU ASN LYS TYR ASP LEU ASN HIS LEU ASP ALA MET LYS LEU TYR GLN TYR SER
ATT TTG GAA AAC AAA TAT GAC TTA AAT CAC CTG GAC GCG ATG AAA TTA TAT CAA TAT TCC
4862
ILE ARG ASN ASN ILE ASP ILE PHE ILE TYR VAL ALA ILE VAL ILE SER ILE LEU ILE LEU
ATA CGG AAC AAT ATA GAT ATC TTT ATT TAT GTG GCG ATT GTC ATT AGT ATT CTT ATT CTA
4922
CYS ARG VAL MET LEU SER LYS PHE ALA LYS TYR PHE ASP GLU ILE ASN THR GLY ILE ASP
TGT CGC GTC ATG CTT TCA AAA TTC GCA AAA TAC TTT GAC GAG ATA AAT ACC GGC ATT GAT

4982
VAL LEU ILE GLN ASN GLU ASP LYS GLN ILE GLU LEU SER ALA GLU MET ASP VAL MET GLU
GTA CTT ATT CAG AAC GAA GAT AAA CAA ATT GAG CTT TCT GCG GAA ATG GAT GTT ATG GAA
5042
GLN LYS LEU ASN THR LEU LYS ARG THR LEU GLU LYS ARG GLU GLN ASP ALA LYS LEU ALA
CAA AAG CTC AAC ACA TTA AAA CGG ACT CTG GAA AAG CGA GAG CAG GAT GCA AAG CTG GCC
5102
GLU GLN ARG LYS ASN ASP VAL VAL MET TYR LEU ALA HIS ASP ILE LYS THR PRO LEU THR
GAA CAA AGA AAA AAT GAC GTT GTT ATG TAC TTG GCG CAC GAT ATT AAA ACG CCC CTT ACA
5162
SER ILE ILE GLY TYR LEU SER LEU LEU ASP GLU ALA PRO ASP MET PRO VAL ASP GLN LYS
TCC ATT ATC GGT TAT TTG AGC CTG CTT GAC GAG GCT CCA GAC ATG CCG GTA GAT CAA AAG
5222
ALA LYS TYR VAL HIS ILE THR LEU ASP LYS ALA TYR ARG LEU GLU GLN LEU ILE ASP GLU
GCA AAG TAT GTG CAT ATC ACG TTG GAC AAA GCG TAT CGA CTC GAA CAG CTA ATC GAC GAG
5282
PHE PHE GLU ILE THR ARG TYR ASN LEU GLN THR ILE THR LEU THR LYS THR HIS ILE ASP
TTT TTT GAG ATT ACA CGG TAT AAC CTA CAA ACG ATA ACG CTA ACA AAA ACG CAC ATA GAC
5342
LEU TYR TYR MET LEU VAL GLN MET THR ASP GLU PHE TYR PRO GLN LEU SER ALA HIS GLY
CTA TAC TAT ATG CTG GTG CAG ATG ACC GAT GAA TTT TAT CCT CAG CTT TCC GCA CAT GGA
5402
LYS GLN ALA VAL ILE HIS ALA PRO GLU ASP LEU THR VAL SER GLY ASP PRO ASP LYS LEU
AAA CAG GCG GTT ATT CAC GCC CCC GAG GAT CTG ACC GTG TCC GGC GAC CCT GAT AAA CTC
5462
ALA ARG VAL PHE ASN ASN ILE LEU LYS ASN ALA ALA ALA TYR SER GLU ASP ASN SER ILE
GCG AGA GTC TTT AAC AAC ATT TTG AAA AAC GCC GCT GCA TAC AGT GAG GAT AAC AGC ATC

5522
ILE ASP ILE THR ALA GLY LEU SER GLY ASP VAL VAL SER ILE GLU PHE LYS ASN THR GLY
ATT GAC ATT ACC GCG GGC CTC TCC GGG GAT GTG GTG TCA ATC GAA TTC AAG AAC ACT GGA
5582
SER ILE PRO LYS ASP LYS LEU ALA ALA ILE PHE GLU LYS PHE TYR ARG LEU ASP ASN ALA
AGC ATC CCA AAA GAT AAG CTA GCT GCC ATA TTT GAA AAG TTC TAT AGG CTG GAC AAT GCT
5642
ARG SER SER ASP THR GLY GLY ALA GLY LEU GLY LEU ALA ILE ALA LYS GLU ILE ILE VAL
CGT TCT TCC GAT ACG GGT GGC GCG GGA CTT GGA TTG GCG ATT GCA AAA GAA ATT ATT GTT
5702
GLN HIS GLY GLY GLN ILE TYR ALA GLU SER ASN ASP ASN TYR THR THR PHE ARG VAL GLU
CAG CAT GGA GGG CAG ATT TAC GCG GAA AGC AAT GAT AAC TAT ACG ACG TTT AGG GTA GAG
5762
LEU PRO ALA MET PRO ASP LEU VAL ASP LYS ARG ARG SER
CTT CCA GCG ATG CCA GAC TTG GTT GAT AAA AGG AGG TCC TAA GA GAT GTA TAT AAT TTT
5821
TTA GGA AAA TCT CAA GGT TAT CTT TAC TTT TTC TTA GGA AAT TAA CAA TTT AAT ATT AAG
5881
AAA CGG CTC GTT CTT ACA CGG TAG ACT TAA TAC CGT AAG AAC GAG CCG TTT TCG TTC TTC
5941
AGA GAA AGA TTT GAC AAG ATT ACC ATT GGC ATC CCC GTT TTA TTT GGT GCC TTT CAC AGA
6001
VanH MET ASN ASN ILE GLY ILE THR VAL TYR GLY CYS GLU GLN ASP GLU
AAGGGTTGG TCT TAA TT ATG AAT AAC ATC GGC ATT ACT GTT TAT GGA TGT GAG CAG GAT GAG
6063
ALA ASP ALA PHE HIS ALA LEU SER PRO ARG PHE GLY VAL MET ALA THR ILE ILE ASN ALA
GCA GAT GCA TTC CAT GCT CTT TCG CCT CGC TTT GGC GTT ATG GCA ACG ATA ATT AAC GCC

6123
ASN VAL SER GLU SER ASN ALA LYS SER ALA PRO PHE ASN GLN CYS ILE SER VAL GLY HIS
AAC GTG TCG GAA TCC AAC GCC AAA TCC GCG CCT TTC AAT CAA TGT ATC AGT GTG GGA CAT
6183
LYS SER GLU ILE SER ALA SER ILE LEU LEU ALA LEU LYS ARG ALA GLY VAL LYS TYR ILE
AAA TCA GAG ATT TCC GCC TCT ATT CTT CTT GCG CTG AAG AGA GCC GGT GTG AAA TAT ATT
6243
SER THR ARG SER ILE GLY CYS ASN HIS ILE ASP THR THR ALA ALA LYS ARG MET GLY ILE
TCT ACC CGA AGC ATC GGC [?]GC AAT CAT ATA GAT ACA ACT GCT GCT AAG AGA ATG GGC ATC
6303
THR VAL ASP ASN VAL ALA TYR SER PRO ASP SER VAL ALA ASP TYR THR MET MET LEU ILE
ACT GTC GAC AAT GTG GCG TAC TCG CCG GAT AGC GTT GCC GAT TAT ACT ATG ATG CTA ATT
6363
LEU MET ALA VAL ARG ASN VAL LYS SER ILE VAL ARG SER VAL GLU LYS HIS ASP PHE ARG
CTT ATG GCA GTA CGC AAC GTA AAA TCG ATT GTG CGC TCT GTG GAA AAA CAT GAT TTC AGG
6423
LEU ASP SER ASP ARG GLY [?]YS VAL LEU SER ASP MET THR VAL GLY VAL VAL GLY THR GLY
TTG GAC AGC GAC CGT GGC [?]AG GTA CTC AGC GAC ATG ACA GTT GGT GTG GTG GGA ACG GGC
6483
GLN ILE GLY LYS ALA VAL ILE GLU ARG LEU ARG GLY PHE GLY CYS LYS VAL LEU ALA TYR
CAG ATA GGC AAA GCG GTT ATT GAG CGG CTG CGA GGA TTT GGA TGT AAA GTG TTG GCT TAT
6543
SER ARG SER ARG SER ILE GLU VAL ASN TYR VAL PRO PHE ASP GLU LEU LEU GLN ASN SER
AGT CGC AGC CGA AGT ATA GAG GTA AAC TAT GTA CCG TTT GAT GAG TTG CTG CAA AAT AGC
6603
ASP ILE VAL THR LEU HIS VAL PRO LEU ASN THR ASP THR HIS TYR ILE ILE SER HIS GLU
GAT ATC GTT ACG CTT CAT GTG CCG CTC AAT ACG GAT ACG CAC TAT ATT ATC AGC CAC GAA
6663
GLN ILE GLN ARG MET LYS GLN GLY ALA PHE LEU ILE ASN THR GLY ARG GLY PRO LEU VAL
CAA ATA CAG AGA ATG AAG CAA GGA GCA TTT CTT ATC AAT ACT GGG CGC GGT CCA CTT GTA

6723
ASP THR TYR GLU LEU VAL LYS ALA LEU GLU ASN GLY LYS LEU GLY GLY ALA ALA LEU ASP
GAT ACC TAT GAG TTG GTT AAA GCA TTA GAA AAC GGG AAA CTG GGC GGT GCC GCA TTG GAT
6783
VAL LEU GLU GLY GLU GLU GLU PHE PHE TYR SER ASP CYS THR GLN LYS PRO ILE ASP ASN
GTA TTG GAA GGA GAG GAA GAG TTT TTC TAC TCT GAT TGC ACC CAA AAA CCA ATT GAT AAT
6843
GLN PHE LEU LEU LYS LEU GLN ARG MET PRO ASN VAL ILE ILE THR PRO HIS THR ALA TYR
CAA TTT TTA CTT AAA CTT CAA AGA ATG CCT AAC GTG ATA ATC ACA CCG CAT ACG GCC TAT
6903
TYR THR GLU GLN ALA LEU ARG ASP THR VAL GLU LYS THR ILE LYS ASN CYS LEU ASP PHE
TAT ACC GAG CAA GCG TTG CGT GAT ACC GTT GAA AAA ACC ATT AAA AAC TGT TTG GAT TTT
6963
VanA
METASN ARG ILE LYS VAL ALA ILE LEU PHE GLY GLY CYS SER
GAA AGG AGA CAG GAG CATGAAT AGA ATA AAA GTT GCA ATA CTG TTT GGG GGT TGC TCA
GLU ARG ARG GLN GLU HISGLU
7021
GLU GLU HIS ASP VAL SER VAL LYS SER ALA ILE GLU ILE ALA ALA ASN ILE ASN LYS GLU
GAG GAG CAT GAC GTA TCG GTA AAA TCT GCA ATA GAG ATA GCC GCT AAC ATT AAT AAA GAA
7081
LYS TYR GLU PRO LEU TYR ILE GLY ILE THR LYS SER GLY VAL TRP LYS MET CYS GLU LYS
AAA TAC GAG CCG TTA TAC ATT GGA ATT ACG AAA TCT GGT GTA TGG AAA ATG TGC GAA AAA
7141
PRO CYS ALA GLU TRP GLU ASN ASP ASN CYS TYR SER ALA VAL LEU SER PRO ASP LYS LYS
CCT TGC GCG GAA TGG GAA AAC GAC AAT TGC TAT TCA GCT GTA CTC TCG CCG GAT AAA AAA
7201
MET HIS GLY LEU LEU VAL LYS LYS ASN HIS GLU TYR GLU ILE ASN HIS VAL ASP VAL ALA
ATG CAC GGA TTA CTT GTT AAA AAG AAC CAT GAA TAT GAA ATC AAC CAT GTT GAT GTA GCA

7261
PHE SER ALA LEU HIS GLY LYS SER GLY GLU ASP GLY SER ILE GLN GLY LEU PHE GLU LEU
TTT TCA GCT TTG CAT GGC AAG TCA GGT GAA GAT GGA TCC ATA CAA GGT CTG TTT GAA TTG
7321
SER GLY ILE PRO PHE VAL GLY CYS ASP ILE GLN SER SER ALA ILE CYS MET ASP LYS SER
TCC GGT ATC CCT TTT GTA GGC TGC GAT ATT CAA AGC TCA GCA ATT TGT ATG GAC AAA TCG
7381
LEU THR TYR ILE VAL ALA LYS ASN ALA GLY ILE ALA THR PRO ALA PHE TRP VAL ILE ASN
TTG ACA TAC ATC GTT GCG AAA AAT GCT GGG ATA GCT ACT CCC GCC TTT TGG GTT ATT AAT
7441
LYS ASP ASP ARG PRO VAL ALA ALA THR PHE THR TYR PRO VAL PHE VAL LYS PRO ALA ARG
AAA GAT GAT AGG CCG GTG GCA GCT ACG TTT ACC TAT CCT GTT TTT GTT AAG CCG GCG CGT
7501
SER GLY SER SER PHE GLY VAL LYS LYS VAL ASN SER ALA ASP GLU LEU ASP TYR ALA ILE
TCA GGC TCA TCC TTC GGT GTG AAA AAA GTC AAT AGC GCG GAC GAA TTG GAC TAC GCA ATT
7561
GLU SER ALA ARG GLN TYR ASP SER LYS ILE LEU ILE GLU GLN ALA VAL SER GLY CYS GLU
GAA TCG GCA AGA CAA TAT GAC AGC AAA ATC TTA ATT GAG CAG GCT GTT TCG GGC TGT GAG
7621
VAL GLY CYS ALA VAL LEU GLY ASN SER ALA ALA LEU VAL VAL GLY GLU VAL ASP GLN ILE
GTC GGT TGT GCG GTA TTG GGA AAC AGT GCC GCG TTA GTT GTT GGC GAG GTG GAC CAA ATC
7681
ARG LEU GLN TYR GLY ILE PHE ARG ILE HIS GLN GLU VAL GLU PRO GLU LYS GLY SER GLU
AGG CTG CAG TAC GGA ATC TTT CGT ATT CAT CAG GAA GTC GAG CCG GAA AAA GGC TCT GAA
7741
ASN ALA VAL ILE THR VAL PRO ALA ASP LEU SER ALA GLU GLU ARG GLY ARG ILE GLN GLU
AAC GCA GTT ATA ACC GTT CCC GCA GAC CTT TCA GCA GAG GAG CGA GGA CGG ATA CAG GAA
7801
THR ALA LYS LYS ILE TYR LYS ALA LEU GLY CYS ARG GLY LEU ALA ARG VAL ASP MET PHE
ACG GCA AAA AAA ATA TAT AAA GCG CTC GGC TGT AGA GGT CTA GCC CGT GTG GAT ATG TTT

7861
LEU GLN ASP ASN GLY ARG ILE VAL LEU ASN GLU VAL ASN THR LEU PRO GLY PHE THR SER
TTA CAA GAT AAC GGC CGC ATT GTA CTG AAC GAA GTC AAT ACT CTG CCC GGT TTC ACG TCA
7921
TYR SER ARG TYR PRO ARG MET MET ALA ALA ALA GLY ILE ALA LEU PRO GLU LEU ILE ASP
TAC AGT CGT TAT CCC CGT ATG ATG GCC GCT GCA GGT ATT GCA CTT CCC GAA CTG ATT GAC
7981
ARG LEU ILE VAL LEU ALA LEU LYS GLY
CGC TTG ATC GTA TTA GCG TTA AAG GGG TGATAAGC ATG GAA ATA GGA TTT ACT TTT TTA GAT
VanX MET GLU ILE GLY PHE THR PHE LEU ASP
8043
GLU ILE VAL HIS GLY VAL ARG TRP ASP ALA LYS TYR ALA THR TRP ASP ASN PHE THR GLY
GAA ATA GTA CAC GGT GTT CGT TGG GAC GCT AAA TAT GCC ACT TGG GAT AAT TTC ACC GGA
8103
LYS PRO VAL ASP GLY TYR GLU VAL ASN ARG ILE VAL GLY THR TYR GLU LEU ALA GLU SER
AAA CCG GTT GAC GGT TAT GAA GTA AAT CGC ATT GTA GGG ACA TAC GAG TTG GCT GAA TCG
8163
LEU LEU LYS ALA LYS GLU LEU ALA ALA THR GLN GLY TYR GLY LEU LEU LEU TRP ASP GLY
CTT TTG AAG GCA AAA GAA CTG GCT GCT ACC CAA GGG TAC GGA TTG CTT CTA TGG GAC GGT
8223
TYR ARG PRO LYS ARG ALA VAL ASN CYS PHE MET GLN TRP ALA ALA GLN PRO GLU ASN ASN
TAC CGT CCT AAG CGT GCT GTA AAC TGT TTT ATG CAA TGG GCT GCA CAG CCG GAA AAT AAC
8283
LEU THR LYS GLU SER TYR TYR PRO ASN ILE ASP ARG THR GLU MET ILE SER LYS GLY TYR
CTG ACA AAG GAA AGT TAT TAT CCC AAT ATT GAC CGA ACT GAG ATG ATT TCA AAA GGA TAC
8343
VAL ALA SER LYS SER SER HIS SER ARG GLY SER ALA ILE ASP LEU THR LEU TYR ARG LEU
GTG GCT TCA AAA TCA AGC CAT AGC CGC GGC AGT GCC ATT GAT CTT ACG CTT TAT CGA TTA

8403
ASP THR GLY GLU LEU VAL PRO MET GLY SER ARG PHE ASP PHE MET ASP GLU ARG SER HIS
GAC ACG GGT GAG CTT GTA CCA ATG GGG AGC CGA TTT GAT TTT ATG GAT GAA CGC TCT CAT
8463
HIS ALA ALA ASN GLY ILE SER CYS ASN GLU ALA GLN ASN ARG ARG ARG LEU ARG SER ILE
CAT GCG GCA AAT GGA ATA TCA TGC AAT GAA GCG CAA AAT CGC AGA CGT TTG CGC TCC ATC
8523
MET GLU ASN SER GLY PHE GLU ALA TYR SER LEU GLU TRP TRP HIS TYR VAL LEU ARG ASP
ATG GAA AAC AGT GGG TTT GAA GCA TAT AGC CTC GAA TGG TGG CAC TAT GTA TTA AGA GAC
8583
GLU PRO TYR PRO ASN SER TYR PHE ASP PHE PRO VAL LYS
GAA CCA TAC CCC AAT AGC TAT TTT GAT TTC CCC GTT AAA TAAA CTT TTA ACC GTT GCA
8641
CGG ACA AAC TAT ATA AGC TAA CTC TTT CGG CAG GAA ACC CGA CGT ATG TAA CTG GTT CTT
8701
AGG GAA TTT ATA TAT AGT AGA TAG TAT TGA AGA TGT AAG GCA GAG CGA TAT TGC GGT CAT
8761
TAT CTG CGT GCG CTG CGG CAA GAT AGC CTG ATA ATA AGA CTG ATC GCA TAG AGG GGT GGT
8821
ATT TCA CAC CGC CCA TTG TCA ACA GGC AGT TCA GCC TCG TTA AAT TCA GCA TGG GTA TCA
8881
CTT ATG AAA ATT CAT CTA CAT TGG TGA TAA TAG TAA ATC CAG TAG GGC GAA ATA ATT GAC
8941
TGT AAT TTA CGG GGC AAA ACG GCA CAA TCT CAA ACG AGA TTG TGC CGT TTA AGG GGA AGA
9001
VanY MET LYS LYS
TTC TAG AAA TAT TTC ATA CTT CCA ACT ATA TAG TTA AGG AGG AGA CTG AAA ATG AAG AAG
9061
LEU PHE PHE LEU LEU LEU LEU LEU PHE LEU ILE TYR LEU GLY TYR ASP TYR VAL ASN GLU
TTG TTT TTA TTG TTA TTG TTA TTC TTA ATA TAC TTA GGT TAT GAC TAC GTT AAT GAA

9121
ALA LEU PHE SER GLN GLU LYS VAL GLU PHE GLN ASN TYR ASP GLN ASN PRO LYS GLU HIS
GCA CTG TTT TCT CAG GAA AAA GTC GAA TTT CAA AAT TAT GAT CAA AAT CCC AAA GAA CAT
9181
LEU GLU ASN SER GLY THR SER GLU ASN THR GLN GLU LYS THR ILE THR GLU GLU GLN VAL
TTA GAA AAT AGT GGG ACT TCT GAA AAT ACC CAA GAG AAA ACA ATT ACA GAA GAA CAG GTT
9241
TYR GLN GLY ASN LEU LEU LEU ILE ASN SER LYS TYR PRO VAL ARG GLN GLU SER VAL LYS
TAT CAA GGA AAT CTG CTA TTA ATC AAT AGT AAA TAT CCT GTT CGC CAA GAA AGT GTG AAG
9301
SER ASP ILE VAL ASN LEU SER LYS HIS ASP GLU LEU ILE ASN GLY TYR GLY LEU LEU ASP
TCA GAT ATC GTG AAT TTA TCT AAA CAT GAC GAA TTA ATA AAT GGA TAC GGG TTG CTT GAT
9361
SER ASN ILE TYR MET SER LYS GLU ILE ALA GLN LYS PHE SER GLU MET VAL ASN ASP ALA
AGT AAT ATT TAT ATG TCA AAA GAA ATA GCA CAA AAA TTT TCA GAG ATG GTC AAT GAT GCT
9421
VAL LYS GLY GLY VAL SER HIS PHE ILE ILE ASN SER GLY TYR ARG ASP PHE ASP GLU GLN
GTA AAG GGT GGC GTT AGT CAT TTT ATT ATT AAT AGT GGC TAT CGA GAC TTT GAT GAG CAA
9481
SER VAL LEU TYR GLN GLU MET GLY ALA GLU TYR ALA LEU PRO ALA GLY TYR SER GLU HIS
AGT GTG CTT TAC CAA GAA ATG GGG GCT GAG TAT GCC TTA CCA GCA GGT TAT AGT GAG CAT
9541
ASN SER GLY LEU SER LEU ASP VAL GLY SER SER LEU THR LYS MET GLU ARG ALA PRO GLU
AAT TCA GGT TTA TCA CTA GAT GTA GGA TCA AGC TTG ACG AAA ATG GAA CGA GCC CCT GAA
9601
GLY LYS TRP ILE GLU GLU ASN ALA TRP LYS TYR GLY PHE ILE LEU ARG TYR PRO GLU ASP
GGA AAG TGG ATA GAA GAA AAT GCT TGG AAA TAC GGG TTC ATT TTA CGT TAT CCA GAG GAC
9661
LYS THR GLU LEU THR GLY ILE GLN TYR GLU PRO TRP HIS ILE ARG TYR VAL GLY LEU PRO
AAA ACA GAG TTA ACA GGA ATT CAA TAT GAA CCA TGG CAT ATT CGC TAT GTT GGT TTA CCA

9721
HIS SER ALA ILE MET LYS GLU LYS ASN PHE VAL LEU GLU GLU TYR MET ASP TYR LEU LYS
CAT AGT GCG ATT ATG AAA GAA AAG AAT TTC GTT CTC GAG GAA TAT ATG GAT TAC CTA AAA
9781
GLU GLU LYS THR ILE SER VAL SER VAL ASN GLY GLU LYS TYR GLU ILE PHE TYR TYR PRO
GAA GAA AAA ACC ATT TCT GTT AGT GTA AAT GGG GAA AAA TAT GAG ATC TTT TAT TAT CCT
9841
VAL THR LYS ASN THR THR ILE HIS VAL PRO THR ASN LEU ARG TYR GLU ILE SER GLY ASN
GTT ACT AAA AAT ACC ACC ATT CAT GTG CCG ACT AAT CTT CGT TAT GAG ATA TCA GGA AAC
9901
ASN ILE ASP GLY VAL ILE VAL THR VAL PHE PRO GLY SER THR HIS THR ASN SER ARG ARG
AAT ATA GAC GGT GTA ATT GTG ACA GTG TTT CCC GGA TCA ACA CAT ACT AAT TCA AGG AGG
9961
TAA GGA TGG CGG AAT GAA ACC AAC GAA ATT AAT GAA CAG CAT TAT TGT ACT AGC ACT TTT
10021
GGG GTA ACG TTA GCT TTT TAA TTT AAA ACC CAC GTT AAC TAG GAC ATT GCT ATA CTA ATG
VanZ
10081 LEU GLY LYS ILE LEU SER ARG GLY LEU
ATA CAA CTT AAA CAA AAG AATTAGAGG AAA TTA TA TTG GGA AAA ATA TTA TCT AGA GGA TTG
10143
LEU ALA LEU TYR LEU VAL THR LEU ILE TRP LEU VAL LEU PHE LYS LEU GLN TYR ASN ILE
CTA GCT TTA TAT TTA GTG ACA CTA ATC TGG TTA GTG TTA TTC AAA TTA CAA TAC AAT ATT
10203
LEU SER VAL PHE ASN TYR HIS GLN ARG SER LEU ASN LEU THR PRO PHE THR ALA THR GLY
TTA TCA GTA TTT AAT TAT CAT CAA AGA AGT CTT AAC TTG ACT CCA TTT ACT GCT ACT GGG
10263
ASN PHE ARG GLU MET ILE ASP ASN VAL ILE ILE PHE ILE PRO PHE GLY LEU LEU LEU ASN
AAT TTC AGA GAG ATG ATA GAT AAT GTT ATA ATC TTT ATT CCA TTT GGC TTG CTT TTG AAT

10323
VAL ASN PHE LYS GLU ILE GLY PHE LEU PRO LYS PHE ALA PHE VAL LEU VAL LEU SER LEU
GTC AAT TTT AAA GAA ATC GGA TTT TTA CCT AAG TTT GCT TTT GTA CTG GTT TTA AGT CTT
10383
THR PHE GLU ILE ILE GLN PHE ILE PHE ALA ILE GLY ALA THR ASP ILE THR ASP VAL ILE
ACT TTT GAA ATA ATT CAA TTT ATC TTC GCT ATT GGA GCG ACA GAC ATA ACA GAT GTA ATT
10443
THR ASN THR VAL GLY GLY PHE LEU GLY LEU LEU LYS LEU TYR GLY LEU SER ASN LYS HIS MET
ACA AAT ACT GTT GGA GGC TTT CTT GGA CTG AAA TTA TAT GGT TTA AGC AAT AAG CAT ATG
10503
ASN GLN LYS LYS LEU ASP ARG VAL ILE ILE PHE VAL GLY ILE LEU LEU LEU VAL LEU LEU
AAT CAA AAA AAA TTA GAC AGA GTT ATT ATT TTT GTA GGT ATA CTT TTG CTC GTA TTA TTG
10563
LEU VAL TYR ARG THR HIS LEU ARG ILE ASN TYR VAL
CTC GTT TAC CGT ACC CAT TTA AGA ATA AAT TAC GTG TAAG ATG TCT AAA TCA AGC AAT
10621
CTG ATC TTT CAT ACA CAT AAA GAT ATT GAA TGA ATT GGA TTA GAT GGA AAA CGG GAT GTG
10681
GGG AAA CTC GCC CGT AGG TGT GAA GTG AGG GGA AAA CCG GTG ATA AAG TAA AAA GCT TAC
10741
CTA ACA CTA TAG TAA CAA AGA AAG CCC AAT TAT CAA TTT TAG TGC TGA GGA ATT GGT CTC
10801
TTT AAT AAA TTT CCT TAA CGT TGT AAA TCC GCA TTT TCC TGA CGG TAC CCC

Ib brin(-)

(correspond à la séquence du brin complémentaire du brin (+) de la position 1 à 3189

1
CAA AAT ATC ACC TCA TTT TTG AGA CAA GTC TTA TGA GAC GCT CTT AAC TAT GAT TTT ATC
61
AGT CTA CTA CAT TTG TAT CAA TAG AGT ACA CTC TAT TGA TAT ATA ATT GAA CTA ATA AAT

Transposase
121 MET LYS ILE ALA ARG GLY ARG GLU LEU LEU THR
TGA AAA TAC AGA AAT GGA ATGATACTG AA ATG AAA ATT GCG AGA GGT AGA GAA TTG CTT ACA
182
PRO GLU GLN ARG GLN ALA PHE MET GLN ILE PRO GLU ASP GLU TRP ILE LEU GLY THR TYR
CCG GAA CAG AGA CAG GCT TTT ATG CAA ATC CCT GAA GAT GAA TGG ATA CTG GGG ACC TAC
242
PHE THR PHE SER LYS ARG ASP LEU GLU ILE VAL ASN LYS ARG ARG ARG GLU GLU ASN ARG
TTC ACT TTT TCC AAA CGG GAT TTA GAA ATA GTT AAT AAG CGA AGG AGG GAA GAA AAC CGT
302
LEU GLY PHE ALA VAL GLN LEU ALA VAL LEU ARG TYR PRO GLY TRP PRO TYR THR HIS ILE
TTA GGA TTT GCT GTT CAA TTA GCT GTT CTT CGG TAT CCC GGT TGG CCA TAC ACT CAT ATC
362
LYS SER ILE PRO ASP SER VAL ILE GLN TYR ILE SER LYS GLN ILE GLY VAL SER PRO SER
AAA AGC ATC CCA GAT TCG GTC ATA CAA TAT ATA TCG AAA CAG ATT GGT GTT AGT CCA TCC
422
SER LEU ASP HIS TYR PRO GLN ARG GLU ASN THR LEU TRP ASP HIS LEU LYS GLU ILE ARG
TCG CTT GAT CAT TAT CCT CAA AGG GAA AAT ACA CTT TGG GAT CAT TTG AAA GAA ATT CGA

482
SER GLU TYR ASP PHE VAL THR PHE THR LEU SER GLU TYR ARG MET THR PHE LYS TYR LEU
AGT GAA TAC GAC TTT GTA ACT TTT ACC CTG AGT GAA TAT CGA ATG ACA TTT AAG TAC CTT
542
HIS GLN LEU ALA LEU GLU ASN GLY ASP ALA ILE HIS LEU LEU HIS GLU CYS ILE ASP PHE
CAT CAA TTA GCT TTG GAA AAT GGT GAT GCC ATT CAT CTA CTG CAT GAA TGC ATA GAT TTT
602
LEU ARG LYS ASN LYS ILE ILE LEU PRO ALA ILE THR THR LEU GLU ARG MET VAL TRP GLU
CTA AGA AAA AAC AAA ATT ATA CTG CCT GCT ATC ACT ACA CTT GAA AGA ATG GTG TGG GAA
662
ALA ARG ALA MET ALA GLU LYS LYS LEU PHE ASN THR VAL SER LYS SER LEU THR ASN GLU
GCA AGG GCA ATG GCT GAA AAG AAG CTA TTT AAT ACG GTT AGT AAA TCT CTA ACA AAT GAG
722
GLN LYS GLU LYS LEU GLU GLY ILE ILE THR SER GLN HIS PRO SER GLU SER ASN LYS THR
CAA AAA GAA AAG CTT GAA GGG ATT ATT ACC TCG CAG CAT CCA TCC GAA TCC AAT AAA ACG
782
ILE LEU GLY TRP LEU LYS GLU PRO PRO GLY HIS PRO SER PRO GLU THR PHE LEU LYS ILE
ATA TTG GGT TGG TTA AAA GAG CCA CCG GGT CAT CCT TCA CCC GAA ACT TTT CTA AAA ATA
842
ILE GLU ARG LEU GLU TYR ILE ARG GLY MET ASP LEU GLU THR VAL GLN ILE SER HIS LEU
ATA GAA CGA CTC GAA TAC ATA CGA GGA ATG GAT TTA GAA ACA GTG CAA ATT AGT CAT TTG
902
HIS ARG ASN ARG LEU LEU GLN LEU SER ARG LEU GLY SER ARG TYR GLU PRO TYR ALA PHE
CAC CGT AAC CGC CTG TTG CAG CTG TCT CGC TTA GGC TCA AGA TAC GAG CCG TAT GCA TTC
962
ARG ASP PHE GLN GLU ASN LYS ARG TYR SER ILE LEU THR ILE TYR LEU LEU GLN LEU THR
CGT GAC TTT CAA GAA AAT AAA CGT TAT TCG ATA TTA ACC ATC TAT TTA TTA CAA CTT ACT

1022
GLN GLU LEU THR ASP LYS ALA PHE GLU ILE HIS ASP ARG GLN ILE LEU SER LEU LEU SER
CAG GAG CTA ACG GAT AAA GCG TTT GAA ATT CAT GAT AGG CAA ATA CTT AGT TTG TTA TCA
1082
LYS GLY ARG LYS ALA GLN GLU GLU ILE GLN LYS GLN ASN GLY LYS LYS LEU ASN GLU LYS
AAA GGT CGT AAG GCT CAA GAG GAA ATC CAG AAA CAA AAC GGT AAA AAG CTA AAT GAG AAA
1142
VAL ILE HIS PHE THR ASN ILE GLY GLN ALA LEU ILE LYS ALA ARG GLU GLU LYS LEU ASP
GTT ATA CAC TTT ACG AAC ATC GGA CAA GCA TTA ATT AAA GCA AGA GAG GAA AAA TTA GAC
1202
VAL PHE LYS VAL LEU GLU SER VAL ILE GLU TRP ASN THR PHE VAL SER SER VAL GLU GLU
GTT TTT AAG GTT TTA GAA TCG GTT ATT GAA TGG AAT ACC TTT GTC TCT TCA GTA GAA GAG
1262
ALA GLN GLU LEU ALA ARG PRO ALA ASP TYR ASP TYR LEU ASP LEU LEU GLN LYS ARG PHE
GCT CAG GAA CTT GCA CGT CCT GCC GAC TAT GAT TAT TTA GAC TTA CTG CAA AAA CGG TTT
1322
TYR SER LEU ARG LYS TYR THR PRO THR LEU LEU ARG VAL LEU GLU PHE HIS SER THR LYS
TAT TCA CTA AGA AAA TAT ACG CCA ACG CTA TTA AGA GTA TTG GAA TTT CAT TCT ACA AAG
1382
ALA ASN GLU PRO LEU LEU GLN ALA VAL GLU ILE ILE ARG GLY MET ASN GLU SER GLY LYS
GCA AAT GAG CCA CTT TTA CAA GCT GTT GAG ATT ATC CGA GGA ATG AAC GAA TCT GGA AAG
1442
ARG LYS VAL PRO ASP ASP SER PRO VAL ASP PHE ILE SER LYS ARG TRP LYS ARG HIS LEU
CGA AAA GTG CCT GAT GAC TCA CCT GTG GAT TTT ATT TCA AAA CGA TGG AAA AGA CAT TTA
1502
TYR GLU ASP ASP GLY THR THR ILE ASN ARG HIS TYR TYR GLU MET ALA VAL LEU THR GLU
TAC GAG GAT GAT GGT ACA ACA ATT AAT CGT CAT TAC TAT GAA ATG GCT GTT TTA ACA GAA
1562
LEU ARG GLU HIS VAL ARG ALA GLY ASP VAL SER ILE VAL GLY SER ARG GLN TYR ARG ASP
CTT CGG GAG CAT GTT CGG GCA GGA GAT GTT TCC ATT GTT GGC AGC AGA CAA TAT AGG GAT

1622
PHE GLU GLU TYR LEU PHE SER GLU ASP THR TRP ASN GLN SER LYS GLY ASN THR ARG LEU
TTT GAG GAA TAT TTG TTT TCG GAA GAT ACA TGG AAT CAA TCG AAG GGG AAT ACG AGA TTA
1682
SER VAL SER LEU SER PHE GLU ASP TYR ILE THR GLU ARG THR SER SER PHE ASN GLU ARG
TCA GTT AGT TTA TCA TTC GAA GAT TAT ATA ACG GAG AGA ACC AGC AGC TTT AAT GAA AGG
1742
LEU LYS TRP LEU ALA ALA ASN SER ASN LYS LEU ASP GLY VAL SER LEU GLU LYS GLY LYS
TTA AAG TGG TTA GCT GCC AAT TCC AAT AAG TTA GAT GGG GTT TCT CTT GAA AAA GGA AAG
1802
LEU SER LEU ALA ARG LEU GLU LYS ASP VAL PRO GLU GLU ALA LYS LYS PHE SER ALA SER
CTA TCA CTT GCA CGC TTA GAA AAA GAT GTT CCA GAA GAA GCA AAA AAA TTT AGT GCA AGC
1862
LEU TYR GLN MET LEU PRO ARG ILE LYS LEU THR ASP LEU LEU MET ASP VAL ALA HIS ILE
CTT TAT CAG ATG CTA CCA AGA ATA AAA TTA ACT GAT TTA CTC ATG GAT GTG GCC CAT ATA
1922
THR GLY PHE HIS GLU GLN PHE THR HIS ALA SER ASN ASN ARG LYS PRO ASP LYS GLU GLU
ACA GGA TTT CAT GAG CAA TTC ACT CAT GCT TCC AAT AAT CGA AAA CCA GAT AAG GAA GAA
1982
THR ILE ILE ILE MET ALA ALA LEU LEU GLY MET GLY MET ASN ILE GLY LEU SER LYS MET
ACA ATC ATT ATC ATG GCT GCC CTT TTA GGA ATG GGA ATG AAT ATT GGC TTG AGC AAG ATG
2042
ALA GLU ALA THR PRO GLY LEU THR TYR LYS GLN LEU ALA ASN VAL SER GLN TRP ARG MET
GCC GAA GCC ACA CCC GGA CTT ACA TAT AAG CAA CTA GCC AAT GTA TCT CAA TGG CGC ATG
2102
TYR GLU ASP ALA MET ASN LYS ALA GLN ALA ILE LEU VAL ASN PHE HIS HIS LYS LEU GLN
TAT GAA GAT GCC ATG AAT AAA GCC CAA GCC ATA TTA GTA AAC TTT CAT CAT AAA TTA CAA
2162
LEU PRO PHE TYR TRP GLY ASP GLY THR THR SER SER SER ASP GLY MET ARG MET GLN LEU
TTG CCT TTC TAT TGG GGC GAC GGT ACA ACA TCT TCG TCA GAT GGT ATG AGA ATG CAG CTA

2222
GLY VAL SER SER LEU HIS ALA ASP ALA ASN PRO HIS TYR GLY THR GLY LYS GLY ALA THR
GGT GTT TCA TCA CTA CAT GCA GAT GCA AAT CCA CAT TAT GGA ACT GGA AAA GGA GCC ACC
2282
ILE TYR ARG PHE THR SER ASP GLN PHE SER SER TYR TYR THR LYS ILE ILE HIS THR ASN
ATC TAC CGA TTT ACA AGT GAT CAA TTC TCT TCT TAC TAC ACA AAG ATT ATT CAT ACT AAT
2342
SER ARG ASP ALA ILE HIS VAL LEU ASP GLY LEU LEU HIS HIS GLU THR ASP LEU ASN ILE
TCA AGA GAT GCG ATT CAT GTT TTG GAT GGT TTG TTA CAT CAT GAG ACG GAT CTA AAC ATA
2402
GLU GLU HIS TYR THR ASP THR ALA GLY TYR THR ASP GLN ILE PHE GLY LEU THR HIS LEU
GAG GAA CAT TAT ACA GAC ACT GCC GGT TAC ACT GAC CAA ATA TTC GGA CTG ACT CAT TTA
2462
LEU GLY PHE LYS PHE ALA PRO ARG ILE ARG ASP LEU SER ASP SER LYS LEU PHE THR ILE
TTA GGA TTT AAA TTT GCC CCA AGA ATA AGG GAT TTA TCG GAC TCA AAA TTA TTT ACG ATA
2522
ASP LYS ALA SER GLU TYR PRO LYS LEU GLU ALA ILE LEU ARG GLY GLN ILE ASN THR LYS
GAT AAA GCA AGT GAG TAT CCA AAA CTA GAA GCC ATT TTA CGT GGA CAA ATA AAT ACA AAG
2582
VAL ILE LYS GLU ASN TYR GLU ASP VAL LEU ARG LEU ALA HIS SER ILE ARG GLU GLY THR
GTC ATT AAA GAA AAT TAT GAG GAT GTT TTG CGA TTA GCT CAT TCT ATA AGG GAG GGA ACA
2642
AGT TTC AGC ATC CCT TAT TAT GGG GAA GCT AGG TTC TTC CTA TTC AAG ACA AAA CAG CTT AGC
VAL SER ALA SER LEU ILE MET GLY LYS LEU GLY SER SER TYR SER ARG GLN ASN SER LEU ALA
GTT TCA GCA TCC CTT ATT ATG GGG AAG CTA GGT TCC TCC TAT TCA AGA CAA AAC AGC TTA GCT
2702
THR ALA LEU ARG GLU MET GLY ARG ILE GLU LYS THR ILE PHE ILE LEU ASN TYR ILE SER
ACA GCC TTA CGT GAG ATG GGC CGA ATA GAA AAA ACG ATC TTT ATT TTG AAT TAT ATA TCG

2762
ASP GLU SER LEU ARG ARG LYS ILE GLN ARG GLY LEU ASN LYS GLY GLU ALA MET ASN GLY
GAT GAA TCA TTA AGA AGA AAA ATA CAA AGA GGA TTG AAT AAA GGA GAA GCC ATG AAT GGA
2822
LEU ALA ARG ALA ILE PHE PHE GLY LYS GLN GLY GLU LEU ARG GLU ARG THR ILE GLN HIS
TTG GCA AGA GCT ATT TTC TTC GGA AAA CAA GGT GAG CTT AGA GAA CGC ACC ATA CAG CAT
2882
GLN LEU GLN ARG ALA SER ALA LEU ASN ILE ILE ILE ASN ALA ILE SER ILE TRP ASN THR
CAA TTG CAA AGA GCC AGT GCT TTA AAC ATA ATT ATC AAT GCT ATA AGT ATT TGG AAT ACT
2942
TCT CCA CCT AAC AAC AGC AGT TGA ATA TAA AAA ACG GAC AGG TAG CTT TAA TGA AGA TTT
LEU HIS LEU THR THR ALA VAL GLU TYR LYS LYS ARG THR GLY SER PHE ASN GLU ASP LEU
CTC CAC CTA ACA ACA GCA GTT GAA TAT AAA AAA CGG ACA GGT AGC TTT AAT GAA GAT TTG
3002
LEU HIS HIS MET SER PRO LEU GLY TRP GLU HIS ILE ASN LEU LEU GLY GLU TYR HIS PHE
TTA CAC CAT ATG TCG CCC TTA GGT TGG GAA CAT ATT AAT TTA CTA GGA GAA TAC CAT TTT
3062
ASN SER GLU LYS VAL VAL SER LEU ASN SER LEU ARG PRO LEU LYS LEU SER
AAC TCA GAG AAA GTA GTC TCA TTA AAT TCT TTA AGA CCA CTA AAA CTT TCT TAA CGT TG
3121
TTA AAA ACG AGG GAT TCG TCA GGA AAA TAG GCT TAG CGT TGT AAA TCC GCA TTT TCC TGA
3181
CGC TAC CCC

LISTE DES SEQUENCES : II

SacI
GAGCTCTTCCTTCAACGCACTTCTGTACCAAGAGTTGTTGTC 42

CATTTGATCACTAACAATAGCTTCCCCTGCTTTCTTCAAGCCCTTTGTCATAAAATCGTTAGATTTTCA 111

TCATAAAAATACGAGAAAGACAACAGGAAGACCGCAAATTTTTCTTTTTCCTAGGTACACTGAATG 180

RBS
M K K I A V L F G G
TAACCTTAAAAGAAAAAAAGGAAAGGAAGAAAAATGATGAAAAAAATTGCCGTTTTATTTGGAGGG 244

N S P E Y S V S L T S A A S V I Q A I D
AATTCTCCAGAATACTCAGTGTCACTAACCTCAGCAGCAAGTGTGATCCAAGCTATTGAC 304

P L K Y E V M T I G I A P T M D W Y W Y
CCGCTGAAATATGAAGTAATGACCATTGGCATCGCACCAACAATGGATTGGTATTGGTAT 364

Q G N L A N V R N D T W L E D H K N C H
CAAGGAAACCTCGCGAATGTTCGCAATGATACTTGGCTAGAAGATCACAAAAACTGTCAC 424

Q L T F S S Q G F I L G E K R I V P D V
CAGCTGACTTTTTCTAGCCAAGGATTTATATTAGGAGAAAAACGAATCGTCCCTGATGTC 484

L F P V L H G K Y G E D G C I Q G L L E
CTCTTTCCAGTCTTGCATGGAAAGTATGGCGAGGATGGCTGTATCCAAGGACTGCTTGAA 544

L M N L P Y V G C H V A A S A L C M N K
CTAATGAACCTGCCTTATGTTGGTTGCCATGTCGCTGCCTCCGCATTATGTATGAACAAA 604

W L L H Q L A D T M G I A S A P T L L L
TGGCTCTTGCATCAACTTGCTGATACCATGGGAATCGCTAGTGCTCCCACTTTGCTTTTA 664

S R Y E N D P A T I D R F I Q D H G F P
TCCCGCTATGAAAACGATCCTGCCACAATCGATCGTTTTATTCAAGACCATGGATTCCCG 724

I F I K P N E A G S S K G I T K V T D K
ATCTTTATCAAGCCGAATGAAGCCGGTTCTTCAAAAGGGATCACAAAAGTAACTGACAAA 784

T A L Q S A L T T A F A Y G S T V L I Q
ACAGCGCTCCAATCTGCATTAACGACTGCTTTTGCTTACGGTTCTACTGTGTTGATCCAA 844

K A I A G I E I G C G I L G N E Q L T I
AAGGCGATAGCGGGTATTGAAATTGGCTGCGGCATCTTAGGAAATGAGCAATTGACGATT 904

G A C D A I S L V D G F F D F E E K Y Q
GGTGCTTGTGATGCGATTTCTCTTGTCGACGGTTTTTTTGATTTTGAAGAGAAATACCAA 964

L I S A T I T V P A P L P L A L E S Q I
TTAATCAGCGCCACGATCACTGTCCCAGCACCATTGCCTCTCGCGCTTGAATCACAGATC 1024

K E Q A Q L L Y R N L G L T G L A R I D
AAGGAGCAGGCACAGCTGCTTTATCGAAACTTGGGATTGACGGGTCTGGCTCGAATCGAT 1084

F F V T N Q G A I Y L N E I N T M P G F
TTTTTCGTCACCAATCAAGGAGCGATTTATTTAAACGAAATCAACACCATGCCGGGATTT 1144

T G H S R Y P A M M A E V G L S Y E I L
ACTGGGCACTCCCGCTACCCAGCTATGATGGCGGAAGTCGGGTTATCCTACGAAATATTA 1204

V E Q L L E A L A E E D K R *
GTAGAGCAATTGATTGCACTGGCAGAGGAGGACAAACGATGAACACATTACAATTGATCAATA 1267

AAAACCATCCATTGAAAAAATCAAGAGCCCCGCACTTAGTGCTAGCTCCTTTTAGCGATCACGATG 1336

TTTACCTGCAG 1347
PstI

## Revendications

1. Séquence nucléotidique de 7,3 kb environ, correspondant au fragment de restriction *HindIII-EcoRI* tel qu'obtenu à partir du plasmide pIP816, comprenant ce fragment *HindIII-EcoRI* ou toute partie de ce fragment, en particulier le fragment de 3,4 kb *EcoRI-Xbal,* le fragment d'environ 1,7 kb *EcoRV-SacII* et le fragment de 3,3 kb *HindIII-EcoRI.*

2. Séquence nucléotidique selon la revendication 1, **caractérisée en ce qu'**elle comporte dans l'ordre lessites de restriction suivants tel qu'obtenus à partir du plasmide pIP816: *HindIII, Bglll, BglII, EçoRI, BamHI, Xbal, EcoRI.*

3. Séquence nucléotidique selon la revendication 1 ou 2, **caractérisée en ce qu'**elle correspond à l'un des enchaînements identifiés par SEQ ID NO 6, SEQ ID NO 7 ou SEQ ID NO 22, ou **en ce qu'**elle comprend un de ces enchaînements ou toute partie de l'un de ces enchaînements, ou encore tout enchaînement ou partie d'enchaînement d'ADN complémentaire, ou tout enchaînement d'ARN correspondant à l'un de ces ADN, susceptible
- soit de constituer une sonde d'hybridation ou une amorce, pour la détection d'une résistance à des antibiotiques de la famille des glycopeptides, notamment à la vancomycine et/ou à la teicoplanine, en particulier dans des souches de la famille des cocci à Gram-positif,
- soit de coder pour une séquence nécessaire à l'expression ou la régulation de la résistance à des antibiotiques de la famille des glycopeptides, notamment la vancomycine et/ou la teicoplanine, en particulier dans des souches de la famille des cocci à Gram-positif.

4. Séquence nucléotidique selon la revendication 3, **caractérisée en ce qu'**elle comprend l'un des enchaînements suivants :

V1 : GGX GAA GAT GGX TCX TTX CAA GGX
           G   C     AG  C     G
                         A

V2 : AAT ACX ATX CCX GGX TTT AC
       C       T           C
               C

5. Séquence nucléotidique selon l'une quelconque des revendications 1 à 3, **caractérisée en ce qu'**il s'agit de l'un des enchaînements SEQ ID NO 14 (vanY), SEQ ID NO 15 (vanZ), SEQ ID NO 8 (vanA), SEQ ID NO 12 (transposase), SEQ ID NO 13 (résolvase), ou d'une variante de l'un de ces enchaînements dès lors qu'elle code pour une protéine ayant des propriétés immunologiques et/ou fonctionnelles similaires à celles des protéines codées par les enchaînements SEQ ID NO 14 (vanY), SEQ ID NO 15 (vanZ), SEQ ID NO 8 (vanA) ,SEQ ID NO 12 (transposase), SEQ ID NO 13 (résolvase), ou dès lors qu'elle permet la détection de souches résistantes à des antibiotiques de la famille des glycopeptides.

6. Séquence nucléotidique selon l'une quelconque des revendications 1 à 3, **caractérisée en ce qu'**elle correspond à l'enchaînement SEQ ID NO 6 ou à l'enchaînement SEQ ID NO 22 ou **en ce qu'**elle comprend cet enchaînement.

7. Séquence recombinante, **caractérisée en ce qu'**elle comprend une séquence de nucléotides selon l'une quelconque des revendications 1 à 5, sous le contrôle d'éléments de régulation susceptibles d'intervenir dans l'expression, de la résistance à des antibiotiques de la famille des glycopeptides, notamment la vancomycine ou la teicoplanine, chez un hôte déterminé.

8. Vecteur recombinant, **caractérisé en ce qu'**il comprend une séquence nucléotidique selon l'une quelconque des revendications 1 à 7, en un site non essentiel pour sa réplication, sous le contrôle d'éléments de régulation susceptibles d'intervenir dans l'expression, de la résistance à des antibiotiques de la famille des glycopeptides, notamment la vancomycine ou' la teicoplanine, chez un hôte déterminé.

9. Vecteur recombinant selon la revendication 8, **caractérisé en ce qu'**il s'agit du plasmide pAT214.

10. Hôte cellulaire recombinant, **caractérisé en ce qu'**il comprend une séquence nucléotidique selon l'une quelconque des revendications 1 à 7 ou un vecteur selon la revendication 8 ou 9, dans des conditions permettant l'expression de la résistance à des antibiotiques de la famille des glycopeptides, notamment la vancomycine ou la teicoplanine, cet hôte étant par exemple choisi parmi les bactéries, notamment les cocci à Gram-positif.

11. Sonde nucléotidique, **caractérisée en ce qu'**il s'agit d'ADN ou d'ARN et **en ce qu'**elle est capable d'hybrider avec une séquence selon l'une quelconque des revendications 1 à 6, cette sonde étant le cas échéant marquée.

12. Sonde nucléotidique selon la revendication 11, **caractérisée en ce qu'**elle est spécifique chez des bactéries à Grain-positif, des séquences codant pour une protéine de résistance à des glycopeptides notamment à la vancomycine et/ou à la teicoplanine et universelle parmi ces séquences.

13. Sonde nucléotidique selon la revendication 11, **caractérisée en ce qu'**elle est spécifique d'une séquence nucléotidique codant pour une protéine nécessaire à l'expression d'un haut niveau de résistance à des antibiotiques de la famille des glycopeptides, en particulier à la vancomycine et à la teicoplanine, chez des bactéries à Gram-positif.

14. Sonde nucléotidique selon la revendication 11, **caractérisée en ce qu'**elle est spécifique d'une séquence nucléotidique codant pour une protéine nécessaire à l'expression d'un bas niveau de résistance à des antibiotiques de la famille des glycopeptides, en particulier à la vancomycine, chez des bactéries à Gram-positif.

15. Sonde nucléotidique selon l'une quelconque des revendications 11 à 14, **caractérisée en ce qu'**elle hybride avec une séquence nucléotidique non chromosomique d'une souche résistante à des glycopeptides, notamment la vancomycine et/ou la teicoplanine, en particulier **en ce qu'**elle hybride avec une séquence nucléotidique non chromosomique d'une souche de cocci Gram-positif, par exemple une souche d'entérocoque et de préférence *E. faecium* 4147.

16. Procédé de détection *in vitro* de la présence de souches résistantes aux glycopeptides, en particulier à la vancomycine et/ou à la teicoplanine, ces souches appartenant en particulier à la famille des cocci Gram-positif, notamment en ce qu'il s'agit de souches d'entérocoques, par exemple *E. faecium* ou *E.qallinarum,* **caractérisé en ce qu'**il comprend :
a) la mise en contact d'un échantillon biologique susceptible de contenir les souches résistantes, avec une amorce constituée par une séquence nucléotidique selon l'une quelconque des revendications 11 à 15, capable d'hybrider avec une séquence nucléotidique recherchée, nécessaire à l'expression de la résistance, cette séquence étant utilisée comme matrice, en présence des 4 différents nucléosides triphosphates, et d'un agent de polymérisation, dans des conditions d'hybridation telles que pour chaque séquence nucléotidique ayant hybride avec une amorce, un produit d'élongation de chaque amorce complémentaire de la matrice est synthétisé,
b) la séparation de la matrice et du produit d'élongation obtenu, ce dernier pouvant alors également se comporter comme une matrice,
c) la répétition de l'étape a) de façon à obtenir une quantité détectable des séquences nucléotidiques recherchées, et
d)la détection du produit d'amplification des séquences nucléotidiques.
